# EUROPEAN PATENT SPECIFICATION

(11) **EP 0 942 746 B1**
(45) Date of publication and mention of the grant of the patent: **16.03.2005**
(21) Application number: 97946746.1
(22) Date of filing: 08.12.1997
(51) Int. Cl.: A61K 38/57, A61K 45/06, G01N 33/50

(54) **INHIBITION OF INVASIVE REMODELLING**
INHIBIERUNG VON INVASIVER REMODULIERUNG
INHIBITION DU REMODELAGE INVASIF

(30) Priority: 06.12.1996 DK 140296
(43) Date of publication of application: 22.09.1999
(73) Proprietor: FONDEN TIL FREMME AF EKSPERIMENTEL CANCERFORSKNING, DK-1552 Copenhagen V (DK)
(72) Inventor: LUND, Leif Roege, DK-2100 Copenhagen (DK); DANOE, Keld, DK-2920 Charlottenlund (DK); STEPHENS, Ross, DK-2920 Charlottenlund (DK); BRÜNNER, Nils, DK-2900 Hellerup (DK); SOLBERG, Helene, DK-3400 Hilleroed (DK); HOLST-HANSEN, Claus, DK-1950 Frederiksberg C (DK); NIELSEN, John Roemer, DK-2100 Copenhagen (DK)
(74) Representative: Plougmann & Vingtoft A/S
(86) International application number: PCT/DK1997/000555
(87) International publication number: WO 1998/024474

(56) References cited:
- EP-A- 0 238 993
- WO-A-92/20361
- WO-A-95/24921
- SCHULTZ ET AL: "TREATMENT OF ALKALI-INJURED RABBIT CORNEAS WITH A SYNTHETIC INHIBITOR OF MATRIX METALLOPROTEINASES" INVESTIGATIVE OPHTHALMOLOGY AND VISUAL SCIENCE, vol. 33, no. 12, November 1992, pages 3325-3331, XP000676618
- BUGGE ET AL: "PLASMINOGEN DEFICIENCY CAUSES SEVERE THROMBOSIS BUT IS COMPATIBLE WITH DEVELOPMENT AND REPRODUCTION" GENES DEV., vol. 9, 1995, pages 794-807, XP002063450 cited in the application
- KHOKHA ET AL: "UTILIZATION OF TRANSGENIC MICE IN THE STUDY OF MATRIX DEGRADING PROTEINASES AND THEIR INHIBITORS" CANCER AND METASTASIS REVIEWS, vol. 14, 1995, pages 97-111, XP002063451
- STONELAKE ET AL: "PROTEINASE INHIBITORS REDUCE BASMENT MEMBRANE DEGRADATION BY HUMAN BREAST CANCER CELL LINES" BRITISH JOURNAL OF CANCER, vol. 75, no. 7, April 1997, pages 951-959, XP002063452

## Description

### FIELD OF THE INVENTION

The present invention pertains to the use of novel compositions for the manufacture of a medicament for preventing or arresting invasive tissue remodelling in mammals by utilising a combination of *in vivo* inhibition of plasmin and *in vivo* inhibition of certain other proteolytic enzymes, notably metalloproteases. The composition can e.g. be used as a novel means for treating malignant neoplastic disorders, but also as an alternative to current methods of contraception as well as antifungal, antibacterial, anti-protozoap, and anti-viral treatment. Further, the invention relates to said use of novel compositions which comprise a plasmin inhibitor in admixture with an inhibitor of another proteolytic enzyme.

### GENERAL BACKGROUND

### Invasive tissue remodelling

A number of physiological and pathological processes involve invasive tissue remodelling, including skin wound healing in which keratinocytes migrate into the wound leading to re-epithelialisation, cancer invasion, a process in which cancer cells migrate into the normal tissue, which is then eventually replaced by cancer tissue, and the invasion of trophoblasts into the uterus after implantation of the egg, eventually leading to formation of the placenta. These invasive tissue remodelling processes share features such as migration of cells, degradation of the extracellular matrix that constitutes a cell scaffold, and formation of a new scaffold. It is becoming increasingly clear that the molecular mechanisms involved are also very similar. The same repertoire of protein degrading enzymes is thus involved in the degradation of extracellular matrix in different invasive tissue remodelling processes (Danø et al, 1985; Hewitt and Danø, 1996), and there are strong similarities in the way epithelial cells interact with stromal connective tissue cells in the different processes (Hewitt and Danø, 1996; Rønnov-Jessen et al, 1996). Particularly striking are the similarities between skin wound healing and cancer. In his classical study of this issue, Harold Dvorak thus termed cancers "wounds that do not heal" (Dvorak, 1986).

With respect to the role of protein-degrading enzymes there are also strong similarities between invasive tissue remodelling processes and certain other tissue remodelling processes, such as mammary gland involution after termination of lactation, uterine involution after parturition, and epithelial shedding into the lumen of the gastrointestinal tract (Danø et al, 1985; Hewitt and Danø, 1996; Lund et al, 1996).

Non-neoplastic stromal cells are strongly involved in generation and regulation of matrix degrading protease activity in cancer invasion (Hewitt and Danm,.1996). The pattern of stromal cell involvement is characteristic for each type of cancer, and it is particularly noteworthy that there are strong similarities not only in the proteases involved, but also in the cellular patterns of expression of these proteases, between cancer originating from a certain tissue and distinct non-malignant tissue remodelling processes in the same tissue, (reviewed by Hewitt and Danø, 1996).

One example of such a similarity is seen when comparing mammary tissue undergoing the normal process of post-lactational involution, with breast carcinoma tissue; mRNAs for uPA (urokinase plasminogen activator), gelatinase A and stromelysin-3 are expressed in both cases and in both cases all three mRNAs are located in fibroblast-like stromal cells that surround the epithelial cells.

Similarities are also seen between patterns of matrix-degrading protease expression associated with the normal process of epithelial shedding into the lumen of the gastrointestinal tract and patterns of matrix degrading protease expression in colon cancer. In colon cancer uPAR (urokinase plasmin activator receptor) is expressed by neoplastic epithelial cells and uPA is expressed by adjacent fibroblast-like cells. Likewise in the normal gastrointestinal tract, uPAR is expressed by luminal epithelial cells, and uPA is expressed by adjacent fibroblast-like cells in the lamina propria. This similarity suggests that in both situations the plasmin generation is controlled by co-operation between epithelial cells and the adjacent fibroblast-like cells.

Similarities are again seen between patterns of matrix-degrading protease expression in healing skin wounds and skin carcinomas: the expression of uPA, PAI-1 (plasminogen activator inhibitor, type I), uPAR, interstitial collagenase and stromelysin-2 in the neoplastic epithelial cells in squamous cell carcinomas resemble the pattern of expression of all these molecules during the re-epithelialisation and healing of skin wounds. All are expressed at the leading edge of the regenerating epithelial outgrowth, which suggests that they are involved in degrading the extracellular matrix during keratinocyte migration. The similarities between skin wound healing and squamous cell skin carcinoma also extend to stromelysin-3, which is expressed by fibroblastic cells in both cases.

The similarities between cancer invasion and non-neoplastic tissue remodelling with respect to proteolytic mechanisms are also reflected in recent studies with mice that have been made plasminogen deficient by targeted gene inactivation. In these mice there is an impairment of skin wound healing (Rømer et al, 1996), mammary gland involution (Bjørn et al, 1997), and cancer metastasis (Bugge et al, 1997). All three processes are delayed but do eventually proceed.

The similarities discussed above strongly support the notion that the same or very similar proteolytic mechanisms at the phenotypic level are involved in non-neoplastic tissue remodelling and cancer invasion. The main differences appear to be in the genetic regulation of the processes. In skin wound healing, the keratinocytes thus stop the migration when the wounds are closed, while in skin cancer the malignant epithelial cells continue their migration/invasion. But, the proteolytic mechanisms per se are virtually the same. Cancer invasion can thus in this respect be considered as a non-neoplastic remodelling process that has escaped normal regulation. Data on the proteolytic mechanisms involved in a non-neoplastic tissue remodelling process can therefore contribute invaluable information to our understanding of cancer invasion.

### Disease Targets

There exist a number of diseases in which the pathogenesis includes a major contribution from invasive tissue remodelling. Particularly important among these diseases are the malignant neoplasms (Dan∅ et al, 1985 and Hewitt and Dan∅, 1996). Since metastatic disease is the main cause of cancer patient morbidity and mortality, a strong need exists for the provision of novel effective pharmaceuticals and methods to interfere in the invasive tissue remodelling which characterises tumour spreading. Also included among these diseases are certain chronic inflammatory diseases (e.g rheumatoid arthritis; DanO et al, 1985), and infectious diseases caused by certain bacteria (e.g. Lyme disease; Coleman et al, 1997) and fungi which have a pathogenesis wherein the invasive tissue remodelling is a major factor. Also, the destruction of articular cartilage in rheumatoid arthritis occurs partly as a result of the growth of invasive pannus over the surface of the cartilage.

### Other targets

It should also be noted that there are quite normal physiological processes, such as pregnancy, which involve invasive remodelling. In order for an embryo to become attached to the uterine wall and reach a favourable blood supply, it must invade and substantially remodel the endometrium. Currently, the contraceptive pharmaceuticals used are based on hormones (e.g. the P pill), but these may cause a variety of undesired side effects in the user, both physically and psychologically. Thus, there is also a definite need for alternative contraceptive methods and pharmaceuticals which could be implemented in daily life with the same ease and convenience as e.g. the P pill, but without subjecting the users to the undesired side effects sometimes associated therewith.

### Malignant Tumours

The invasion process by which malignant tumours spread can now be more accurately described as a process of unregulated relentless tissue remodelling, progressively involving normal tissue which is recruited into the tumour stroma by the neoplastic tumour cells. In this process the barriers which normally define the homeostasis of differentiated tissue are breached by new signals from the de-differentiating and proliferating neoplastic cells. Recruitment and reorganisation of the normal tissue cells progressively disrupts organ structure, as the neoplastic cells develop and continuously remodel a supporting stroma, complete with a new blood capillary network. Tumour tissue remodelling is also accompanied by the detachment of tumour cells and their active invasion of lymph and blood vessels, from where they can metastasise to form new tumours at distant sites.

The hallmark of invasive tissue remodelling is the degradation of the existing extracellular matrix, the fabric or scaffold which normally maintains the integrity of organised tissue structures. In particular, basement membrane is a specialised form of matrix which normally forms a boundary adjacent to the epithelial tissues where carcinomas can arise. Basement membrane consists of numerous glycoproteins, such as proteoglycans, fibronectin, laminin, and collagen type IV. In order to degrade and traverse the first barrier represented by the basement membrane, it is a prerequisite that carcinomas arising in epithelial tissues either acquire the ability themselves to secrete proteolytic enzymes which can cleave basement membrane proteins, or induce the required proteases in the normal tissue cells which are progressively recruited into the tumour stroma. In addition, proteolysis of the extracellular matrix may in turn stimulate growth of tumours by release and/or activation of growth factors and cytokines (Mignatti, 1993). Thus certain proteases are highly expressed in tumour tissue, including members of both the serine protease family, e.g. urokinase-type plasminogen activator (Danø, 1985) and the metalloprotease family (Liotta, 1990). The level of expression of the different enzymes, as well as the tissue distribution or pattern of expression are important in the invasion. Increased expression of serine proteases and metalloproteases has been demonstrated to be responsible for tumour invasion in several different *in vitro* and in vivo invasion models. It is also significant that on examination of the pattern of expression, both classes of enzyme are commonly found together in tumours in vivo, even in the cases e.g. colon carcinoma, where the stromal cells (recruited normal tissue) have been shown to be the main source of proteases (Pyke, 1991; Nielsen, 1996). Furthermore, the level of both urokinase (Ganesh, 1994) and collagenase (Murray, 1996) in human colon tumours has also been shown to have significant prognostic value in predicting those patients who will fare worse from the progress of their tumour, so that the levels of both proteases are related to the invasive capacity of the tumour in vivo. The role of extracellular proteases in tumour growth and metastasis is a subject of current interest and investigation. However, no conclusions exist in the art concerning the relative importance of the various proteases in tumour-directed tissue remodelling (for reviews, cf. Hewitt and Danø, 1996; DeClerk,1996; Mignatti 1993; Liotta 1990). The task becomes even more complex with the discovery of an increasing number of metalloprotease classes as well as new members of classes, e.g. the matrix metalloproteases (MT-MMPs) and the ADAM family of disintegrins.

### Wound Healing

An especially useful experimental model for the study of invasive remodelling which occurs in tumours is the tissue remodelling of a healing wound (e.g. in mouse skin, Rømer et al, 1996). In fact tumours have been called "wounds that do not heal" (Dvorak, 1986). In a healing skin wound the migration of the keratinocytes can be clearly observed, dissecting their way through a fibrin-rich matrix (Rømer et al, 1996). This is accompanied by infiltrating neutrophils and macrophages and formation of granulation tissue containing growing blood capillaries (angiogenesis), all processes known to require the action of proteolytic enzymes, and necessary for the overall tissue remodelling which closes the wound (Martin, 1997). Several of the proteolytic enzymes expressed are in common to both wound healing and tumour-directed tissue remodelling, e.g gelatinase B (MMP-9), stromelysin 1 (MMP-3) and urokinase (uPA) . Furthermore, examination of the pattern of protease expression in the tissues has reinforced the view that wound healing strongly resembles tissue remodelling in cancer. Since wound healing may be initiated in adult animals by, for example, making a simple skin incision of defined length, and measured by monitoring the closing gap on the external surface of the animal, it is a very suitable model for quantitative studies of the effect on invasive tissue remodelling of selected drugs at various dosages and schedules (see below). Studies on mice rendered plasminogen deficient by gene inactivation has shown an impaired wound healing in such mice compared with wild type mice (Rømer et al., 1996) because of a diminished ability of the migrating keratinocytes to dissect their way through the fibrin-rich matrix (Rømer et al., 1996 and Bugge *et al.*, 1996). These studies for the first time unequivocally demonstrate an important role of plasminogen in an invasive tissue remodelling process. However, no conclusions exist in the art concerning the relative importance of the various matrix-degrading proteases in wound healing. In this context it is important to note that fibrin can be degraded by e.g. stromelysin-1 (Bini 1996), and not only plasmin.

A special case of wound healing is that following chemical injury, infection or other insult to the cornea of the eye, where degradation of extracellular matrix by proteolytic enzymes produced by infiltrating inflammatory cells retards, rather than promotes, regrowth of epithelium over the surface of the cornea. This effect is due to the requirement for a matrix substratum to enable adhesion and migration of the epithelial cells over the wound surface. Inhibitors of proteolytic enzymes have been successfully used in this case to protect the matrix substratum from inflammatory cell mediated proteolysis, and thereby actually promote corneal re-epithelialisation (Schultz et al, 1992). In fact it has proven most effective for this purpose to topically apply a mixture comprising a metalloprotease inhibitor (GM-6001, see below), epithelial growth factor, fibronectin (a component of the pericellular matrix) and aprotinin (a serine protease .inhibitor) to the surface of the eye. The effectiveness of this mixture offers some suggestion that metalloproteases and serine proteases may be involved in the inflammatory destruction of the matrix substratum that retards epithelial migration and healing of the cornea, but no conclusive studies of the relative importance of the various inflammatory cell proteases has been performed in this model. As described in WO 95/24921, the combination of GM6001 and Aprotinin has also been used successfully to inhibit contraction of tissue.

### Trophoblast Invasion

Another example of invasive remodelling can be seen in the invasion of the trophoblast which takes place after the fertilised egg enters the uterus. In this process there is a rapid proliferation of embryonic cells which invade the uterus and the uterine wall where it forms the placenta (Harvey, 1995). The parallels between tissue remodelling in trophoblast invasion and tumour invasion are established as a consisting of a cone of trophoblasts is formed on the blastocyst after adhesion to the uterine epithelium. Trophoblasts penetrate the uterine decidua, traverse the membrane structures of the first third of the myometrium and reach down to the maternal blood vessels, where invasion stops and placentation becomes dominant, so that a new bed of capillary network is formed to support rapid growth of the developing embryo.

Like invasive cells in tumours, invasive trophoblasts express high levels of urokinase (Hofmann, 1994) and metalloproteases (Salamonsen, 1995), and hormone modulators and protease inhibitors can inhibit implantation. Some reports have claimed that both urokinase and metalloproteases contribute equally to trophoblast invasion *in vitro* (Yagel, 1988), others that metalloprotease activity (MMP-9) contributes most (Librach, 1991), and still others that urokinase has no role at all (Behrendtsen, 1992). While there are indications that the hormone chorionic gonadotropin retards trophoblast invasion in an *in vitro* model by reducing the activity of both urokinase and collagenase (Yagel, 1993), there exists no indications in the art of the relative importance of these two systems in the invasiveness of trophoblasts, let alone of malignant neoplasms.

Other examples of physiological tissue remodelling include mammary gland involution after lactation (Feng, 1995), and uterine involution after giving birth. These processes have also been shown to be dependent on expression of increased levels of matrix-degrading proteases, as in the invasive remodelling of malignant tumours.

### Prior Art: Experimental Treatments using Protease Inhibitors

The successful topical use of a protease inhibitor mixture to heal eye injuries has been alluded to above (Schultz et al 1992). However the successful topical use of such a protease inhibitor mixture to protect matrix substratum relies on the possibility of immediate access to the site of its desired action. It should be noted that prior art methods using topical application do not enable, let alone suggest nor render probable, the successful interference with invasive remodelling generally in an animal when such remodelling occurs internally in body viscera and compartments; immediate access in such cases (eg. tumours) can only be gained through the blood circulation. Thus different methods of administration, doses and schedules of selected protease inhibitors of appropriate specificity have to be defined in order to bring about successful inhibition of invasive tissue remodelling through the systemic route of treatment. In this context, it is instructive to briefly review the extensive literature on systemic treatment of experimental animal tumour models with protease inhibitors.

The serine protease inhibitor aprotinin (Trasylol™, Bayer) and the plasmin inhibitor tranexamic acid (Cyclokapron™, Kabi) have received much attention in the past, as they efficiently block the effects of plasminogen activation and have little or no systemic toxic side-effects over a wide dosage range. Aprotinin at very low concentrations complexes tightly with plasmin in such a way that the enzyme is inactivated, while tranexamic acid blocks binding of plasminogen and plasmin to cell surface and fibrin, so that plasminogen activation, plasmin-mediated activation of pro-urokinase and ultimately fibrinolysis can potentially be inhibited.

Aprotinin was many times reported to reduce the growth rate and/or the spread of transplantable tumour cells in several in vivo animal tumour models (e.g. Lage, 1978; Ohkoshi, 1980). However it is notable that in these reports it was never found that systemic aprotinin treatment could completely arrest tumour growth *in vivo*, let alone block formation of metastases. In fact it was noted that in some cases aprotinin actually increased the number of metastases.

Tranexamic acid has also been tested as a systemic treatment in experimental animal tumour models, and growth inhibition was often reported, sometimes associated with fibrin deposition (Tanaka, 1982), but never complete arrest of tumour progression. Tranexamic acid has also been used to systemically treat human patients with malignant tumours, principally in the studies of Åstedt and coworkers (Åstedt, 1977). At first some promising results were obtained, including fibrous encapsulation of ovarian tumours (Åstedt, 1980), but evidently these positive effects in a few case reports were not always obtained or sustained and tranexamic acid was not recommended for widespread treatment of cancer. Similarly, attempts at inhibition of uPA, and inhibition of uPA interaction with its specific receptor have produced some but not complete tumour control *in vivo* (see Danø *et al.*, 1985 and 1994).

Recently a powerful method has become available to elucidate the physiological and pathological roles of specific proteins in the living organism. This powerful method is targeted gene inactivation, giving rise to animals which specifically and totally lack the expression of a protein of interest (socalled "knock-out" animals). For example, mice have been produced which are completely lacking in plasminogen (plg^{-/-} mice; Bugge, 1995). The mere fact that homozygous plg^{-/-} mice are born at all is definitive evidence that plasmin is not in itself essential for the process of trophoblast invasion, a finding which is not surprising *vis a vis* the knowledge of the limited effect of plasmin inhibition on the invasiveness of tumours and of the similarities between trophoblast invasion and tumour invasion (cf. the above). Therefore, for many, the existence of these plg^{-/-} mice has further removed focus from the plasminogen/plasmin system in the ongoing investigations of the invasive properties of malignant neoplasms.

Suitable inhibitors of metalloproteases with sufficient potency and low toxicity for use *in vivo* appear not to have been available until the advent of N-{2R-2-(hydroxyamidocarbonymethyl)-4-methylpentanoyl)}-L-tryptophan methylamide (in the following: Galardin™; also known as GM-6001: Grobelny, 1992), which made it possible for the first time to inhibit the action of metalloproteases *in vivo*. GM-6001 was first tested as a topical treatment for corneal injury (Schultz et al, 1992; see above).

Galardin™ has since been found to inhibit both the invasive properties of a gelatinase secreting glial tumour cell line *in vitro* (Boghaert, 1994) and tumour extract stimulated neo-angiogenesis of the cornea (Galardy, 1994) *in vivo* following systemic Galardin™ administration. However its potential ability to systemically inhibit tumour growth and metastasis in animals has to our knowledge not been reported yet.

Other synthetic metalloprotease inhibitors have now been prepared using the common theme of zinc chelation, and promising results have already been obtained in systemic inhibition of metastasis of an experimental mammary carcinoma (Eccles, 1996), as well as in systemic inhibition of tumour-induced neo-angiogenesis. These results have led to promising developments in phase I-II clinical trials with Batimastat (BB-94, British Biotech) (Wojtowicz-praga, 1996), and in phase I-II trials of an orally-available related drug, Marimastat (BB-2516, British Biotech; Gore, 1996).

To our knowledge, combination treatment of experimental animal tumours with either BB-94 or BB-2516 and inhibitors of other types of proteases has not yet been reported. The validity of the earlier finding (Werb, 1977) that plasmin activates pro-collagenase has since been upheld and extended to include pro-stromelysin activation (Murphy, 1992), but additional plasmin-independent mechanisms involving membrane type metalloproteases (MT-MMPs) and applying to other members of the metalloprotease family (e.g. gelatinase A) also exist.

In conclusion, despite decades of investigations on the involvement of both serine proteases and of metalloproteases, no conclusions have yet been reached regarding the importance of these classes of enzymes and their possible relationship in the progress of invasive tissue remodelling. The general belief seems to be, that both classes of enzymes are to some extent involved, but their relative degree of importance has not been established.

Further, at present the vast majority of pharmaceuticals used in the systemic treatment of invasive diseases such as cancers are either highly toxic and/or subject the patients to massive adverse side-effects, and there is therefore a strong need for new and alternative approaches in the treatment of such diseases.

### OBJECT OF THE INVENTION

It is an object of the invention to provide relatively non-toxic pharmaceutical compositions for the inhibition of invasive tissue remodelling.

Such pharmaceuticals would, in accordance with the teachings referred to above, be effective in treatment of malignant tumours, but they would also constitute interesting alternatives to the known pharmaceutical contraceptive methods. Hence, it is also an object of the invention to provide such antineoplastic and contraceptive pharmaceuticals.

### SUMMARY OF THE INVENTION

As mentioned above mice have been produced which are completely lacking in plasminogen (plg^{-/-} mice). The present inventors have used such plg^{-/-} mice as a model system in order to investigate a possible relationship between the functions of the plasminogen/plasmin and metalloprotease systems in invasive tissue remodelling.

During this extensive work, the present inventors have surprisingly found that the metalloprotease inhibitor Galardin™ not only inhibits, but totally *blocks* invasive tissue remodelling associated with wound healing in plg^{-/-} mice, an observation which cannot be duplicated in wildtype (plg^{+/+}) mice; whereas untreated plasminogen deficient mice (plg^{-/-}) as well as normal (plg^{+/+}) mice treated with Galardin™ are always able to heal skin wounds, although there is in both cases a strong delay, plg^{-/-} mice treated with Galardin™ are all completely unable to heal wounds, cf. Example 1. In other words,.the simultaneous absence of the actions of plasmin and of the metalloproteases inhibited by Galardin™ has surprisingly been demonstrated to abolish wound healing, *i.e*. produce a strongly synergistic effect in a situation where no more than an additive effect would have been expected. Furthermore, similar effects have been demonstrated in other tissue remodelling processes where normal pregnancy, post- lactational mammary gland involution, and uterine involution after parturition were virtually abolished in plg^{-/-} mice upon administration of an effective amount of the metalloprotease inhibitor galardin.

In this connection it should be noted that, since the plg^{-/-} mice exhibit a fertility which is approximately the same as that of wild-type mice, it is a proven fact that a complete block of the actions of plasmin alone does not have any detectable influence on trophoblast invasion, cf. the above. Furthermore, as shown in Example 2, the inhibition by Galardin™ of metalloproteases in wild-type mice does not have any significant effect on their fertility. Hence, it was not to be expected *à priori* that the combined inhibition of plasmin and metalloproteases would have any significant effect on trophoblast invasion.

Not only do these new findings by the inventors provide the first evidence for redundancy with respect to the functions of plasmin, i.e. that at least one of the physiological effects of plasmin is duplicated by other proteolytic enzymes (*i.e.* the physiological effect related to invasive tissue remodelling) on a molecular level, but it also opens the doors to a number of therapeutical methods in the treatment of malignancies wherein relatively non-toxic enzyme inhibitors will be used instead of the highly toxic traditional chemotherapeutical agents.

In summary, it has surprisingly been found by the present inventors that the protein cleaving actions of plasmin seem to be duplicated by certain metalloproteases and that by simultaneously inhibiting the actions of both plasmin and the metalloproteases in question, it becomes possible to obtain a synergistic effect which is much more far-reaching than a simple additive effect of the two types of inhibition (cf. the examples).

In a further extension of the discovery, the inventors have mimicked the effect of plasminogen deficiency by treatment with a combination of two plasmin inhibitors which they find can delay skin wound healing substantially although not to the same extent as plasminogen deficiency. Studies on the pharmacokinetics of one of these plasmin inhibitors suggest that this at least in part is due to a very short half-life of this inhibitor and subsequent studies on the scheduling support this assumption because the same total dose divided in up to five daily administrations delays the wound healing process significantly more than when it is divided into three or four daily administrations.

In accordance with these findings it was found that combination of the metalloprotease inhibitor and the plasmin inhibitors lead to a delay in wound healing which was at least additive, but on the other hand not complete. This was interpreted as the residual plasmin activity being sufficient to complete wound healing even in the presence of the metalloprotease inhibitor. The combination of metalloprotease inhibitors with plasmin inhibitors did not lead to any detectable decrease in the rate of normal pregnancies in contrast to the complete abolition observed with a combination of the metalloprotease inhibitor and plasminogen deficiency, the conclusion being that the residual plasmin activity was sufficient for normal implantation even in the presence of the metalloprotease inhibitor.

Furthermore, treatment of the transplanted highly metastatic Lewis lung tumour with the metalloprotease inhibitor and with the two plasmin inhibitors showed that each of these two treatment regimens lead to a substantial delay in tumour growth, and when combined there was a strong additive effect of the two regimens on this parameter. Both regimens also alone prolonged the survival of the mice, an effect which was also observed in the group of mice which received the combination of the regimens. In this latter group there were surprisingly three out of 17 mice that were long-term survivors (more than 300 days) in contrast to no long-term survivors in either of the two groups treated with only one type of inhibitor and likewise no long-term survivors in the mock-treated group. The combined treatment with the two types of inhibitor thus lead to a clear synergistic effect with respect to survival. It is contemplated that in accordance with the wound healing studies, a survival of all mice may be obtained by a combination of metalloprotease inhibition and complete abolition of plasmin activity e.g. by using sufficiently inbred plasminogen deficient mice in such studies, when such-mice become available.

Hence, in one broad and general aspect the invention pertains to the use of a composition for the manufacture of a medicament for the prevention or arrest of invasive remodelling in a mammal. The composition effects 1) inhibition or abolition, in the mammal, of the *in vivo* protein cleaving actions of plasmin as well as of active derivatives thereof and 2) simultaneously therewith inhibiting or abolishing the *in vivo* protein cleaving actions of at least one proteolytic enzyme which is different from plasmin as well as from active derivatives thereof and which exerts its action on at least one extracellular protein, said at least one proteolytic enzyme being non-murine (including human) analogue(s) of murine enzyme(s) which, isolated or in combination, is/are essential for embryo implantation and/or wound healing in Plg^{-/-} mice but not in wildtype mice.

It is preferred that the *in vivo* protein cleaving actions of plasmin (and the active derivatives thereof) and/or of the at least one proteolytic enzyme different from plasmin are substantially abolished, since even a small residual activity may have the effect that invasive remodelling can be accomplished.

As will be explained in detail below, the inhibition and/or abolishment of the protein cleaving actions of both classes of enzymes may be effected in a number of ways but one interesting embodiment of the invention is the use of the compositon for the manufacture of a medicament that can be used for administering, preferably systemically, to the mammal, an effective amount of a combination of 1) at least one first substance which, in the mammal, effects inhibition of the in vivo protein cleaving actions of plasmin as well as of active derivatives thereof, and 2) at least one second substance which, in the mammal, effects inhibition of the in vivo protein cleaving actions of at least one proteolytic enzyme which is different from plasmin as well as from active derivatives thereof and which exerts its action on at least one extracellular protein, said second substance being one(s) which, upon administration in an effective amount, result(s) in a significantly higher inhibition of embryo implantation and/or wound healing in Plg^{-/-} mice than in wildtype mice,

the at least one first and second substances being administered, preferably systemically, either simultaneously or with such an interval that they both are simultaneously present in concentrations which effects substantial *in vivo* inhibition, preferably blocking, of their respective target proteases.

The term "preventing" denotes a prophylactic event, *i.e.* cells which would have exhibited invasive remodelling if not the inventive composition used in a method had been exercised will not exhibit the invasive remodelling upon effective instigation of the inventive composition, whereas the term "arresting" is used in connection with remodelling which already is invasive, but which is stopped as a consequence of application of the inventive composition.

The term "inhibiting" has its usual meaning in the present context, i.e. a partial or total block of the protein cleaving actions of the relevant enzyme. In contrast, when "abolishing" the protein cleaving actions of either of the two groups of enzymes, no residual enzymatic activity should be detectable upon instigation of the inventive treatment. As explained above, this is a preferred embodiment of the inventive compositions, since the experiments disclosed herein unambiguously demonstrates that in order to obtain prevention/arrest of invasive remodelling in two model systems (implantation and wound healing) it is necessary that at least the activities of plasmin and its active derivatives are abolished.

If one of the enzyme activities are in fact abolished, notably that of plasmin or its derivatives, side effects are expected to occur, e.g. ligneous conjunctivitis. Therefore, in such a situation care should be taken to alleviate these side effects, e.g. by administering locally to the patient, plasminogen. One example would be to supply plasminogen directly to the eye in order to avoid ligneous conjunctivitis or as a aerosol to the airways to avoid airway obstructions due to the mucus becoming to viscous. At any rate, the side effects have been shown to be reversible.

The term "invasive remodelling" (or "invasive tissue remodelling") refers in general to processes characterized by cell migration, activation and/or release of growth factors, invasion and tissue degradation, such as associated with the growth of malignant tumours, the invasion of uterine endometrium by trophoblasts of an implanting embryo, the migration of reparative keratinocytes in a healing wound, and infiltration of a site of chronic inflammation by macrophages, neutrophils and T-lymphocytes, and certain bacterial and/or fungal and/or protozoan and/or viral infections which are accompanied by tissue degradation (e.g. necrotizing fascietis caused by certain *Streptococcus* strains as well as Lyme disease caused by *Borrelia burgdorferi sensu lato*) .

By "simultaneous" inhibition/abolishment is herein meant that both types of enzymatic activity are inhibited at the same time. In essence, it is preferred that said simultaneous inhibition/abolishment is sustained for a longer continuous period in order to ensure that the invasively remodelling cells will find no "window in time" where the invasive remodelling can be resumed. Hence, it is necessary to determine the pharmacologically effective dosages and concentrations of e.g. substances used in order to achieve the effects and thereafter it is also necessary to make sure that said concentrations are kept at the necessary level in order to sustain the simultaneous action; for further details, cf. the discussion below of determination of effective concentrations of substances used according to the invention.

When discussing "active derivatives" of plasmin is meant the naturally occurring derivatives of plasmin which can be found *in vivo*. Most of these are products of proteolytic cleavage of plasmin and a notable example is miniplasmin.

The term "extracellular protein" means a protein which constitutes part of the extracellular phase. Preferred examples are extracellular matrix proteins present in the extracellular matrix wherein cells are embedded, fibrin, and growth factors. Such proteins are well-known in the art and encompass various forms of collagen, elastin, and proteoglycans. Preferred examples of extracellular proteins, the proteolysis of which should be inhibited, are fibrin, fibronectin, laminin, transforming growth factor β (TGFβ), TNFα, basic fibroblast growth factor, and precursors TGF*β* or of other growth factors.

The "at least one proteolytic enzyme which is different from plasmin as well as from active derivatives thereof" is any other proteolytic enzyme which exerts an *in vivo* cleaving effect on extracellular proteins which are also proteolytically acted upon or cleaved by plasmin. Specific examples of such proteolytic enzymes are discussed in detail below.

The above-mentioned "non-murine (including human) analogues of murine enzymes" are those homologous proteins which are found in a non-murine system. Thus, if the murine proteolytic enzyme in question is e.g. the metalloprotease MMP-3, then the human analogue thereof is the metalloprotease which in man shares the same group of substrates and which at the same time has a sequence homology (and is encoded by a homologous gene) which would define it as the closest homologue in a human system to that in a mouse.

When stating that a murine analogue of the proteolytic enzyme in question is "essential" for wound healing or implantation in a Plg^{-/-} mouse is simply meant that a certain level of activity of the murine analogue of the proteolytic enzyme(s) is/are necessary for wound healing or implantation to occur in such a mouse. As demonstrated by the examples, metalloproteases inhibited by Galardin™ are necessary for implantation and wound healing, respectively, in Plg^{-/-} mice, but not in wildtype (Plg^{+/+}) mice. In other words, when plasmin is totally absent, the role of the metalloproteases inhibited by Galardin™ becomes all-important for these two physiological cell-invasive events to occur.

It has to the best of the inventors' knowledge never been demonstrated before that a combination of substantially total inhibition of on the one hand the plasminogen/plasmin system and on the other hand a set of metalloproteases could lead to total abolishment of invasive remodelling and since such a total abolishment in the case of cancer renders a malignant tumour benign, this novel approach is believed to have great promise in future strategies for combatting cancer.

### DETAILED DISCLOSURE OF THE INVENTION

As mentioned above, it is preferred to administer substances which will inhibit and or abolish protein cleavage effected by plasmin and its derivatives on the one hand and other proteolytic enzymes on the other. It will be understood, however, that such a set of pharmaceutical effects might be incorporated into one single substance and hence the invention also pertains to a composition of the invention wherein an effective amount is to be administered of at least one third substance which, in the mammal, effects inhibition of both the *in vivo* protein cleaving actions of plasmin as well as of active derivatives thereof and of the *in vivo* protein cleaving actions of at least one proteolytic enzyme as defined above, said at least one third substance(s) being one(s) which, upon administration in an effective amount, result(s) in a significantly higher inhibition of embryo implantation and/or wound healing in Plg^{-/-} mice than in wildtype mice. Consequently, the first and second substances mentioned above may be such a third substance, the important thing being that a substantial inhibition is achieved in both the plasminogen/plasmin system and in the system comprising the other proteolytic enzyme.

As evidenced by the examples disclosed herein, the main candidates for proteolytic enzymes to inhibit apart from the obligate inhibition of plasmin (and its active derivatives) are the metalloproteases, *i.e.* proteolytic enzymes which are only active in the presence of certain metal ions, notably Zinc ions. Preferably, the metalloprotease is selected from the group consisting of a collagenase, a stromelysin, a gelatinase, an elastase, and a membrane type metalloprotease.

Preferred examples of collagenases are MMP-1, MMP-8, and MMP-13. Examples of preferred stromelysins are MMP-3, MMP-7, MMP-10, and MMP-11. Further, the gelatinase is preferably MMP-2 or MMP-9. The elastase is conveniently MMP-12. Finally, the membrane type metalloprotease is preferably MMP-14, MMP-15, and MMP-16.

Although the metalloproteases as mentioned above are the main candidates for proteases to inhibit, other enzymes are practical possibilities. Important examples of non-metalloproteases are cysteine proteases, notably cathepsin B and cathepsin H. The invention is, however, not limited to the inhibition of the specific examples mentioned above. It will be understood, that those proteases which should be inhibited according to the invention are envisaged to be those which on the substrate level have at least one action in common with the plasmin family of proteases, or in other words, which are able to take over when the concentration of plasmin becomes limiting *vis à vis* the full protein cleaving potential.

As mentioned above, a number of conditions are known wherein invasive remodelling plays a predominant role. According to the invention it is possible to interfere with the progress of these conditions as a consequence of the interference with the general ability of cells in question to cleave extracellular proteins.

The most, important group of conditions to treat by employing the compositions of the invention is the group of malignant neoplasms. As is well-known, the main reason for.the lethality of malignant neoplasms is the invasive character of the neoplastic cells which is reflected in their ability to cross the boundaries between different types of tissue (both by direct invasive remodelling at the site of a primary tumour and during metastasis).

Hence, according to the invention the invasive remodelling which is inhibited/arrested is preferably that of a malignant neoplasm.

Said malignant neoplasm may be any known solid tumour or tumour of the haematopoietic system, the pathogenesis of which is characterized by invasive remodelling. Preferred malignant neoplasms to be treated by the composition, according to the invention are selected from the group consisting of carcinoma such as adenocarcinoma and squamous cell carcinoma, and sarcoma such as liposarcoma, fibrosarcoma, chondrosarcoma, osteosarcoma, leiomyosarcoma, rhabdomyosarcoma, glioma, neuroblastoma, medullablastoma, malignant melanoma, neurofibrosarcoma, hemangiosarcoma, and lymphangiosarcoma. Also malignant teratoma, dysgerminoma, seminoma, and choriocarcinoma and certain systemic (*i.e.* of haematopoietic origin) neoplasms are interesting as targets for treatment, notably leukaemia such as acute leukemia (AL), chronic leukemia (CL) , T-cell acute leukemia (T-ALL), B-cell acute leukemia (B-ALL), T-cell chronic leukemia (T-CLL), B-cell chronic leukemia (B-CLL), prolymphocytic leukemia (PLL), acute undifferentiated leukemia (AUL), acute myelogenous leukemia (AML), chronic myelogenous leukemia (CML), chronic myelomonocytic leukemia (CMML), acute promyelocytic leukemia (APL), pre-B-ALL, and pro-B-ALL;
lymphoma such as Burkitt's lymphoma (BL), non-Hodgkins lymphoma (NHL), Hodgkins lymphoma (HL), follicular lymphoma (FL), diffuse large cell lymphome (DLCL), T-cell lymphoma, B-cell lymphoma; and myeolodysplasia.

The preferred neoplasms to be treated by the composition according to the invention are the carcinomas and adenocarcinomas, preferably of the lung, the breast, the prostate, and the colon, but of course also other invasive and/or metastasizing neoplasms are candidates for the treatments according to the invention.

A number of other diseases are characterized by an element of invasive remodelling. Notable members of this group are the chronic inflammatory diseases such as inflammatory bowel disease (e.g. Crohn's disease and ulcerative colitis), and the inflammatory arthritides, such as rheumatoid arthritis; certain skin conditions, such as psoriasis and pemphigus bullosa; conditions/diseases characterized by macrophage invasion, notably demyelinization of nerve tissue (e.g. multiple sclerosis and encephalitis) and arteriosclerosis; degenerative disorders of the central nervous system (e.g. Alzheimer's disease and Parkinson's disease); fungal infections such as *Candida albicans;* and certain bacterial infections with infectious agents such as *Clostridium perfringens,. Cryptococcus neoformans,* and various strains and isolates of *Yersinia* spp. and *Streptococcus* spp. Interesting aspects of the invention pertains to the use for the manufacture of a medicament of the composition of the invention for the treatment of the above-indicated diseases.

Finally, as demonstrated in Example 2, the composition of the invention is a remarkably efficacious contraceptive method which utilises a totally different strategy than the presently available hormonally based contraceptives. Hence, also use of the methods of the invention in contraception is an important embodiment of the invention.

As discussed above, a preferred embodiment of the invention includes the administration, preferably systemic, of at least one first substance and at least one second substance (and optionally at least one third substance, which may or may not be identical to either the at least one first or the at least one second substance).

Preferred examples of the at least one first substance are aprotinin, tranexamic acid, Nα-trans-4-aminomethylcyclohexane carbonyllysine 4 benzoylanilide, Na-trans-4-aminomethylcyclohexane carbonyl-O-bromobenzyloxycarbonyltyrosine-4-acetylanilide, 1-(ethoxy-carbonyloxy)ethyl trans-4-aminomethylcyclohexanecarboxylate hydrochloride (KABI 2161), alpha-2-antiplasmin, alpha-2-makroglobulin, tumour associated trypsin inhibitor, urinary trypsin inhibitor, leupeptin, pyroglutamyl-Leu-Arg-CHO, 6-aminocaproic acid, p-aminobenzamidine, bis(5-amidino-2-benzimidazolyl)methane, alpha-N-acetyl-L-lysine methyl ester, tosyl-lysine chloromethyl ketone, or Boc-D-Phe-ProBoro-Arg-OH, *i.e.* all well-known inhibitors of the plasminogen/plasmin system which may be used in vivo with acceptable toxicity.

Preferred examples of the at least one second substance are tissue inhibitor of metalloproteases (such as TIMP-1, TIMP-2, and TIMP-3), alpha-2-macroglobulin, Galardin™, N- [2R-2- (hydroxamidocarbonylmethyl)-4-methylpentanolyl]-L-tryptophan methylamide, batimastat, marimastat, Gl 129471, Gl 168, Gl 173, Gl 179, Gl 184, Cl-A, Cl-B, RP59794, SC-44463, Ro31-4724, CT1746, SCH 47890, a peptide hydroxamate (such as Pro-Leu-Gly-NHOH), LMHKPRCGVPDVGG, TNF-α releasing protease inhibitor, Zincov® , Pro-Ileu, phosphoramidon, thiorphan, tiopronin, a tetracycline, N-acetylcysteine, EDTA, or 1,10 phenanthrolene, *i.e.* known inhibitors of metalloproteases which may be used in vivo with acceptable toxicity. Further, various prodrugs of Galardin™ would also be interesting candidates.

The at least one third substance includes both of the proteolysis inhibiting activities of the first and second substances within the same chemical entity. Preferred examples are a conjugate of Galardin™ with aprotinin (e.g. an ester from the active site lysine carboxy terminus of aprotinin with a hydroxamate function of Galardin™, cf. Mehlich et al. 1988, Biochim. Biophys. Acta, 957(3): 420-429), a conjugate of Galardin™ with tranexamic acid (e.g. a spacer linkage between an amide of Galardin™ and an amino group of tranexamic acid, and a conjugate of Galardin™ with leupeptin (e.g. an amide formed from a carboxy function of Galardin™ and the amino terminus of leupeptin).

According to the invention, it is not only an interesting possibility to achieve abolishment or inhibition by directly inhibiting the proteases themselves, but also by inhibiting possible proenzymes thereof (e.g. proMMP), inhibiting possible activation of the proteases (e.g. inhibition of uPA or tPA [tissue-type plasminogen activator), inhibiting translation of mRNA encoding the enzymes/proenzymes, and inhibiting the transcription of DNA into RNA encoding the enzymes proenzymes. All these strategies may be used alone or in combination.

Specifically, inhibition of the production of the enzymes/proenzymes can be achieved in a number of ways which may in fact be more effective in producing a total block of the production of the relevant enzymes or their active forms (both the plasmin family and the other proteolytic enzymes to be blocked). Prominent examples would be the blocking of formation of plasmin by activation of plasminogen, this blocking being achieved by inhibition of either uPA or tPA or notably by inhibition of the interaction between uPA and its cell surface receptor, uPAR (Danø *et al.* (1994), Fibrinolysis 8: 189-203, WO 90/12091, EP-A-0 691 350, WO 92/07083). In this context it is interesting to note that mice totally deficient in uPAR exhibit no visible side effects.

For instance, the application of antisense technology wherein antisense nucleotide fragments (or even PNA fragments) are utilized which will block translation of mRNA to protein is believed to provide an effective blocking of the proteolytic effects of the relevant proteins. Since sequence information exists for virtually all known metalloproteases and since the components of the plasminogen/plasmin system are well-known, it is a question of mere routine work by the skilled person to provide the relevant antisense nucleic acid fragments in order to institute a block of transcription of the protein in question.

Similarly, ribozyme technology may provide the same results by targeting an RNase to the relevant mRNA sequence and thereby break down the targeted mRNA and ensure that translation will not occur.

Also, various gene transfer techniques can be employed. For instance, the introduction of an inducible repressor (e.g. acting on the level or the activity of transcription factors) which controls a gene of interest encoding one of the proteolytic enzymes would provide the option of selectively shutting off the production of the proteolytic enzyme. Alternatively, the introduction of an inducible gene sequence which encodes an anti-sense RNA for one of the relevant proteolytic enzymes is also an interesting possibility.

Another part of the invention pertains to compositions, e.g. pharmaceutical compositions, which comprises 1) at least one first substance as defined above and 2) at least one second substance as defined above, the relative amounts of the at least first and at least second substances being so related that the simultaneous or sequential administration of an effective amount thereof to the mammal will lead to effective prevention or arrest of invasive remodelling in said mammal. Of course, a composition of the invention may also comprise at least one third substance as defined and discussed above.

Finally, the first, second and third substances defined above may, according to the invention, be utilized in the production of compositions adapted for the treatment of any of the conditions discussed above, notably for the treatment of malignant neoplasms, as well as in the production of compositions for contraception.

In addition to the specific substances mentioned above, derivatives thereof which show an activity of the same kind as the substance specifically mentioned are also useful for the purpose of the present invention. The kind of derivatives which come into consideration will, of course, depend on the specific character of the substance in question, but as general examples of derivatives which may be relevant for many of the substances may be mentioned introduction of or change of alkyl substituents (typically with a chain length from one to five carbon atoms) on aliphatic chains, cycloalkanes, aromatic and heterocyclic ring systems, introduction of or change of substituents such as halogens or nitro groups, change of ring size for cycloalkanes, change of aromatic or heterocyclic ring systems, change of alkyl substituents on O-and N-atoms, change of the alcohol part of ester groups, and bioisosteric replacement of functional groups, especially use of carboxylic acid bioisosteres such as phosphonic acids, phosphinic acids, tetrazoles, 3-hydroxyisoxazoles, sulphonamides and hydroxamic acids. Salts of acidic or basic compounds will be equally useful compared to the free acids or free bases. In case of racemic compounds, can racemates as well as pure enantiomers and diastereoisomers be used, and in the case of substances interacting with antagonist action be required. Of course, derivatives to be used should be derivatives which, in addition to their desired activity, show an acceptably low toxicity, and, in general, the derivates should, just as the substances themselves, be pharmaceutically acceptable.

The substance used according to the invention may be administered as such or in the form of a suitable prodrug thereof. The term "prodrug" denotes a bioreversible derivative of the drug, the bioreversible derivative being therapeutically substantially inactive *per se* but being able to convert in the body to the active substance by an enzymatic or non-enzymatic process.

Thus, examples of suitable prodrugs of the substances used according to the invention include compounds obtained by suitable bioreversible derivatization of one or more reactive or derivatizable groups of the parent substance to result in a bioreversible derivative. The derivatization may be performed to obtain a higher bioavailability of the active substance, to stabilize an otherwise unstable active substance, to increase the lipophilicity of the substance administered, etc.

Examples of types of substances which may advantageously be administered in the form of prodrugs are carboxylic acids, other acidic groups and amines, which may be rendered more lipophilic by suitable bioreversible derivatization. As examples of suitable groups may be mentioned bioreversible esters or bioreversible amides. Amino acids are typical examples of substances which, in their unmodified form, may have a low absorption upon administration. Suitable prodrug derivatives of amino acids will be one or both of the above-mentioned types of bioreversible derivatives.

For the administration to a patient, a substance having any of the activities as defined above or a prodrug thereof is preferably formulated in a (pharmaceutical) composition containing one or more substances having any of the activities as defined above or prodrugs thereof and one or more pharmaceutically acceptable excipients.

The composition comprising a combination of substances according to the invention serves as a drug delivery system. In the present context the term "drug delivery system" denotes a composition (a pharmaceutical formulation or a dosage form) which upon administration presents the active substance to the body of a human or an animal. Thus, the term "drug delivery system" embraces plain compositions as well as more sophisticated formulations and the composition may be in form of, e.g., a liquid, a spray, a solution, a dispersion, a suspension, an emulsion, tablets, capsules, pills, powders, granulates, gels including hydrogels, lotions, pastes, ointments, creams, drenches, dressings, hydrogel dressings, hydrocolloid dressings, films, foams, sheets, bandages, plasters, delivery devices, suppositories, enemas, implants, aerosols, microcapsules, microspheres, nanoparticles, liposomes, and in other suitable form.

The choice of pharmaceutically acceptable excipient(s) in a composition according to the invention and the optimum concentration thereof cannot generally be predicted and must be determined on the basis of an experimental evaluation of the final composition. However, methods for this are generally well-known in the art and the compositions may be formulated according to conventional pharmaceutical practice, see, e.g., "Remington's Pharmaceutical Sciences" and "Encyclopedia of Pharmaceutical Technology", edited by Swarbrick, J. & J. C. Boylan, Marcel Dekker, Inc., New York, 1988.

Thus, the substance or substances and/or prodrugs thereof to be administered may be formulated in the compositions in pharmaceutically acceptable media, the character of which are adapted to the chemical character of the substance. The compositions may be adapted for administration by any suitable method, for example by parenteral (such as intravenous and intraarterially), intraperitoneal, intramuscular, subcutaneous, intradermal, oral, buccal, sublingual, nasal, rectal or transdermal administration.

In a composition according to the invention, the combination of substances are generally present in a concentration in a range of from about 0.01% to about 99.9% w/w.

The concentration of the combination in a composition depends on the nature of the second compound in question, its potency, the severity of the disease to be prevented or treated, the age and condition of the patient, and potential side effects. Methods applicable to selecting relevant concentrations of the combination in the composition are well known to a person skilled in the art and may be performed according to established guidelines for good clinical practice (GCP) or Investigational New Drug Exemption ("IND") regulations as described in e.g. Drug Applications, Nordic Guidelines, NLN Publication No. 12, Nordic Council on Medicines, Uppsala 1983 and Clinical Trials of Drugs, Nordic Guidelines, NLN Publication No. 11, Nordic Council on Medicines, Uppsala 1983. A person skilled in the art would, by use of the methods described in standard textbooks, guidelines and regulations as described above as well as common general knowledge within the field, be able to select the exact dosage regimen to be implemented for any combination and/or selected active substance and dosage form using merely routine experimentation procedures.

However, it is relatively simple to titrate the useful dosages of the substances used according to the invention. If one starts out with the first substance, and by use of techniques known in the art determines the maximum pharmacologically acceptable concentration, it is preferred that the at least one first substance is administered in an amount which gives rise to a concentration which is at or below the maximum pharmacologically acceptable concentration of said first substance alone (both systemically and locally), and, when the concentration is below the maximum pharmacologically acceptable concentration of the first substance alone, said concentration is one which gives rise to substantially the same inhibition as the maximum pharmacologically acceptable concentration of the first substance alone, and, upon a similar titration, the at least one second substance is administered in an amount which gives rise to a concentration which, when the second substance is administered simultaneously with or after the administration of the at least one first substance, is at or below the maximum pharmacologically acceptable concentration (both systemically and locally), and which, if below the maximum pharmacologically acceptable concentration, is a concentration which gives rise to substantially the same inhibition as the maximum pharmacologically acceptable concentration.

A similar consideration can of course be applied when setting out from the at least one second substance and thereafter titrating the optimum amount of the first substance.

For the at least one third substance defined above, it should preferably be administered, upon a similar titration, in an amount which gives rise to a concentration at or below the maximum pharmacologically acceptable concentration of the third substance alone (both systemically and locally), and, when the concentration is below the maximum pharmacologically acceptable concentration of the third substance alone, said concentration is one which gives rise to substantially the same inhibition as the maximum pharmacologically acceptable concentration of the third substance alone.

By using the above-indicated scheme for determining the useful concentrations of first and second substances, it is ensured that the pharmacological effect of any of the substances will always be substantially the same as that achieved when using the maximum pharmacologically acceptable concentration. It will be understood, however, that after titration of the substances according to these rules, it might still be possible to obtain the maximum pharmacological effect of the "starting substance" in the titration. For instance, when beginning with the first substance, and having titrated a useful concentration of the second substance as indicated above, the first substance can also be administered in an amount which, when it is administered simultaneously with or after the administration of the second substance, gives rise to a concentration which results in substantially the same inhibition as the maximum pharmacologically acceptable concentration. Of course, also this scheme may be inverted with respect to the starting substance in the titration.

In general, the physiologically acceptable substances or prodrugs are normally administered in a daily dosage of between 1 mg and 1000 mg for a grown-up person, usually between 10 mg and 800 mg, such as between 10 mg and 400 mg orally, or an intravenous, subcutaneous or intramuscular dose of between 0.1 mg and 100 mg, preferably between 0.1 and 50 mg, such as between 1 mg and 25 mg of the substance. The substance or prodrug is preferably administered 1 to 4 times daily. As mentioned above, the administration is normally aimed at maintaining a therapeutically effective plasma concentration of the substance for at least one month, preferably at least two months or at least three months. Controlled release type compositions will often be suitable for maintaining an effective serum concentration with a small number of daily unit dosages. As can be seen from the examples in the present application, the in vivo half-lifes of aprotinin and traneximic acid are relatively short and therefore the effective dosage regimens for these inhibitors seem to be of high importance. Methods known to the skilled persons for preparing controlled release compositions will thus enable a reasonable dosage scheme and thereby avoid e.g. the need for continuous or short-interval administration of the short-lived inhibitors. An attractive alternative approach for ensuring a maintained effective concentration level is to use continuous administration of the inhibitors or their precursors: this can e.g. be done by providing a drug-pump (as known in the art of insulin delivery) which will continuously deliver the drug to the subject in need thereof.

When the compositions of the invention are in unit dosage form (such as a tablet, capsule or ampoule), such unit dosage forms will normally contain from 1 to 1000 mg (and for parenteral administration from 0.1 to 25 mg) of a substance or prodrug used according to the invention, calculated as the free active substance.

In the following is given a general review over relevant compositions according to the invention. The review is based on the particular route of administration. However, it is appreciated that in those cases where a pharmaceutically acceptable excipient may be employed in different dosage forms or compositions, the application of a particular pharmaceutically acceptable excipient is not limited to a particular dosage form of a particular function of the excipient.

### Compositions for parenteral use

Examples of parenteral compositions are solutions or suspensions of the substances or prodrugs in a sterile aqueous carrier or parenterally acceptable oil, such as polyethylene glycol, polyvinyl pyrrolidone, lecithin, arachis oil or sesame oil. The compositions may contain pharmaceutically acceptable auxiliary substances as required to approximate physiological conditions, such as buffering agents, wetting agents, detergents, and the like. Additives may also include additional active ingredients, e.g. bactericidal agents, or stabilizers. If desired, the solution or suspension can be lyophilized and reconstituted with a suitable carrier such as a sterile aqueous carrier prior to administration.

### Compositions for oral use

Substances which are suitable for oral administration may be formulated in solid dosage forms such as, e.g., powders, granules, sachets, tablets, capsules, effervescent tablets, chewable tablets, lozenges, immediate release tablets, and modified release tablets as well as in fluid or liquid formulations such as, e.g. powders, dispersible powders, or granules suitable for preparation of an aqueous suspension by addition of an aqueous medium, emulsions, dispersions, and mixtures.

For oral administration, a pharmaceutically acceptable non-toxic composition may be formed by incorporating normally used excipients, such as those carriers listed below, and generally 1-95% of active ingredient, that is, a substance used according to the invention or a prodrug thereof, often preferably 25-75% of the substance of the prodrug.

A liquid composition will normally comprise a suspension or solution of the substance in a suitable liquid carrier or suitable liquid carriers, for example an aqueous solvent such as water, ethanol or glycerol, or a non-aqueous solvent, such as polyethylene glycol or an oil. The composition may also contain a suspending agent, preservative, flavouring or colouring agent.

In solid form, the composition contain the combination and any further active substance optionally in admixture with one or more pharmaceutically acceptable excipient. These excipients may be, for example,
inert diluents or fillers, such as sucrose, sorbitol, sugar, mannitol, microcrystalline cellulose, starches including potato starch, calcium carbonate, sodium chloride, lactose, calcium phosphate, calcium sulfate, or sodium phosphate; granulating and disintegrating agents, for example, cellulose derivatives including microcrystalline cellulose, starches including potato starch, croscarmellose sodium, alginates, or alginic acid;
binding agents, for example, sucrose, glucose, sorbitol, acacia, alginic acid, sodium alginate, gelatin, starch, pregelatinized starch, microcrystalline cellulose, magnesium aluminum silicate, carboxymethylcellulose sodium, methylcellulose, hydroxypropyl methylcellulose, ethylcellulose, polyvinylpyrrolidone such as, e.g., PVP K12, PVP K15, PVP K17, PVP K25, PVP K30, PVP K60, PVP K90, or PVP K120, or combinations thereof, polyvinylacetate, or polyethylene glycol; and
lubricating agents including glidants and antiadhesives, for example, magnesium stearate, zinc stearate, stearic acid, silicas, hydrogenated vegetable oils, or talc.

Other pharmaceutically acceptable excipients can be colorants, flavouring agents, plasticizers, humectants, buffering agents, etc.

In those cases where the composition for oral use is in the form of a solid dosage form in unit dosage form (e.g. a tablet or a capsule), the unit dosage form may be provided with a coating like one or more of the coatings mentioned below.

In those cases where the composition is in the form of a tablet, capsule or a multiple unit composition, the composition or the individual units or a tablet or a capsule containing the individual units may be coated e.g. with a sugar coating, a film coating (e.g. based on hydroxypropyl methylcellulose, methylcellulose, methyl hydroxyethylcellulose, hydroxypropylcellulose, carboxymethylcellulose, acrylate copolymers (Eudragit), polyethylene glycols and/or polyvinylpyrrolidone) or an enteric coating (e.g. based on methacrylic acid copolymer (Eudragit), cellulose acetate phthalate, hydroxypropyl methylcellulose phthalate, hydroxypropyl methylcellulose acetate succinate, polyvinyl acetate phthalate, shellac and/or ethylcellulose). Furthermore, a time delay material such as, e.g., glyceryl monostearate or glyceryl distearate may be employed.

### Compositions for application to mucosal surfaces

For application to the nasal mucosa, nasal sprays and aerosols for inhalation are suitable compositions according to the invention. In a typical nasal composition, the combination is present in the form of a particulate formulation optionally dispersed in a suitable vehicle. In the case of aerosols, the substance or prodrug is preferably supplied in finely divided form along with a surfactant and propellant. In general, the pharmaceutically acceptable vehicles and excipients and optionally other pharmaceutically acceptable materials present in the composition such as diluents, enhancers, flavouring agents, preservatives, etc. are all selected in accordance with conventional pharmaceutical practice in a manner understood by the persons skilled in the art of formulating pharmaceuticals.

Typical percentages of the substance or prodrug in a nasal composition are 0.01-20% by weight, preferably 1-10%. The surfactant must, of course, be non-toxic, and preferably soluble in the propellant. Representative of such surfactants are the esters or partial esters of fatty acids containing from 6 to 22 carbon atoms, such as caproic, octanoic, lauric, palmitic, stearic, linoleic, linolenic, olesteric and oleic acids with an aliphatic polyhydric alcohol or its cyclic anhydride such as, for example, ethylene glycol, glycerol, erythritol, arbitol, mannitol, sorbitol, the hexitol anhydrides derived from sorbitol, and the polyoxyethylene and polyoxypropylene derivatives of these esters. Mixed esters, such as mixed or natural glycerides may be employed. The surfactant may constitute 0.1-20% by weight of the composition, preferably 0.25-5%. The balance of the composition is ordinarily propellant. Liquified propellants are typically gases at ambient conditions, and are condensed under pressure. Among suitable liquified propellants are the lower alkanes containing up to 5 carbons, such as butane and propane; and preferably fluorinated or fluorochlorinated alkanes. Mixtures of the above may also be employed. In producing the aerosol, a container equipped with a suitable valve is filled with the appropriate propellant, containing the substance according to the invention and surfactant. The ingredients are thus maintained at an elevated pressure until released by action of the valve.

After administration of a nasal composition according to the invention, the active substance may be adsorbed on the nasal mucosa. The adsorption on the mucosa is believed to lead to a less irritative effect than when, e.g., a liquid vehicle, e.g. containing a penetration enhancer or promoter, is employed.

Compositions for buccal or sublingual administration are, for example, tablets, lozenges and pastilles, in which the substance or the prodrug is formulated with a carrier such as sugar and acacia, tragacanth, or gelatin and glycerol. Compositiohs for rectal administration are suitably in the form of suppositories containing a suppository base such as cocoa butter. Compositions for transdermal application are for example ointments, gels and transdermal patches.

For application to the rectal or vaginal mucosa, suitable compositions according to the invention include suppositories (emulsion or suspension type), enemas, and rectal gelatin capsules (solutions or suspensions). Appropriate pharmaceutically acceptable suppository bases include cocoa butter, esterified fatty acids, glycerinated gelatin, and various water-soluble or dispersible bases like polyethylene glycols and polyoxyethylene sorbitan fatty acid esters. Various additives like, e.g., enhancers or surfactants may be incorporated.

### Compositions for topical application

For application to the skin, the formulations according to the invention may contain conventionally non-toxic pharmaceutically acceptable carriers and excipients including microspheres and liposomes. The compositions include creams, ointments, hydrophilic ointments, lotions, liniments, gels, hydrogels, solutions, suspensions, sticks, sprays, pastes, plasters, films, powders, soaps, shampoos, jellies, dressings such as absorbent dressings, pads, bandages, foams, plasters, and transdermal drug delivery systems.

The pharmaceutically acceptable excipients may include emulsifying agents, antioxidants, buffering agents, preservatives, humectants, penetration enhancers, chelating agents, gelforming agents, ointment bases, perfumes, and skin protective agents.

Examples of emulsifying agents are naturally occurring gums, e.g. gum acacia or gum tragacanth; naturally occurring phosphatides, e.g. soybean lecithin; sorbitan monooleate derivatives; wool fats; wool alcohols; sorbitan esters; monoglycerides; and fatty alcohols.

Examples of antioxidants are butylated hydroxy anisole (BHA), ascorbic acid and derivatives thereof, tocopherol and derivatives thereof, butylated hydroxy anisole, and cysteine.

Suitable examples of preservatives for use in compositions according to the invention are parabens, such as methyl, ethyl, propyl p-hydroxybenzoate, butylparaben, isobutylparaben, isopropylparaben, potassium sorbate, sorbic acid, benzoic acid, methyl benzoate, phenoxyethanol, bronopol, bronidox, MDM hydantoin, iodopropynyl butylcarbamate, EDTA, propyleneglycol (increases the solubility of preservatives) benzalconium chloride, and benzylalcohol, or mixtures of preservatives like Germaben II & IIE (mixture of imidazolidinyl urea, methyl and propyl parabens and propylene glycol; available from ISP-Sutton Labs.)

Examples of humectants are glycerin, propylene glycol, sorbitol, lactic acid, and urea.

Examples of chelating agents are sodium EDTA, citric acid, and phosphoric acid.

Examples of other excipients are edible oils like almond oil, castor oil, cacao butter, coconut oil, corn oil, cottonseed oil, linseed oil, olive oil, palm oil, peanut oil, poppyseed oil, rapeseed oil, sesame oil, soybean oil, sunflower oil, and teaseed oil; and of polymers such as carmelose, sodium carmelose, hydroxypropylmethylcellulose, hydroxyethylcellylose, hydroxypropylcellulose, pectin, xanthan gum, carrageenan, locust bean gum, acacia gum, gelatin, carbomer, emulsifiers like vitamin E, TPGS, glyceryl stearates, cetanyl glucoside, and alginates.

Examples of ointment bases, in general, are beeswax, paraffin, cetanol, cetyl palmitate, vegetable oils, sorbitan esters of fatty acids (Span), polyethylene glycols, and condensation products between sorbitan esters of fatty acids and ethylene oxide, e.g. polyoxyethylene sorbitan monooleate (Tween).

Examples of hydrophobic or water-emulsifying ointment bases are paraffins, vegetable oils, animal fats, synthetic glycerides, waxes, lanolin, and liquid polyalkylsiloxanes.

Examples of hydrophilic ointment bases are solid macrogols (polyethylene glycols).

Other examples of ointment bases are triethanolamine soaps, sulphated fatty alcohol and polysorbates.

Examples of gel bases or components which, when appropriate, are able to take up exudate are: liquid paraffin, polyethylene, fatty oils, colloidal silica or aluminium, zinc soaps, glycerol, propylene glycol, tragacanth, starch, cellulose derivatives, carboxyvinyl polymers, magnesium-aluminium silicates, Carbopol, hydrophilic polymers such as, e.g. starch or cellulose derivatives, liquid absorbing bandages, water-swellable hydrocolloid, and alginates.

Examples of powder components are: alginate, collagen, lactose, powder which is able to form a gel when applied to an exuding surface. Normally, powders intended for application on large open wounds (e.g. in connection with incarcerated tumours) must be sterile and the particles present must be micronized.

Alginic acid or alginates are important excipients in connection with compositions according to the present invention. As explained above, alginates can form a gel and, furthermore, they can absorb exudates and thus contributing to controlling the moisture content of e.g. an incarcinated area. Alginic acid and alginates are available in various qualities having a mean molecular weight in the range of e.g. about 32,000-200,000. The relative proportion of mannuronic and guluronic acid residues varies from one quality to another.

Other excipients for use in topical compositions include tackifier resin, viscous elastomeric binders, elastic film, elastic adhesive material, elastomers and plasticizers.

Dressings and/or bandages are also important delivery systems for a combination according to the invention. Dressings may be in the form of absorbent dressings for application to exuding areas. Such dressings are frequently made of cotton or viscose fibres which are enclosed in a sleeve of gauze or a suitable non-wowen fabric. Other relevant materials are cellulose fibres, cellulose wood pulp (fine powdery material). Dressings may also be in the form of hydrogel dressings such as, e.g., i) dressings having a fixed three-dimensional macro-structure, and ii) dressings involving amorphous hydrogels. Examples of other kinds of dressings are i) hydrocolloid dressings (gel-forming agents combined with other material such as, e.g. elastomers and adhesives) including hydrocolloid granules or paste and hydrocolloid sheet, ii) alginate sheet (rope or ribbon, alginate with integral absorbent pad), iii) foams (foam dressings, silastic foam, polyurethane foam), iv) various polysaccharide materials, v) occlusive dressings, vi) semipermeable dressings, vii) paraffin gauze dressings, viii) Tulle dressings, ix) polysaccharide pastes, granules and beads (may be manufactured from dextran derivatives), and x) odour-absorbing dressings. Suitable bandages may be i) non-extensible bandages, ii) extensible bandages, iii) adhesive/cohesive bandages, iv) tubular bandages, v) medicated paste bandages, and vi) orthopaedic casting materials.

The compositions mentioned above for topical administration may also be suitable for application to or for introduction into relevant orifice(s) of the body, e.g. the rectal, urethral, vaginal aural, nasal or oral orifices. The composition may simply be applied directly on the part to be treated such as, e.g., on the mucosa, or by any convenient route of administration.

Suitable dispersing or wetting agents are, for example, naturally occurring phosphatides, e.g., lecithin, or soybean lecithin; condensation products of ethylene oxide with e.g. a fatty acid, a long chain aliphatic alcohol, or a partial ester derived from fatty acids and a hexitol or a hexitol anhydride, for example polyoxyethylene stearate, polyoxyethylene sorbitol monooleate, polyoxyethylene sorbitan monooleate, etc.

Finally, it is an interesting possibility to target any of the substances (such as protease inhibitors) used according to the invention to a target molecule or tissue. In this way, relatively high concentrations of the inhibitor in question will be present in said tissue or in areas where the target molecule is especially abundant. A number of ways exists to achieve this effect, but in general they all rely on the use of a carrier molecule having a high affinity for the chosen tissue (such as a carrier antibody or fragment thereof) to which is covalently or non-covalently linked the active substance in question. For the purposes of the present invention, an antibody (or fragment thereof) directed against a specific antigens overexpressed in tumours (such as carcino-embryonic antigen, Lewis antigen, transferrin, multi-drug resistance pump, glucose transporters, and uPAR) would be useful as carrier molecule for any of the substances used according to the invention.

Instead of relying on implantation and/or wound healing in Plg^{-/-} mice in order to assess the suitability of putative second and/or third substance as defined herein, it is instead possible to assess this suitability on the basis of effects achieved in a mouse model wherein plasmin's actions are substantially abolished (e.g. by use of one of the above-mentioned inhibitors). As is apparent from the examples, it is possible to abolish tumour growth in mice treated simultaneously with inhibitors of plasmin and metalloproteases, and hence, such a mouse model where plasmin's actions are blocked would also be suitable in determining the suitability of other protease inhibitors, especially for cancer treatment. Hence another part of the invention pertains to method for preventing or arresting invasive remodelling in a mammal, the method comprising 1) inhibiting or abolishing, in the mammal, the *in vivo* protein cleaving actions of plasmin as well as of active derivatives thereof and 2) simultaneously therewith inhibiting or abolishing the *in vivo* protein cleaving actions of at least one proteolytic enzyme which is different from plasmin as well as from active derivatives thereof and which exerts its action on at least one extracellular protein, said at least one proteolytic enzyme being non-murine (including human) analogue(s) of murine enzyme(s) which, isolated or in combination, is/are essential for invasive tissue destruction associated with malignant growth in mice where the protein cleaving actions of plasmin are substantially abolished.

All the above-mentioned embodiments of the other compositions of the invention apply *mutatis mutandis* to this composition, and in fact the compositions described above could as well be provided on the basis of evaluation in such a mouse model instead of in the Plg^{-/-} model. Hence, all embodiments described herein which rely on the effects of the second substance in the Plg^{-/-} model also apply when the second substance abolishes tissue destruction associated with malignant growth in a mouse where the protein cleaving actions of plasmin is substantially abolished.

Finally, it is a possibility that some types of invasive remodelling utilises other enzymes than the plasminogen/plasmin system. Nevertheless, it is believed that the concept of the invention, namely to exploit the redundancy in the actions of proteases which are active against extracellular proteins, is a general way to attack invasive remodelling. Hence, in a general aspect the invention also relate to a method for preventing or arresting invasive remodelling in a mammal, the method. comprising 1) inhibiting or abolishing, in the mammal, the *in vivo* protein cleaving actions of at least one first protease, A, and 2) simultaneously therewith inhibiting or abolishing the *in vivo* protein cleaving actions of at least one other protease, B, which is of a different class of proteases than A, wherein A and B have at least one extracellular protein as a common substrate, said at least one protease, B, being essential for embryo implantation and/or wound healing and/or necessary for invasive tissue destruction associated with malignant growth in the mammal when the protein cleaving actions of A are substantially abolished in the mammal but not when the protein cleaving actions of A are substantially intact in the mammal.

The above indicated proteases A and B are preferably chosen from the group consisting of metalloproteases, cysteine proteases, serine proteases and aspartic proteases.

The above principle of using substances A and B also combines with all pertinent embodiments of other compositions of the invention with respect to the choice of substances used for obtaining inhibition or abolition of their proteolytic activity as well as choices of regimens, choices of conditions to be treated etc. In fact, all embodiments of the compositions used according to the invention disclosed *supra* apply *mutatis mutandis* to this certain aspect of the invention, and all limitations on the compositions used according to the present invention described above are thus intended to also be possible limitations on this aspect of the invention.

In another aspect of the invention, it is recognized that the use of animals wherein the *in vivo* protein cleaving actions of proteases have been substantially abolished provide a completely novel tool in the search for substances which are active in invasive tissue remodelling. Since an activity of such a substance could be "obscured" by the fact that the actions of the protease with which it interferes are supplemented by other proteases, the use of such an animal can give rise to substantial insight in the relation between known and novel substances on the one hand and invasive tissue remodelling on the other.

An aspect of the invention therefore relates to a method of screening for a substance which is capable of interfering with invasive remodelling in a mammal, including a human being, the method comprising providing an animal wherein has been substantially abolished the *in vivo* protein cleaving actions of at least one protease which contributes to invasive remodelling, and thereafter assessing the effect of administration of the substance to the animal on at least one process known to involve invasive remodelling, and finally establishing as a result that the substance is capable of interfering with invasive remodelling if the at least one process is inhibited to a significantly higher degree than in both the animal when it does not receive the substance and in a reference animal which has not had the in vivo protein cleaving actions of the at least one protease substantially abolished. The substantial abolishment of the in vivo protein cleaving actions of the at least one protease can e.g. be accomplished by genetic modification of the animal (e.g. by knocking out the gene for the protease as in the presently used plg^{-/-} mouse) or by pharmaceutical inhibition of the actions of the protease. The protease can be as defined above. Of course, the preferred processes known to involve invasive remodelling are those described above, *i.e.* wound healing, embryo implantation, etc.

### BRIEF DESCRIPTION OF THE DRAWING

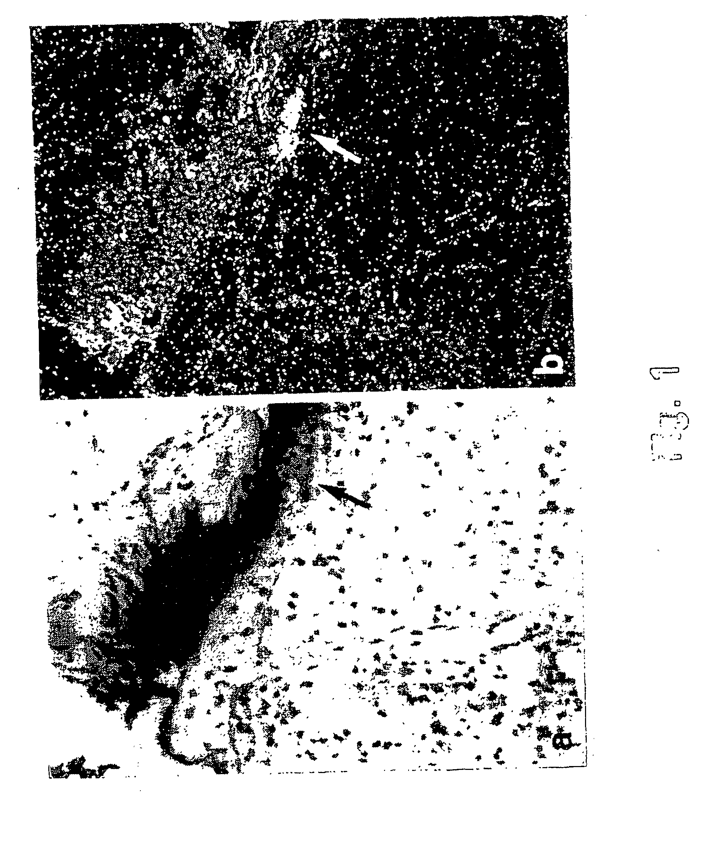

Fig. 1: Gelatinase B mRNA is expressed in the leading-edge keratinocytes during re-epithelialization of mouse skin incisional wounds. Gelatinase B mRNA expression is detected by in situ hybridization in the leading-edge keratinocytes 48 hours after experimental full thickness skin wounding (arrows in A and B). At that time point the leading-edge keratinocytes have flattened and are moving under the wound clot, and the Gelatinase B expressing cells are located exactly in the front of the moving epidermal layer. At time points ranging from 24 to 120 hours after wounding Gelatinase B mRNA is exclusively expressed by the keratinocytes located in the tip and at the basal area of the moving epidermal layer. Eventually, when the wounds are covered with newformed epidermis after approximately 7 days, no Gelatinase B mRNA expression can be detected. Expression of gelatinase B mRNA could not be detected in any other cells in the wounds or in normal skin (not shown). Fig. 1a is bright-field and Fig. 1b is dark-fields micrographs. Magnification: × 85.

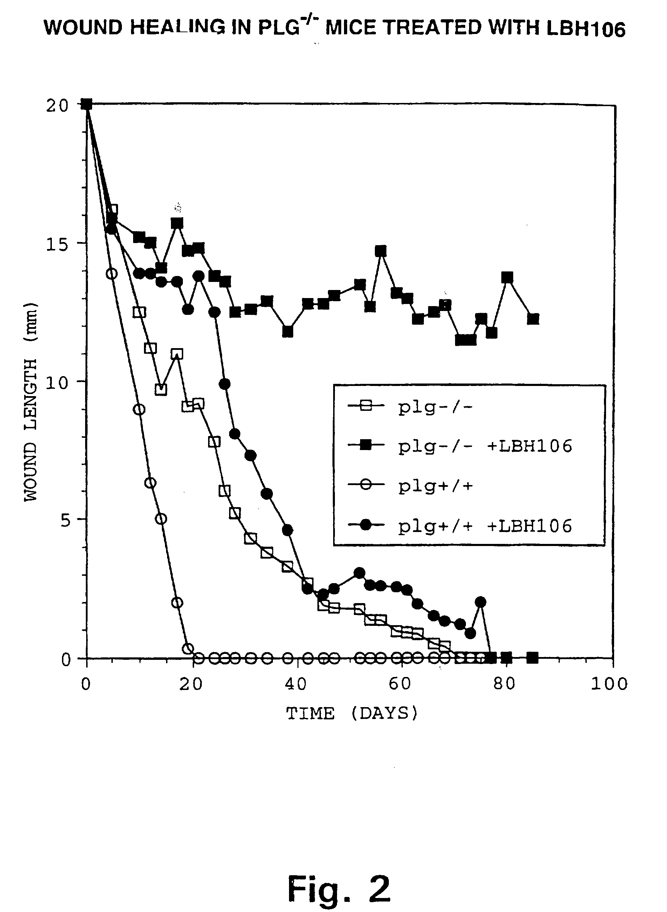

Fig. 2: Wound healing in plasminogen deficient (plg^{-/-}) mice and wild type plg^{+/+} mice treated systemically with Galardin™. Four groups of 12 mice were wounded with a standardised 20 mm full-thickness skin incision. The first group (denoted plg^{-/-}) consisted of plg^{-/-} mice and were treated i.p. only with buffered vehicle (4% carboxymethyl cellulose, CMC) following wounding, while another group (denoted plg^{-/-} + LBH106) of 12 plg^{-/-} mice were treated i.p. with 2.5 mg Galardin™ per day after wounding. A third group (denoted plg^{+/+}) consisting of control plg^{+/+} mice were treated i.p. only with buffer vehicle following wounding, while the fourth group (plg^{+/+} + LBH106) were plg^{+/+} mice treated i.p. with 2.5 mg Galardin™ per day after wounding. The graph shows the mean open wound length (mm) as a function of healing time.

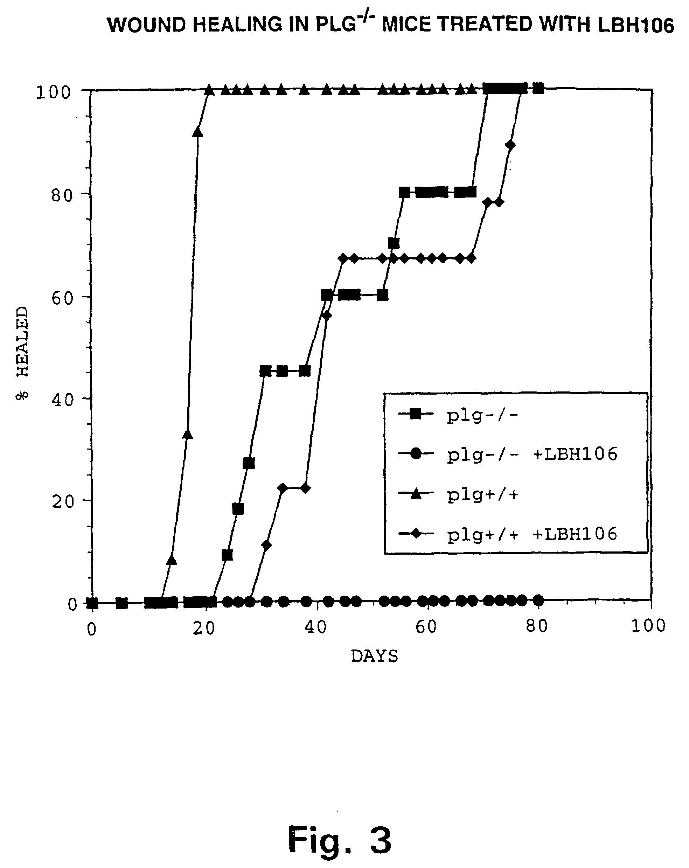

Fig. 3: Wound healing in plg^{-/-} and plg^{+/+} mice. These results are from the same experiment as in Fig. 2, but they are presented as the percentage of wounds in each group which are completely healed (wound length = 0 mm) at each time point.

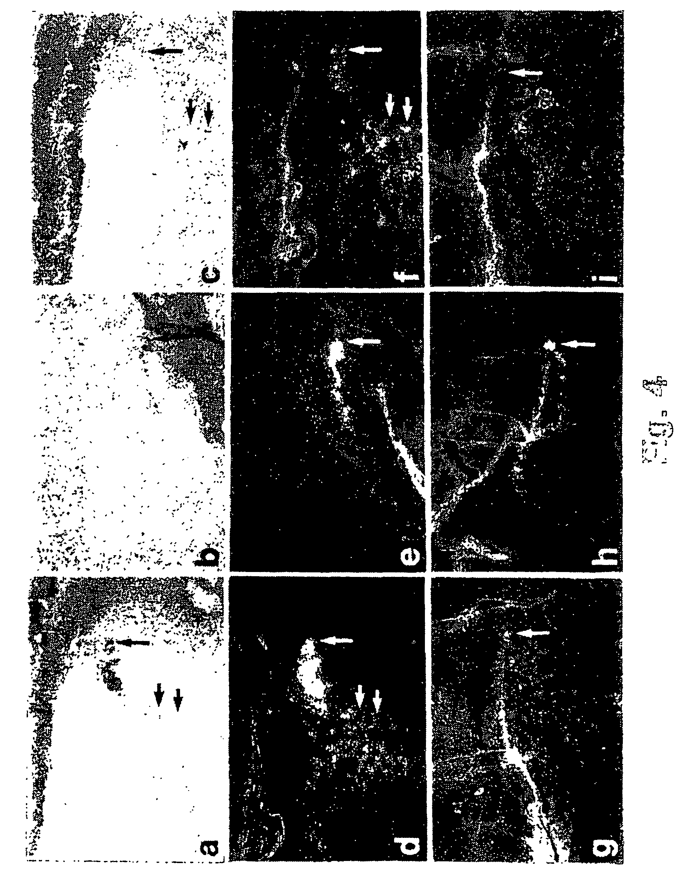

Fig. 4: Expression of metalloproteases in healing wounds from plg^{-/-} mice treated systemically with Galardin™ and untreated wild-type mice. Sections of tissue removed day 7 after surgery from plg^{-/-} mice treated systemically with Galardin™. The sections were analyzed by *in situ* hybridisation for expression of mRNAs for metalloproteases and exposed to an autoradiographic emulsion. mRNA expression of gelatinase B (a, d, and g), collagenase-3 (b, e and h) and gelatinase A (c, f, and i) in wounds from Galardin™ treated plg^{-/-} mice (a-f) or untreated wild-type plg^{+/+} mice (g-i). The long straight arrows show the tip of the leading-edge keratinocytes. Small arrows show the border of the granulation tissue. The micrographs a-c are bright-field, and those in d-i are dark-field. Magnification: × 100.

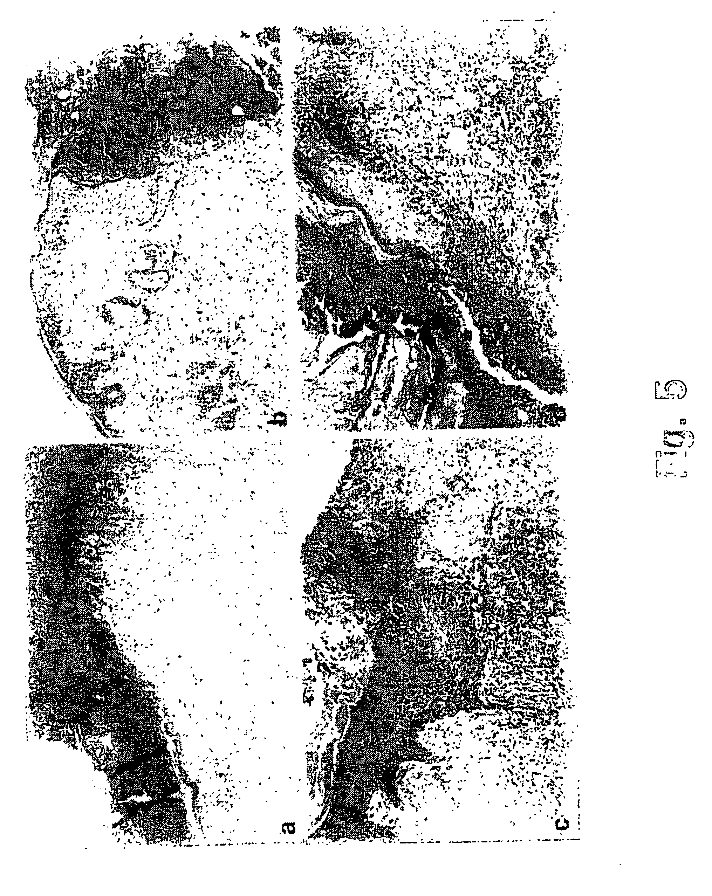

Fig. 5: Microscopic appearance of skin wounds in wildtype mice treated with vehicle (a) or Galardin™ (b) and Plg-deficient mice treated with vehicle (c) or Galardin™ (d) 7 days after surgery. Sections were stained with haematoxylin and eosin. The wedge-shaped appearance of the migrating layer of keratinocytes seen in the control mice (a) is clearly changed to blunted ends in the three groups of mice either lacking plasminogen (b), being treated with a metalloprotease inhibitor (c) or both (d). In these cases the keratinocytes are apparently halted by a rim of the provisional wound matrix, which is marked by arrows in b-d. In the mice which are Plg-deficient and treated with Galardin™, this layer of matrix is especially thick (d). Magnification: × 165.

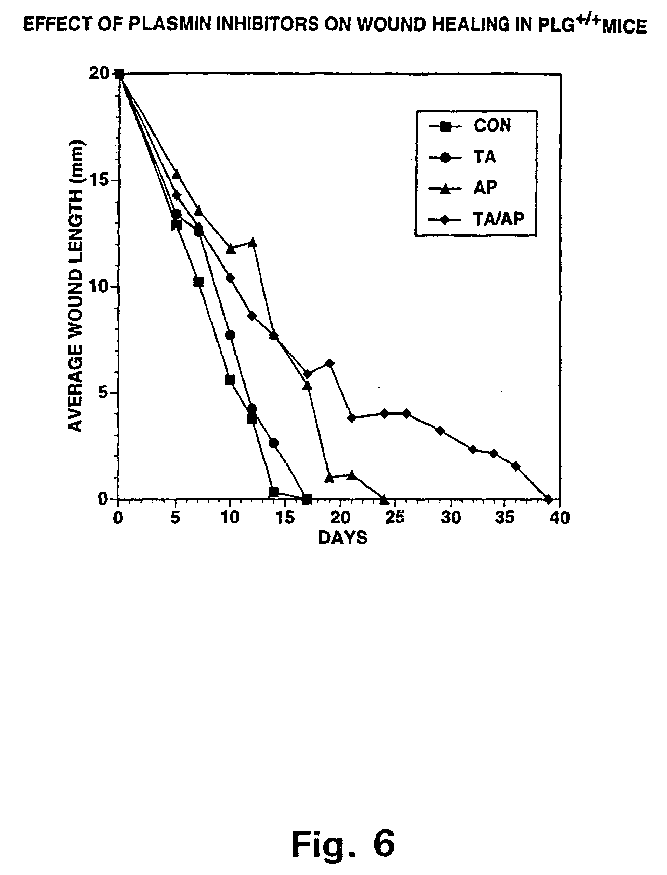

Fig. 6: Wound healing in plg^{+/+} mice treated systemically with aprotinin (AP) and tranexamic acid (TA). Twenty plg^{+/+} mice were wounded with a standardised 20 mm long, full thickness skin incision on the back and then divided into four groups: group 1 received each day three i.p. injections of buffer vehicle; group 2 received each day aprotinin (13,000 KIU) divided in three i.p. injections at least six hours apart; group 3 received each day tranexamic acid (10 mg) divided in three i.p. injections at least six hours apart, and group 4 received each day aprotinin (13,000 KIU) and tranexamic acid (10 mg) divided in three i.p. injections at least six hours apart.

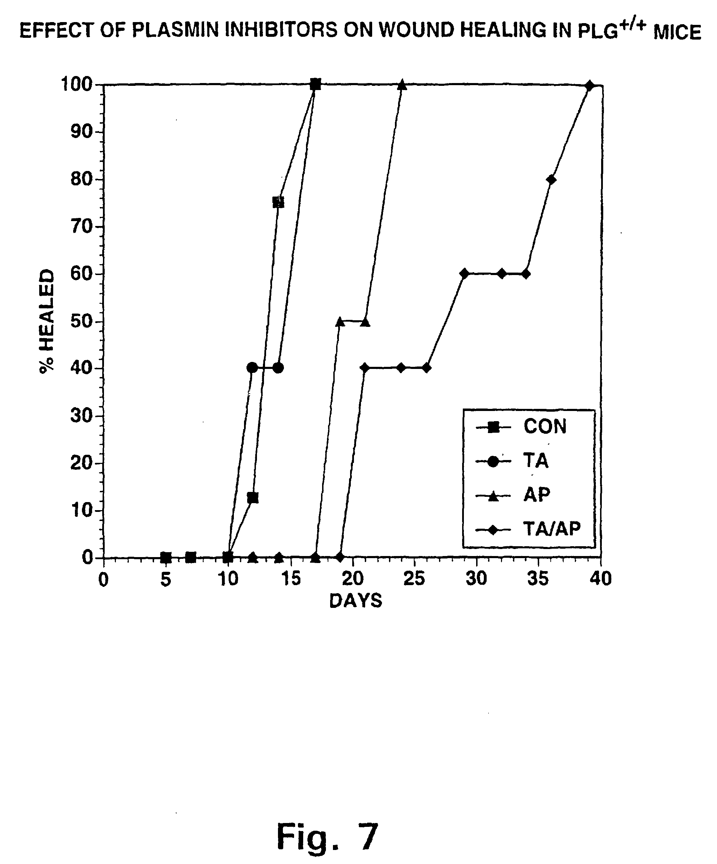

Fig. 7: Wound healing in plg^{+/+} mice treated systemically with aprotinin (AP) and tranexamic acid (TA). These results are from the same experiment as in Fig. 6, but they are presented as the percentage of wounds in each group which are completely healed (wound length = 0 mm) at each time point.

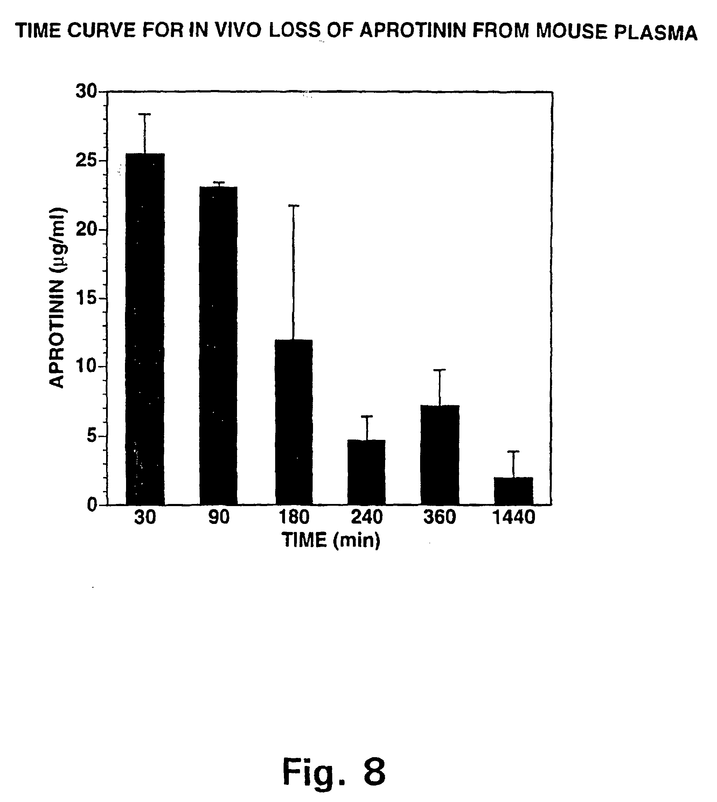

Fig. 8: Biological half-life of aprotinin in plg^{+/+} mice. Fifteen mice (weighing approx. 25 g each) were injected i.p. with 0.75 ml of aprotinin solution (20,000 KIU/ml; approx. 2.1 mg) and at time intervals of 0.5, 1.5, 3.0, 6.0 and 24 h thereafter three mice were sacrificed and blood collected by cardiac puncture into citrate anti-coagulant tubes. Each plasma sample was ultrafiltrated through a 20,000 MW cut-off membrane, and the aprotinin level in the filtrate was determined by inhibition of the colorimetric activity of a standard trypsin solution.

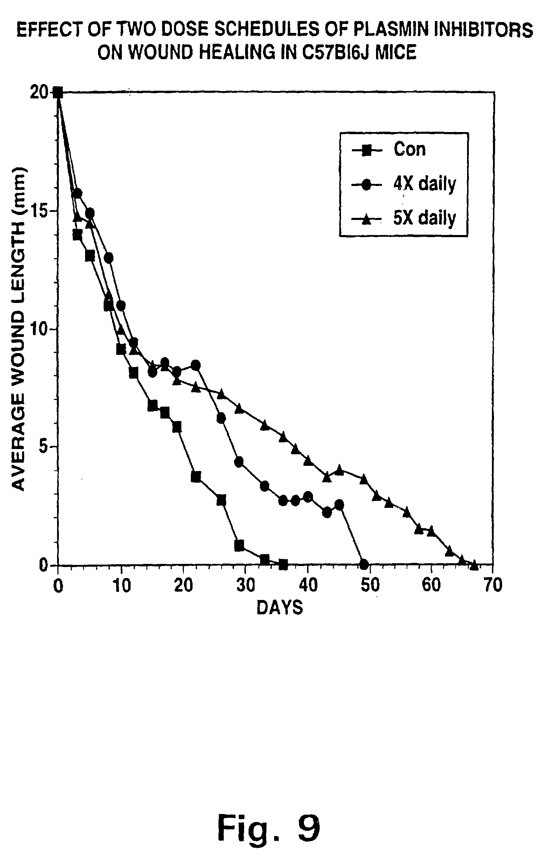

Fig. 9: Effect of systemic plasmin inhibitor treatment scheduling on wound healing in plg^{+/+} mice. Fifteen plg^{+/+} mice were wounded with a standardised 20 mm long, full thickness skin incision on the back and then divided into three groups: group 1 received 5 i.p. injections of buffer vehicle per day; group 2 received each day aprotinin (650,000 KIU/kg) and tranexamic acid (1 g/kg) divided in 4 i.p. injections 6 hours apart, and group 3 received each day thereafter aprotinin (650,000 KIU/kg) and tranexamic acid (1 g/kg) divided in 5 i.p. injections at least 4 hours apart; Each day the length of the open wounds was carefully measured and recorded. The time taken for each skin wound to completely close was also noted.

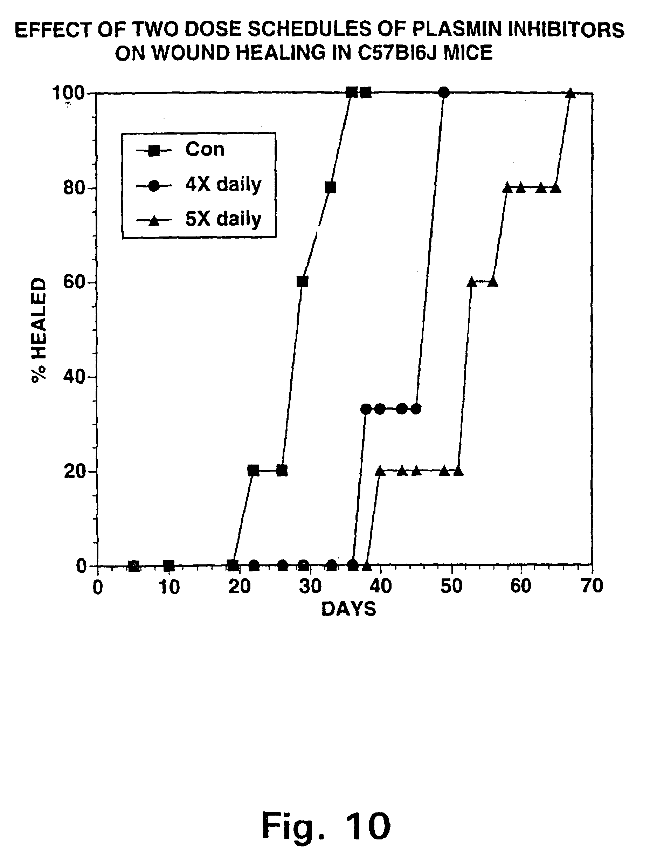

Fig. 10: Effect of systemic plasmin inhibitor scheduling on wound healing in plg^{+/+} mice. These results are from the same experiment as in Fig. 9, but they are presented as the percentage of wounds in each group which are completely healed (wound length = 0 mm) at each time point.

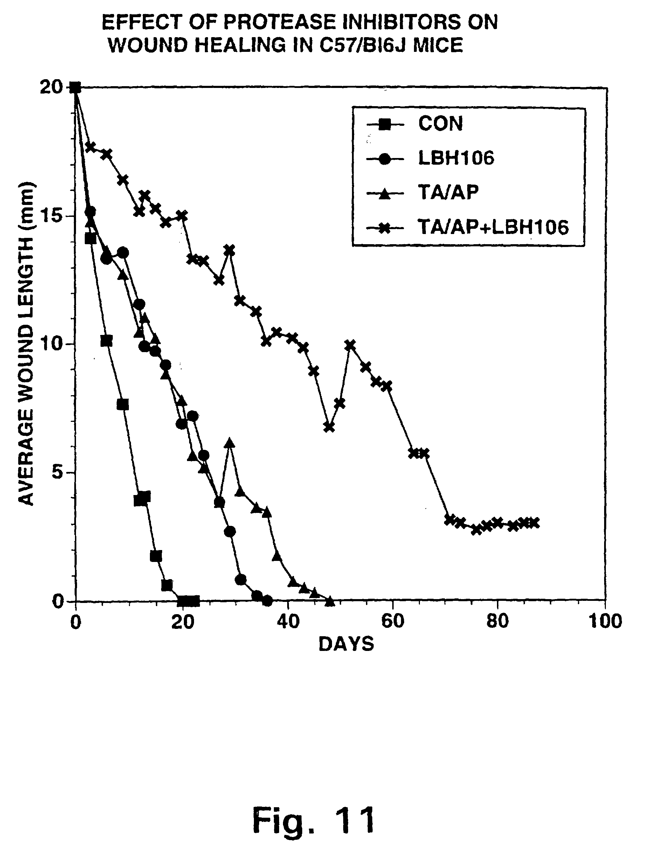

Fig 11: Effect of systemic combination treatment of plasmin inhibitors and metalloprotease inhibitors on wound healing in normal plg^{+/+} mice. Forty-eight plg^{+/+} mice were wounded with a standardised 20 mm long, full thickness skin incision on the back and then divided into four groups: Group 1 received 5 i.p. injections of buffered vehicle per day; group 2 received each day aprotinin (AP; 13,000 KIU/day/mouse) and tranexamic acid (TA; 20 mg/day/mouse) divided in 4 i.p. injections at least five hours apart; group 3 were injected once i.p. each day with Galardin™ (LBH-106; 2.5 mg in 125 µl of CMC); group 4 received combination treatment of aprotinin, tranexamic acid and Galardin™ at the same doses above. Each day the length of the open wounds was carefully measured and recorded.

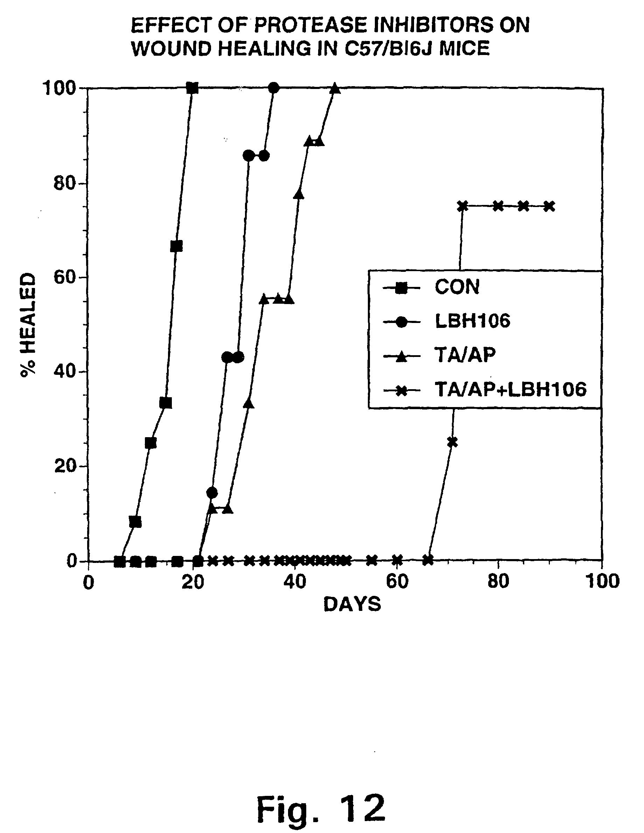

Fig 12: Effect of systemic combination treatment of plasmin inhibitors and metalloprotease inhibitors on wound healing in normal plg^{+/+} mice. These results are from the same experiment as in Fig. 11, but they are presented as the percentage of wounds in each group which are completely healed (wound length = 0 mm) at each time point.

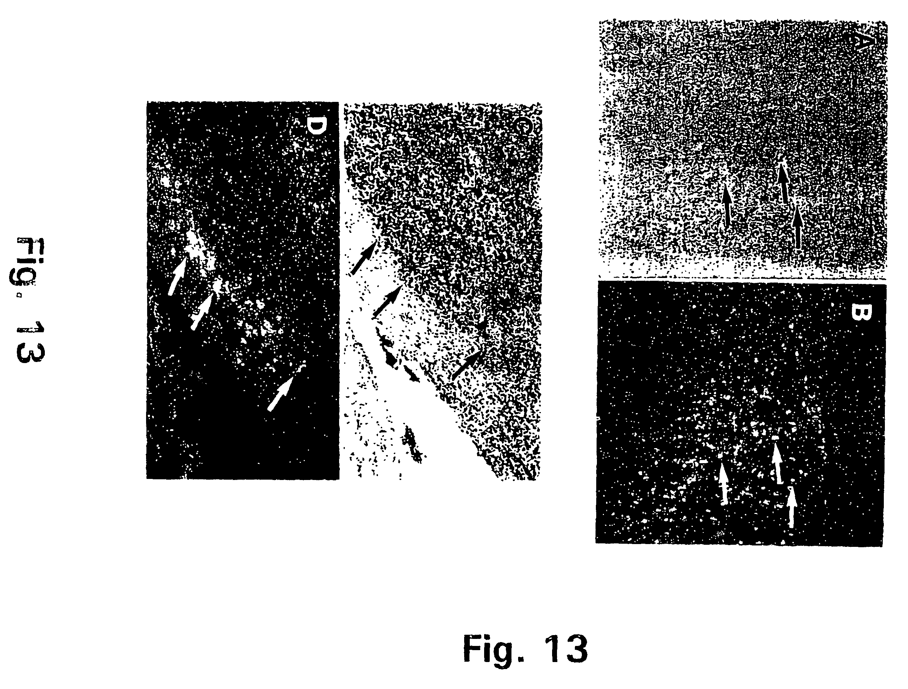

Fig. 13: Expression of mRNAs for metalloproteases in Lewis lung carcinomas detected by *in situ* hybridization. Figs. 13A and 13B show the same image photographed with a brightfield (A) and a darkfield condensor (B). The gelatinase-B expressing cells (arrows in Figs. 13A and 13B) are easily identified with the darkfield condensor, where the signal for the positive cells appear as bright silver grains (arrows in Fig. 13 B). Similarly, using a darkfield condensor, stromelysin-1 expressing cells could easily be identified in the stroma surrounding the Lewis Lung carcinoma cells (arrows in Fig. 13D). Fig. 13C is a heamatoxylin and eosin stained section adjacent to the section in Fig. 13D, and is included to show the tissue structure in Fig. 13D. Arrows in Fig. 13C indicate the virtual location of the positive cells indicated by arrows in Fig. 13D.

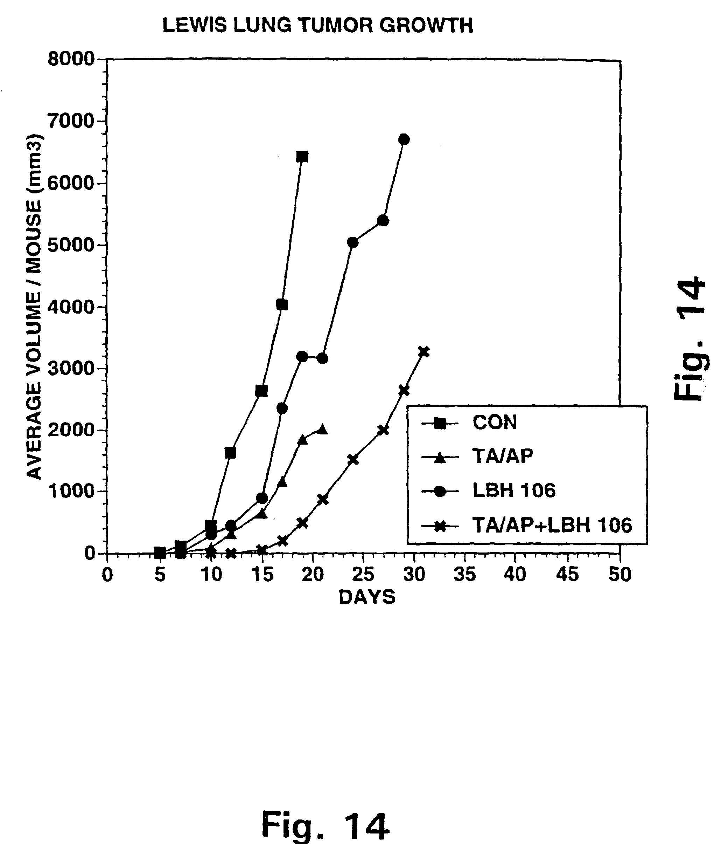

Fig. 14: Effect of systemic treatment with plasmin inhibitors and Galardin™ on growth of Lewis lung carcinomas in wildtype (plg^{+/+}) mice. Four groups of 17 mice were injected subcutaneously with Lewis lung carcinoma tissue mince (approx. 1.0 x10⁶ cells). The first group (CON) were treated only with buffered vehicle. The second group (TA/AP) were injected i.p. three times each day with a mixture of tranexamic acid (TA) and aprotinin (AP), so that the total dosage was 10 mg TA and 13,000 KIU AP per day per mouse. The third group (LBH 106) was treated with 1.8 mg Galardin™ per day per mouse (in buffered CMC vehicle), and the fourth group received a combination of TA, AP and Galardin™ at the same doses and schedules as above. The tumour growth in the mice was measured every 2-3 days and plotted as a function of time.

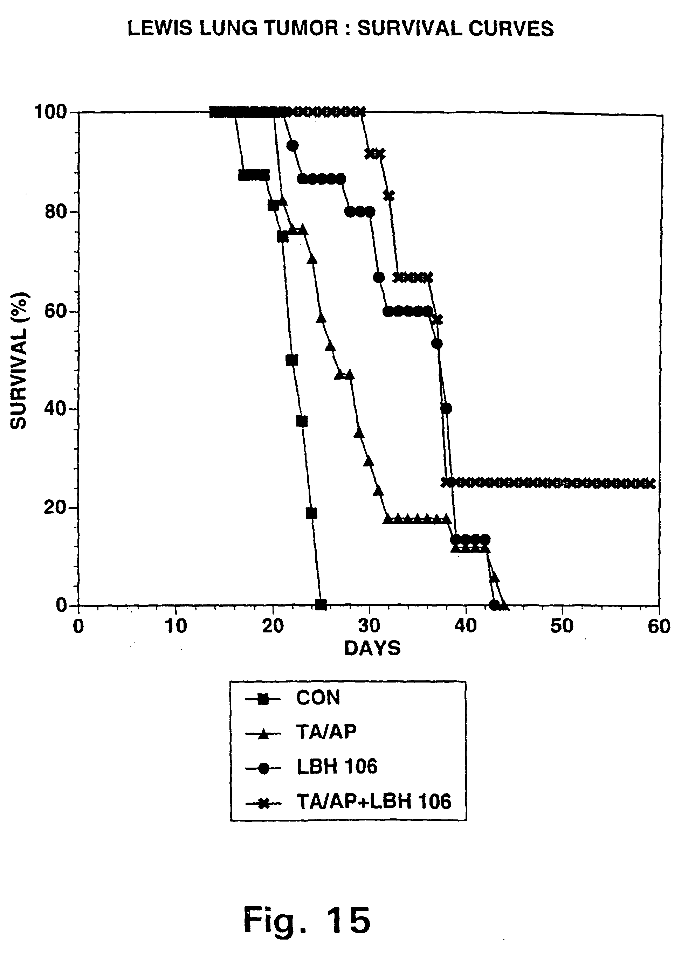

Fig. 15: Effect of systemic treatment with plasmin inhibitors and Galardin™ on survival of wild-type (plg^{+/+}) mice inoculated with Lewis lung carcinoma. Kaplan-Meier plot for survival of the mice in the four groups described above in Fig. 14.

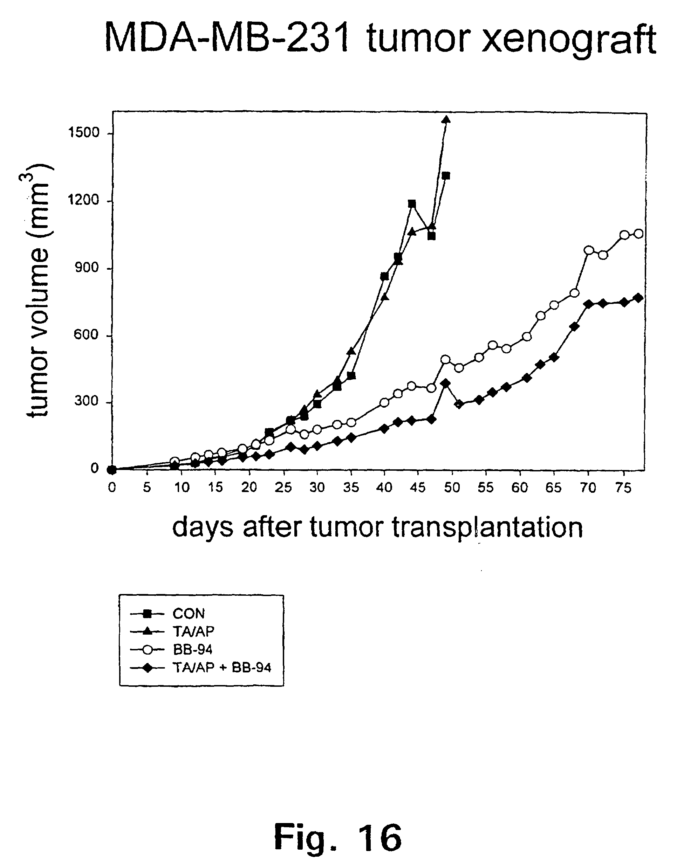

Fig. 16: Effect of systemic treatment with plasmin inhibitors and BB-94 on growth of MDA-MB-231 human breast cancer xenograft in nude mice. Four groups of 11 mice were injected subcutaneously with 2 x 10⁶ cultured tumour cells. The first group (control) were treated only with buffer vehicle. The second group (TA/AP) were treated with a mixture of tranexamic acid and aprotinin, so that the total dosage was 650,000 KIU/kg aprotinin and 1 g/kg tranexamic acid divided in 5 i.p. injections at least four hours apart. The third group (BB-94) was treated with 125 mg/kg BB-94 as a single i.p. injection each day, and the fourth group received a combination of plasmin inhibitors and BB-94 at the same doses and schedules as above. The tumour growth in the mice was measured every 2-3 days and plotted as a function of time.

### EXAMPLE 1

### Wound Healing in Plg^{-/-} mice Treated with a Metalloprotease Inhibitor.

In this example, mice totally deficient in plasmin were treated systemically with a potent metalloprotease inhibitor. The model tested was wound healing. Successful wound healing was read as the complete closure of a skin incision.

### Background:

Wound healing shares several features in common with tumour invasion. Thus the plasminogen/plasmin system as well as metalloproteases are found to be over expressed at the site of keratinocyte migration, and total deficiency of plasminogen/plasmin is associated with partially retarded keratinocyte migration and slower wound closure (Rømer, 1996) .

The metalloprotease inhibitor Galardin™ inhibits a number of metalloproteases: Fibroblast interstitial collagenase (MMP-1), Kᵢ=0.4 nM; 72 kd gelatinase A (MMP-2), Kᵢ= 0.5 nM; stromelysin-1 (MMP-3), Kᵢ= 30 nM; neutrophil interstitial collagenase (MMP-8), Kᵢ= 0.1 nM; 92 kd gelatinase B (MMP-9), Kᵢ= 0.2 nM) . In contrast, Galardin™ exhibits very weak inhibition of the serine proteases known as angiotensin converting enzyme (ACE), and plasmin. The Kᵢ's for these enzymes are in the millimolar range, i.e. a million-fold higher.

### Protocol:

The methods used followed those described by Rømer et al, 1996. Four groups of mice were set up, consisting of 12 plg^{-/-} mice (group 1), another 12 plg^{-/-} mice (group 2), 12 plg^{+/+} mice (group 3) and another 12 plg^{+/+} mice (group 4). All mice were then wounded with a standardised 20 mm long, full thickness skin incision on the back. Groups 2 and 4 were then injected once i.p. each day thereafter with Galardin™ (2.5 mg in 125 µl of a buffered 4% suspension of carboxymethyl cellulose; CMC) while groups 1 and 2 received only i.p. injections of the vehicle.

Each day after wounding the length of the open wounds was carefully measured and recorded. The time taken for each skin wound to completely close was also noted.

In parallel experiments, the mice were sacrificed and tissue sections prepared from the wound site. The tissue sections were analyzed for expression of various metalloproteases by in situ hybridisation and for matrix content by staining.

**In situ hybridization**. *In situ* hybridization was performed on paraffin sections essentially as described (Kristensen, 1991; Rømer *et al.*, 1996). ³⁵S labelled RNA sense and antisense probes were generated by in vitro transcription from the following mouse cDNA subclones: mouse gelatinase A fragments (604-1165) in pSP64 and pSP65 and (1924-2259) in pGEM-3 (Reponen et al., 1992, J. Biol. Chem. 267(11), 7856-62); mouse gelatinase B fragments (805-1099) and (1944-2267) in pSP64 and pSP65 (Reponen *et al.*, 1994, J. Cell. Biol. **124**, 1091-1102); mouse stromelysin-1 fragments (3115-4051) and (2205-2918) in pBluescript KS(+) (Hammani *et al.,* 1992, Gene **120**(2), 321-322); mouse collagenase-3 EcoRI fragments (485 bp) and (811 bp) in pBluescript KS(+) (Henriet *et al*., 1992, FEBS Letters **310**(2), 175-178); mouse macrophage elastase fragment (900-1250) in pBluescript KS(+) (Shapiro et al., 1992, J. Biol. Chem. **267**, 4664-4671); and mouse MT-1 MMP fragments (1122-1258) and (1259-1705) in pBluescript KS(+) (Okada *et al*., 1995, PNAS U.S.A., **92**(7), 2730-2734). The mouse macrophage elastase expression system was investigated with a single probe, while the expression of all other metalloproteases were investigated with two different non-overlapping probes, which in all cases gave identical results.

### Results:

### Several metalloproteases are expressed during wound healing.

To examine the expression of MMP's during skin repair, full thickness incisional wounds were made in wild-type mice, and sections were analyzed microscopically at time points ranging from 12 hours to 7 days after wounding. mRNA encoding the respective proteases were detected by *in situ* hybridization with probes specific for seven different MMP's, including gelatinase A (MMP-2), gelatinase B (MMP-9), collagenase-3 (MMP-13), stromelysin-1 (MMP-3), MT-1 MMP (MMP-14), and macrophage metalloelastase (MMP-12). In normal skin there was no detectable mRNA for any of these MMP's, while they were all expressed in the skin wounds.

Gelatinase B mRNA was already detected 12 hours after the wounding in the keratinocytes at the wound edge, which had just begun to flatten at that time and move into and under the wound clot. These gelatinase B expressing cells were located exactly at the front of the moving epidermal layer, and this was also found at subsequent time points until seven days after wounding; gelatinase B mRNA was exclusively found in keratinocytes located basally at the front of the moving epidermal layer (Fig. 1a and b). Collagenase-3 mRNA was expressed in a pattern similar to that of gelatinase B, the only difference being that collagenase-3 mRNA was also detected in many basal keratinocytes located further behind the leading-edge. Expression of gelatinase B or collagenase-3 mRNA was not detected in the granulation tissue or in any cell-type other than the keratinocytes. When the wounds were eventually completely covered with a newly formed epidermal layer, which in some cases was already observed seven days after wounding, expression of mRNA for the two MMPs was no longer detectable in any cells in the skin (not shown).

Gelatinase A and MT-1 MMP mRNA were both detected in fibroblasts located in the lower dermis at the edge of the wound site from 12 hours after wounding. At later time points strong signals for both messengers were also seen in the granulation tissue, located in fibroblasts which were then migrating under the wound site. Comparison of adjacent sections showed that gelatinase A and MT-1 MMP mRNA were expressed in the very same fibroblasts. In the last phase of the healing-processes leading to closure of the wound site with epidermis, expression of the two MMPs was seen in fibroblasts located very close to the newly formed epidermal basement membrane. After the closure of the wound site with a new epidermal layer, weak signals for gelatinase A and MT-1 MMP mRNA were still found in some fibroblasts. Expression of mRNAs for any of these two molecules was not detected in keratinocytes or any other cells in the wounds (not shown).

The expression pattern of stromelysin-1 and macrophage metalloelastase were different from those of all the other MMP's studied. Stromelysin-1 mRNA was found in both keratinocytes and fibroblasts. The expression was strongest in the basal keratinocytes behind the migrating front in the area corresponding to the initial wound edge, and in fibroblasts located below and adjacent to these keratinocytes, but stromelysin-1 mRNA was also detected in the leading-edge keratinocytes. Macrophage metalloelastase mRNA was expressed in a subset of macrophages located below and adjacent to the wound clot. Expression of stromelysin-1 and macrophage metalloelastase mRNA was not detected in any other cells in the wounds.

The expression of gelatinase B, collagenase-3, and stromelysin-1 in the leading edge keratinocytes is particularly noteworthy because the very same keratinocytes previously were found to express both of the two key regulators of plasmin generatio, uPA and uPAR (Rømer *et al*., 1996).

**Complete arrest of wound healing in plasminogen deficient mice treated with metalloprotease inhibitor.** The results for wound healing measurements are shown in Figs. 2 & 3. It can be seen that the earlier observation (Rømer 1996) of partially retarded wound closure in plg^{-/-} mice was reproduced (45% of wounds healed after 38 days), and that treatment of plg^{+/+} mice with Galardin™ could produce a stronger but still incomplete retardation of wound healing (22% of wounds healed after 38 days). However when the metalloprotease inhibitor treatment was combined with plasmin deficiency in the Galardin™ treated plg^{-/-} mice, the wound healing was surprisingly completely abolished: 38 days after wounding, none of these mice had healed wounds, while all wounds were closed after only 20 days in plg^{+/+} mice. Even after 80 days, none of the wounds in metalloprotease inhibitor treated plg^{-/-} mice had healed wounds.

**Keratinocyte migration.** In a separate experiment we analyzed the impact of Galardin™ treatment on keratinocyte migration in wild-type and littermate Plg-deficient mice. For each of the genotypes groups of 5 mice were either treated with 100 mg/kg daily of Galardin™ for 7 days after skin wounding or mock treated. 18-20 sections of wound tissue from each group of mice were then analyzed microscopically, and the keratinocyte migration distance along the base of the epidermal outgrowth from the wound edge to the tip of the wedge (see Rømer *et al.*, Fig. 2) was measured blindly by computer-assisted morphometry. In the mock treated wild-type mice, the keratinocyte migration distance was 354 ± 70 (mean value ± standard deviation in arbitrary units) compared with 268 ± 80 in the Galardin™ treated wild-type mice, 290 ± 59 in the mock treated Plg-deficient mice and 208 ± 60 in the Galardin™ treated Plg-deficient mice. Evaluated by the Students t-test, the impairment in keratinocyte migration caused by Galardin™ treatment of wild-type mice was statistically significant (p=0,007) and this was also the case for the impairment caused by Plg-deficiency alone (p=0,01). In the Galardin™ treated Plg-deficient mice the impairment was significant both in comparison with the mock treated wild-type mice (p<0,00001), the Galardin™ treated wild-type mice (p=0,02f and the mock treated Plg-deficient mice (p=0.0008). Thus MMP-inhibition and Plg deprivation each reduced the keratinocyte migration distance, and in combination had an additive effect on this parameter.

The microscopic appearance of the skin wounds in wild-type and plasminogen deficient mice treated with vehicle or Galardin™ is shown in Fig. 5. The wedge-shaped appearance of the migrating layer seen in the control vehicle-treated wild-type mice is changed to blunted ends in the three other groups of mice, the keratinocytes apparently being halted by a rim of the provisional wound matrix which is clearly most abundant in the group of plasminogen deficient mice treated with Galardin™.

**Increased MMP expression after treatment with metalloprotease inhibitor.** Expression of gelatinase B, collagenase-3, stromelysin-1, and gelatinase A was examined by *in situ* hybridization of wound-tissue from mock treated Plg-deficient and wild-type mice, and from mice of both genotypes which had been treated with daily doses of 100 mg/kg of Galardin™. In the mock treated mice the expression of these MMPs was as described above for untreated animals. However, in both wild-type and Plg-deficient mice, the expression of gelatinase B, collagenase-3 and stromelysin-1 mRNA were dramatically upregulated in the leading-edge keratinocytes in the Galardin™ treated mice compared with the mock treated mice of the same genotype (see Fig. 4). Furthermore gelatinase B expression was also found in both groups of Galardin™ treated mice in cells in the granulation tissue located just beneath the leading edge keratinocytes, in contrast to the absence of gelatinase B expression in the granulation tissue in the two groups of mock treated mice. Furthermore, expression of both gelatinase A and stromelysin-3 mRNA was upregulated in granulation tissue fibroblasts in the Galardin™ treated wild-type and Plg-deficient mice, and gelatinase A mRNA expression in these animals was also detectable in the leading-edge keratinocytes, in contrast to the lack of detectable gelatinase A expression in keratinocytes in the mock treated mice.

### Conclusion:

Wound healing in mice is retarded but nevertheless proceeds in the absence of plasmin (Rømer, 1996), and again it is retarded but nevertheless proceeds when metalloproteases are inhibited in the presence of plasmin(ogen), but it is completely arrested when the absence of plasmin(ogen) is combined with inhibition of metalloproteases. These findings are interpreted as showing that the absence of plasmin and inhibition of metalloproteases act synergistically, reflecting a dual contribution or functional overlap between these two types of enzymes to the remodelling process of wound healing.

Furthermore, systemic treatment with a metalloprotease inhibitor produces an increase in the level of expression of target metalloprotease mRNA at the very tissue site where it is functionally involved in wound repair, demonstrating a compensatory response to the metalloprotease inhibition. This and the increase in matrix content provides evidence that the inhibitor treatment is acting effectively on its target enzyme system, and that the remodelling tissue is capable of an attempt at overcoming this inhibition by upregulation of the effected enzymes.

### EXAMPLE 2

**Implantation in Plg**^{**-/-**} **Mice Treated with a Metalloprotease Inhibitor.**

In this example, plasminogen deficient mice were treated systemically with a potent metalloprotease inhibitor. The model tested was embryo implantation. Successful implantation was read as the presence of viable embryos in the pregnant mice.

### Background for the model:

The mean time for onset of oestrus in a normal female mouse is 4 to 6 hours after onset of darkness. Ovulation occurs from 2 to 3 hours after the onset of oestrus. The female mouse only copulates during oestrus when ova are or are becoming ready for fertilisation. Since oestrus usually begins around midnight, mating is most common during the night hours, generally about 02:00. Evidence of successful mating is a vaginal plug, a coagulum of fluid from the vesicular and coagulating glands of the male, that occludes the vaginal orifice. Noon the day after mating is set as day 0.5 of gestation. The first of the zygote cleavages begins in the ampulla of the oviduct about 24 hrs after fertilisation and cleavages continue for 2 to 3 days in the transit through the oviduct to the uterus. At day 3 of gestation the embryo enters the uterus and consists of 16 cells. The morula stage is still in the upper part of the uterus, until on the 4th day of gestation the zona pellucida is shed and free blastocysts are found in the uterus. At day 4.5 of gestation the implantation of embryo in the uterine wall begins.

### Protocol:

In the evening, female plg^{-/-} mice (8-10 weeks old) were placed in cages together with male plg^{-/-} mice of proven potency. Each morning the females were closely examined for the characteristic vaginal "plugs" formed after mating, and after plugged females were found they were removed and allocated to two treatment groups. Beginning at day 3.5 (i.e. just before implantation) and up to day 18.5 they were given daily i.p. injections (125 µl) of either CMC vehicle (group 1, three mice), or the same vehicle with addition of Galardin™ (G; 2.5 mg) (group 2, four mice). Similarly, wild-type plg^{+/+} female mice were mated with plg^{+/+} males and ten plugged females were obtained for two treatment groups. Three days after plugging, the mice were injected daily with either vehicle (group 3, five mice), or vehicle with addition of Galardin™ (2.5 mg) (group 4, five mice).

### Results:

At day 18.5 the mice were evaluated for pregnancy, and the results shown in Table 1 were obtained. An 80% pregnancy rate was obtained in plg^{+/+} mice after Galardin™ treatment, and 100% of these pregnancies were viable; the effect of the metalloprotease inhibitor on pregnancy was thus non-existent or weak when the plasminogen/plasmin system was intact. However in plg^{-/-} mice Galardin™ reduced the pregnancy rate to 25%, and this pregnancy was not viable. No viable embryos were obtained when metalloprotease inhibition was combined with the total deficiency of plasmin.

**Table 1**

| Effect of Galardin™ treatment on fertility in Plg^{-/-} mice | | | | | |
|---|---|---|---|---|---|
| Group | No. of mice | Pregnancies | % pregnant | % viable pregnancies in mouse group | % viable of total pregnancies |
| 1 Plg^{-/-} | 3 | 2 | 67 | 67 | 100 |
| 2 Plg^{-/-} (G) | 4 | 1* | 25* | 0 | 0 |
| 3 Plg^{+/+} | 5 | 5 | 100 | 100 | 100 |
| 4 Plg^{+/+} (G) | 5 | 3 | 80 | 80 | 100 |

| | | | | | |
|---|---|---|---|---|---|
| *Non-viable embryo. It cannot be excluded that this conception of this embryo occurred prior to time zero in the experiment and that implantation therefore had taken place prior to Galardin™ treatment. | | | | | |

### Conclusion:

These results are interpreted as follows: Implantation of the mouse embryo proceeds virtually normally when either plasmin or metalloprotease activities are available, but, successful embryo implantation is abolished in mice when plasmin is completely absent and metalloproteases are inhibited, i.e. that deficiency in plasmin and inhibition of metalloproteases act additively in abolition of the invasive implantation process.

### EXAMPLE 3

### Inhibition of Mammary Gland Involution in mice.

In this example post-lactating plasminogen deficient and wild-type mice were treated systemically with a potent metalloprotease inhibitor and the effect on mammary gland involution was assessed by weighing an excised gland after the normal involution interval.

### Background:

After mice have given birth, the lactating mammary glands of the mother become considerably enlarged in order to supply sufficient milk for feeding her pups. When the pups are weaned after about 7-8 days, lactation ceases and the glands undergo an involution process to return to their normal size. This is a typical tissue remodelling process, in which urokinase plasminogen activator and metalloproteases both contribute to matrix degradation. The levels of urokinase, gelatinase A, stromelysins 1 and 3, and MT-MMP-1 all increase rapidly 3-4 days after lactation ceases.

### Protocol:

After mice had given birth, the litter was taken away from the mother and adjusted to eight pups, adding pups from other mothers if necessary in order to provide a constant feeding load on each mother. Lactation and feeding with the eight pups were allowed to proceed for 7-8 days, at which time the litter was removed (day 1). On the morning of weaning day 2, daily systemic treatment with Galardin™ (2.5 mg/mouse by i.p. injection, in CMC vehicle) was commenced, and at weaning day 5 the mice were sacrificed and the most clearly defined gland (the fourth of four on each side) was excised and weighed.

### Results:

The results are shown in Table 2 below.

**TABLE 2**

| Effect of Galardin™ on mammary gland involution in plg^{-/-} mice | | | |
|---|---|---|---|
| Mouse Group | Gland 4 Weight (mg) | Mean Weight (mg) | % Control |
| plg^{+/+} untreated (control) | 103 128 97 | 109 | 100 |
| plg^{-/-} untreated | 157 206 231 | 198 | 182 |
| plg^{+/+} + Galardin™ | 188 198 146 | 177 | 162 |
| plg^{-/-} + Galardin™ | 262 342 251 | 285 | 262 |

Mammary gland involution in plg^{-/-} mice is retarded compared with normal plg^{+/+} mice. Galardin™ treatment of normal plg^{+/+} mice leads to retardation to a similar extent, while Galardin™ treatment in plg^{-/-} mice leads to a much more pronounced retardation of the tissue remodelling associated with mammary gland involution.

### Conclusion:

Mammary gland involution in mice is partially retarded in the absence of plasmin and again when metalloproteases are inhibited, but it is dramatically inhibited when the absence of plasmin is combined with inhibition of metalloproteases. These findings are interpreted as showing that inhibitors of plasmin and inhibitors of metalloproteases act strongly together in this tissue remodelling process.

### EXAMPLE 4

### Inhibition of Uterine Involution in plg^{-/-} mice.

In this example peri-partum plasminogen deficient (plg^{-/-}) mice were treated systemically with a potent metalloprotease inhibitor and the effect on post-partum uterine involution was assessed by weighing the resected uteri.

### Background:

After giving birth, the grossly enlarged mouse uterus previously necessary for gestation undergoes a rapid process of tissue resorption, which produces a marked reduction in size of the uterus over only a few days. Post-partum uterine involution is a typical tissue remodelling process and it is known to involve a great deal of proteolytic activity.

### Protocol:

Nine female plg^{+/+} mice and nine female plg^{-/-} mice were mated with male mice, and beginning 18.5 days post-coitum (i.e. 12-24 h before giving birth) five mice of each genotype were treated each day with a single i.p. injection of Galardin™ (90 mg/kg) in CMC vehicle. Treatment was continued to 4 days post-partum and on the fifth day post-partum the mice were sacrificed and the uteri removed, dissected free of adhering fatty tissue, and blotted dry before weighing.

### Results:

The results are shown in Table 3 below. Systemic treatment of the plg^{-/-} mice with the metalloprotease inhibitor Galardin™ produced a significant increase (p=0.012) in 5 day post-partum uterine weight, indicating a strong inhibition of uterine involution. Treatment of plg^{+/+} mice with the same dose of Galardin™ did not produce a significant increase in 5 day post-partum uterine weight (p=0.158).

**Table 3**

| Effect of a metalloprotease inhibitor on uterine involution | | |
|---|---|---|
| Treatment Group | Weight (mg) | Mean Weight (mg) ± SD |
| plg^{+/+} control | 161 180 100 202 | 160 ± 43.8 |
| plg^{+/+} + Galardin™ | 288 285 135 206 192 | 221 ± 65.3 |
| plg^{-/-} control | 155 202 263 169 | 197 ± 48.1 |
| plg^{-/-} + Galardin™ | 311 408 236 328 401 | 337 ± 70.9 |

### Conclusion:

In the absence of plasmin, the tissue remodelling associated with post-partum uterine involution in the mouse is accomplished through the proteolytic activity of metalloproteases.

### EXAMPLE 5

### (A) Wound Healing in Normal Plg^{+/+} mice Treated with Plasmin Inhibitors.

In this example mice which exhibit normal expression of plasminogen were treated systemically with two plasmin inhibitors, aprotinin and tranexamic acid. The model tested was wound healing. Successful wound healing was read as the complete closure of a skin incision. With respect to the background of the model, cf. Example 1.

### Protocol:

Twenty plg^{+/+} mice were wounded with a standardised 20 mm long, full thickness skin incision on the back and then divided into four groups: group 1 received each day three i.p. injections of buffer vehicle; group 2 received each day aprotinin (AP;13,000 KIU) divided in three i.p. injections at least six hours apart; group 3 received each day tranexamic acid (TA; 10 mg) divided in three i.p. injections at least six hours apart, and group 4 received each day combined treatment of aprotinin (13,000 KIU) and tranexamic acid (10 mg) divided in three i.p. injections at least six hours apart.

Each day the length of the open wounds was carefully measured and recorded. The time taken for each skin wound to completely close was also noted.

### Results:

The results are shown in Figs. 6 and 7. Whereas all wounds in untreated mice were healed after 17 days, and wounds in tranexamic acid treated mice were also all healed after 17 days, the wounds in the aprotinin inhibitor treated mice were only healed after 24 days, and the wounds in the mice treated with both aprotinin and tranexamic acid were only healed after 39 days.

### Conclusion:

These results demonstrate that inhibition of wound healing can be obtained *in vivo* by systemic treatment of normal wildtype mice with plasmin inhibitors, producing an inhibition of the wound healing process which is significant and similar, but not as pronounced, as that seen in untreated plg^{-/-} mice (Rømer, 1996, and example 2 above). We interpret this result to show that with the drugs, doses, and schedules used in the present experiment, a partial but incomplete systemic inhibition of plasmin was obtained in vivo.

### (B) Biological Half-life of Aprotinin in Blood Plasma of Normal plg^{+/+} mice.

The plasmin inhibitor aprotinin was injected intraperitoneally into normal mice, and the blood level of aprotinin was measured at several time points thereafter to determine the half-life of the functional activity in the circulation.

### Protocol:

Fifteen mice (weighing approx. 25 g each) were injected i.p. with 0.75 ml of aprotinin solution (20,000 KIU/ml; approx. 2.1 mg) and at time intervals of 0.5, 1.5, 3.0, 6.0 and 24 h thereafter three mice were sacrificed and blood collected by cardiac puncture into citrate anti-coagulant tubes. Each plasma sample was ultrafiltrated through a 20,000 MW cut-off membrane, and the aprotinin level in the ultrafiltrate was determined by inhibition of the colorimetric activity of a standard trypsin solution.

### Results:

The aprotinin levels found in the mouse blood are shown in Fig. 8. It can be seen that a single i.p. injection of 2.1 mg aprotinin can achieve a plasma level in a mouse of approx. 25 µg/ml, but this decays rapidly, with a half life of approx. 3 h.

### Conclusion:

We interpret these results to show that effective systemic treatment of mice with aprotinin requires that the agent is administered several times i.p. per day (e.g. 6 times), or preferably continuously, in order to maintain a high level of inhibitory activity against plasmin in the body tissues.

### (C) Effect of Plasmin Inhibitor Scheduling on Wound Healing in Normal plg^{+/+} mice.

Normal mice were treated systemically with two different schedules of a mixture of the two plasmin inhibitors, aprotinin and tranexamic acid. The model tested was wound healing. Successful wound healing was read as the complete closure of a skin incision. With respect to the background of the model, cf. Example 1.

### Protocol:

Fifteen plg^{+/+} mice were wounded with a standardised 20 mm long, full thickness skin incision on the back and then divided into three groups: group 1 received 5 i.p. injections of buffer vehicle per day; group 2 received each day aprotinin (650,000 KIU/kg) and tranexamic acid (1 g/kg) divided in 4 i.p. injections 6 hours apart, and group 3 received each day thereafter aprotinin (650,000 KIU/kg) and tranexamic acid (1 g/kg) divided in 5 i.p. injections at least 4 hours apart; Each day the length of the open wounds was carefully measured and recorded. The time taken for each skin wound to completely close was also noted.

### Results:

The results are shown in Figs. 9 and 10. Wound healing was clearly inhibited more strongly by a given dose of protease inhibitors when the systemic treatment was administered more frequently, in this example 5 daily injections compared to 4.

### Conclusion:

These results confirm that effective systemic treatment of mice with plasmin inhibitors requires that the agent is administered several times per day (e.g. 5 times), in order to maintain a high level of inhibitory activity against plasmin in the body tissues. But even with this schedule, the effect of plasmin inhibitors on skin wound healing is not as strong as that of plasminogen deficiency, indicating that the plasmin activity was completely abolished.

### EXAMPLE 6

### Effect of Combination Treatment with Plasmin Inhibitors and a Metalloprotease Inhibitor on Wound Healing in Normal plg^{+/+} mice.

In this example normal mice were treated systemically with (a) a mixture of the two plasmin inhibitors, aprotinin and tranexamic acid, (b) an inhibitor of metalloproteases, and (c) a combination of both protease inhibitor treatments. The model tested was wound healing. Successful wound healing was read as the complete closure of a skin incision. With respect to the background of the model, cf. Example 1.

### Protocol:

Forty-eight plg^{+/+} mice were wounded with a standardised 20 mm long, full thickness skin incision on the back and then divided into four groups: group 1 received 5 i.p. injections of buffered vehicle (4% suspension of carboxymethyl cellulose, CMC) per day; group 2 received each day aprotinin (13,000 KIU/day/mouse) and tranexamic acid (20 mg/day/mouse) divided in 4 i.p. injections at least five hours apart; group 3 were injected once i.p. each day with Galardin™ (2.5 mg in 125 µl of CMC); group 4 received combination treatment of aprotinin, tranexamic acid and Galardin™ at the same doses above. Each day the length of the open wounds was carefully measured and recorded. The time taken for each skin wound to completely close was also noted.

### Results:

The results are shown in Figs. 11 and 12. Wound healing in normal plg^{+/+} mice was clearly inhibited more strongly when a combination of metalloprotease inhibitor and plasmin inhibitors was used for systemic treatment.

### Conclusion:

Tissue remodelling associated with wound healing in mice can be most strongly inhibited *in vivo* when systemic protease inhibitor treatment is directed at both the plasmin and metalloprotease systems. There is however not a complete inhibition of wound healing. This is in contrast to the complete inhibition obtained by metalloprotease inhibitor treatment in plg^{-/-} mice in example 1 above. In accordance with the results in example 5, we conclude that the systemic plasmin inhibitor treatment to some extent, but not completely is able to inhibit the action of plasmin.

### EXAMPLE 7

### Implantation in Normal Plg^{+/+} mice Treated with Plasmin Inhibitors and a Metalloprotease Inhibitor.

In this example, mice which normally express plasminogen were treated systemically with a potent metalloprotease inhibitor in combination with the plasmin inhibitors aprotinin and tranexamic acid. The model tested was embryo implantation. Successful implantation was read as the presence of viable embryos in the pregnant mice. The background for the model is discussed in Example 2.

### Protocol:

In the evening, wild-type (plg^{+/+}) female mice were placed together with plg^{+/+} male mice of proven potency. Each morning the females were closely examined for the characteristic vaginal "plugs" formed after mating, and the mated females were removed and allocated to four groups. Beginning two days after mating they were given daily i.p. injections (125 µl) of either CMC vehicle (group 1), or the same vehicle with addition of Galardin™ (2.5 mg) (group 2), or aprotinin (13,000 KIU) and tranexamic acid (10 mg) divided in three i.p. injections at least six hours apart (group 3), while group 4 received each day aprotinin, tranexamic acid and Galardin™ at the same doses. The groups of mice were treated in these ways on each subsequent day and also observed closely for signs of pregnancy up to the end of the normal period of gestation (three weeks).

Note that the doses of aprotinin and tranexamic acid were those used in the wound healing experiment above (example 5) and thus were known to produce partial inhibition of plasmin *in vivo.*

### Results:

The production of offspring by the mice was unaffected by treatment with plasmin inhibitors, or with metalloprotease inhibitors or with a combination of both.

### Conclusion:

We found that combining Galardin™ with plasmin inhibitors does not lead to abolition of embryo implantation, in contrast to combining Galardin™ in the same dose and schedule with plasminogen deficiency (see Example 2). We interpret this result as an indication that we have not obtained a sufficient systemic inhibition of plasmin, in accordance with the findings of Examples 5 and 6 (see above), and we conclude that the residual plasmin activity is sufficient to allow successful implantation, even in the presence of the metalloprotease inhibitor.

### EXAMPLE 8

### Treatment of Lewis Lung Tumours in Normal C57816 Plg^{+/+} mice with Plasmin Inhibitors and a Metalloprotease Inhibitor.

In this example C57B16 mice which normally express plasminogen were treated systemically with the plasmin inhibitors aprotinin and tranexamic acid, in combination with a potent metalloprotease inhibitor. The model tested was growth of an invasive transplantable mouse tumour, the Lewis lung carcinoma.

### Background:

The Lewis lung carcinoma is a widely used and well characterised transplantable mouse tumour from a C57B1 strain background. Subcutaneous injection of a suspension of tumour fragments produces a rapidly growing aggressively invasive primary tumour which is notable for its ability to destroy adjacent muscle and connective tissue, as well as for its early metastasis to the lungs. The survival time is usually approx. 25-30 days, while metastasis by haematogenous spread has usually occurred within 7 days. The tumour tissue has previously been shown to express high levels of uPA and uPAR.

### Protocol:

Four groups of 17 mice were injected subcutaneously with Lewis lung carcinoma tissue mince (approx. 1.0 x10⁶ cells). The first group (CON) were treated only with buffered vehicle. The second group (TA/AP) was treated i.p. three times each day with a mixture of tranexamic acid (TA) and aprotinin (AP), so that the total dosage was 10 mg TA and 13,000 KIU AP per day per mouse. The third group (LBH 106) was treated with 1.8 mg Galardin™ per day per mouse (in buffered CMC vehicle), and the fourth group received tranexamic acid, aprotinin and Galardin™ at the same doses. The tumour growth in the mice was monitored every 2-3 days by careful measurement with callipers of the longest and shortest axes of the palpable tumours, and the calculated volume was plotted as a function of time. The growth rate of the tumours was followed until death and the survival of mice in the four groups was analyzed by a Kaplan-Meier plot. Histological sections from the primary murine Lewis Lung carcinoma were analyzed by *in situ* hybridization for the expression of stromelysin-1 and gelatinase-B.

For *in situ* hybridization studies mice were at day 15 after transplantation perfusion fixed with 4% paraformaldehyde. The primary tumours were removed and paraffin embedded. 5 µm sections were hybridized with ³⁵S labelled gelatinase-B and stromelysin-1 probes (Lund *et al.*, 1996). Section were exposed to an autoradiographic emulsion and developed 7 days after.

### Results:

In situ hybridisation expression studies showed that the two metalloproteases stromelysin-1 and gelatinase B are both expressed at high levels in Lewis lung tumours (Fig. 13). Figs. 13A and 13B show the same image photographed with a brightfield (A) and a darkfield condenser. The gelatinase-B expressing cells (arrows in Figs. 13 A and B) are easily identified with the darkfield condensor, where the signal for the positive cells appear as light stain (arrows in Fig. 13 B). Similarly, using a darkfield condensor, the stromelysin-1 expressing cells could easily be identified in the stroma surrounding the Lewis Lung carcinoma cells (arrows in Fig. 13D). Fig. 13C is a heamatoxylin and Eosin stained section adjacent to the section in Fig. 13D, and is included to show the tissue structure in Fig. 13D. Arrows in Fig. 13C indicate the virtual location of the positive cells indicated by arrows in Fig. 13D.

The results of systemic protease inhibitor treatments of mice with Lewis lung carcinoma are given in Figs. 14 and 15.

Treatment with plasmin inhibitors alone and also with a metalloprotease inhibitor alone produced clear decreases in the growth rate of these tumours. Combination treatment with both types of inhibitors produced a substantially stronger effect on growth than either type alone. Each of the types of inhibitors also produced a considerable prolongation of survival of the tumour-bearing mice. Also the combination of plasmin inhibitors and metalloproteases increased the survival of the mice substantially and in this group of mice there was a surprising long-term survival of three out of 17 mice. This is in sharp contrast to no long-term survivors in the other three groups of mice. In this context, it should be noted that the mice in this experiment were randomised into the four treatment groups after they had been inoculated with tumour cells. All long-term survivors were free of palpable tumours at the time when treatment was stopped (day 50). They have since been kept without treatment and are still alive and tumour-free at the date of submission of this application (> 300 days after inoculation). Out of the 68 mice which died spontaneously in this experiment, 55 were subjected to autopsy. All these 55 mice had extensive metastasis in the lungs or in other organs.

### Conclusion:

We conclude that systemic treatment of mice with either plasmin inhibitors or a metalloprotease inhibitor can produce partial inhibition of the growth of the primary Lewis lung carcinoma, and combination of the two regimens leads to a strong additive effect on the growth. All three regimens also lead to a substantial increase in the survival of the tumour inoculated mice. In the mice treated with inhibitors of the individual types, all mice eventually died. In contrast, there were three long-term survivors in the mice treated with a combination of the two regimens and we therefore conclude that with respect to survival, there is a clear synergistic effect of the combination. These findings are in good agreement with the results of the combination treatment obtained in other invasive tissue remodelling processes, and in particular in the studies on wound healing (Example 6) in which a substantial delay but not a complete arrest was observed as a result of the same combination treatment.

We contemplate that a combination of the metalloprotease inhibition with a complete plasmin inhibition may lead to a complete inhibition of tumour growth and/or to a survival of all inoculated mice. Such an experiment can readily be done when C57B1 mice have been rendered plasminogen deficient by in-breeding and subsequently by continued back-crossing has been rendered histocompatible with the Lewis lung carcinoma.

### EXAMPLE 9

### Treatment of a Human Breast Cancer Xenograft in Athymic mice with Plasmin Inhibitors and a Metalloprotease Inhibitor.

In this example athymic mice were used, which allowed the heterotransplantation and invasive growth of a human tumour in a mouse. The mice used express plasminogen, and they were treated systemically with the plasmin inhibitors aprotinin and tranexamic acid, with a potent metalloprotease inhibitor (BB-94), and with a combination of the plasmin inhibitors with BB-94. The results were read as the rate of primary tumour growth.

### Protocol:

Four groups of 11 mice were injected subcutaneously with 2 x 10⁶ cultured MDA-MB-231 human breast cancer cells. Group 1 was subsequently injected i.p. only with buffered vehicle; group 2 (TA/AP) were treated with a mixture of tranexamic acid (TA) and aprotinin (AP), so that the total dosage was 650,000 KIU/kg aprotinin and 1 g/kg tranexamic acid divided in 5 i.p. injections per day at least four hours apart; group 3 (BB-94) was treated with 125 mg/kg BB-94 as a single i.p. injection each day, and the fourth group received TA, AP and BB-94 at the same doses. The growth of the tumours was then carefully followed by measurement every 2-3 days of the longest and shortest axes of palpable tumours, and plotted as a function of time.

Tumours from untreated control mice and from mice receiving BB94 were homogenized and assayed for mouse uPA content using a mouse specific uPA ELISA.

### Results:

In this model, systemic treatment with plasmin inhibitors used alone at the dose and schedule shown did not produce a detectable inhibition of tumour growth, while systemic treatment with the metalloprotease inhibitor used alone gave clear inhibition of tumour growth, which could be further strengthened by combined systemic treatment with the plasmin inhibitors and metalloprotease inhibitor used together (Fig 16) .

ELISA measurements of mouse uPA in the tumours showed an almost 2-fold upregulation of this enzyme in mice treated with BB-94 as compared to tumours from mock-treated animals

### Conclusion:

The metalloprotease inhibitor BB-94 clearly inhibits growth of this xenografted tumour, while there is no effect on this parameter of plasmin inhibition alone. Surprisingly however, plasmin inhibition had a significant effect on the growth of tumours which already were treated with the metalloprotease inhibitor. We thus observed a clearly although moderately synergistic effect of the two regimens on tumour growth in the system.

### LIST OF REFERENCES

1. Danø, K., Andreasen, P.A., Grøndahl-Hansen, J., Kristensen, P., Nielsen, L.S., and Skriver, L. Plasminogen activators, tissue degradation, and cancer. Adv. Cancer. Res. 44:139-239 (1985)
2. Hewitt, R. and Danm, K.
   Stromal cell expression of components of matrix-degrading protease systems in human cancer. Enzyme and Protein 49: 163-173 (1996)
3. Ronnov-Jessen, L., Petersen, O.W. and Bissell, M. J. Cellular changes involved in conversion of normal to malignant breast; importance of the stromal reaction. Physiol. Rev. 76:69-125 (1996).
4. Dvorak, H.F.
   Tumors: Wounds that do not heal. Similarities between tumor stroma generation and wound healing. N. Engl. J. Med. 315:1650-1659 (1986).
5 Lund, L.R. Rømer, J., Thomasset, N., Solberg, H., Pyke, C., Bissell, M.J., Danø, K., and Werb, Z. Two distinct phases of apoptosis in mammary gland involution: proteinaseindependent and -dependent pathways. Devlopment 122:181-193 (1996)
6. Rømer. J., Bugge, T.H., Pyke, C., Lund, L.R., Flick, M.J., Degen, J.L., and Danø, K.
   Impaired wound healing in mice with a disrupted plasminogen gene.
   Nature Medicine 2:287-292 (1996).
7. Bjørn S.F. et al., 1997, Fibrinolysis and Proteolysis 11: 19, Abstract 68 (1997).
8. Bugge, T.H., Lund, L.R., Kombrinck, K.K., Nielsen, B.S., Holmback, K., Drew, A.F., Flick, M.J., Witte, D.P., Danø, K., and Degen, J. Reduced breast cancer metastasis in plasminogen deficient mice. Fibrinolysis and Proteolysis 11: 82, Abstract 82 (1997).
9. Coleman, J.L., Gebbia, J.A., Piesman, J. Degen, J.L., Bugge, T.H., Benach, J.L. Plasminogen is required for efficient dissemination of B. burgdorferi in ticks and for enhancement of spirochetemia in mice. Cell 89:1111-1119 (1997).
10. Mignatti, P. and Rifkin, D. B. Biology and biochemistry of proteases in tumour invasion. Physiol. Rev. 73: 161-195 (1993).
11. Liotta, L., and Stetler-Stevenson, W. Metalloproteinases and cancer invasion. Semin. Cancer. Biol. 1:99-106 (1990)
12. Pyke, C., Kristensen, P., Ralfkiær, E., Grøndahl-Hansen, J., Eriksen, J., Blasi, F., and Danø, K. Urokinase-type plasminogen activator is expressed in stromal cells and its receptor in cancer cells at invasive foci in human colon adenocarcinomas. Am. J. Pathol. 138:1059-1067 (1991)
13. Nielsen, B., Timshel, S., Kjeldsen, L., Sehested, M., Pyke, C., Borregaard, N., and Danø, K. 92 kDa type IV collagenase (MMP-9) is expressed in neutrophils and macrophages but not in malignant epithelial cells in human colon cancer. Int. J. Cancer 65:57-62 (1996)
14. Ganesh, S., Sier, C.F., Griffioen, G., Vloedgraven, H.J., de Boer, A., Welvaart, K., van de Velde, C.J., van Krieken, J.H., Verheijen, J.H., Lamers, C.B., and et al. Prognostic relevance of plasminogen activators and their inhibitors in colorectal cancer. Cancer Res. 54:4065-4071 (1994)
15. Murray, G.I., Duncan, M.E., O'Neil, P., Melvin, W.T., and Fothergill, J.E. Matrix metalloproteinase-1 is associated with poor prognosis in colorectal cancer. Nat. Med. 2:461-462 (1996)
16. DeClerk, Y.A., and Laug, W.E.
   Cooperation between matrix metalloproteinases and the plasminogen activator-plasmin system in tumour progression. Enzyme Protein 49:72-84 (1996)
17. Martin, P.
   Wound healing -Aiming for perfect skin regeneration. Science 276:75-81 (1997).
18. Bini, A., Itoh, Y., Kudryk, B., and Nagase, H. Degradation of cross-linked fibrin by matrix metalloprotease 3 (stromelysin 1): Hydrolysi of the gamma Gly 404-Ala 405 peptide bond. Biochemistry 35:13056-13063.
19. Schultz, G.S., Strelow, S., Stern, G.A., Chegini, M.B., Grant, M.B., Galardy, R.E., Grobelny, D., Rowsey, J.J., Stonecipher, K., Parmley, V., and Khaw, P.T.
   Treatment of alkali-injured rabbit corneas with a synthetic inhibitor of matrix metalloproteinases.
   Invest. Ophthalmol. Vis. Sci. 33(12):3325-3331 (1992)
20. Harvey, M.B., Leco, K.J., Arcellana-Panlilio, M.Y., Zhang, X., Edwards, D.R., and Schultz, G.A.
   Proteinase expression in early mouse embryos is regulated by leukaemia inhibitory factor and epidermal growth factor. Development 121:1005-1014 (1995)
21. Yagel, S., Parhar, R.S., Jeffrey, J.J., and Lala, P.K. Normal nonmetastatic human trophoblast cells share in-vitro invasive properties of malignant cells.
   J. Cell. Physiol. 136:455-462 (1988)
22. Librach, C.L, Feigenbaum, S.L., Bass, K.E., Cui, T., Verastas, N., Sadovsky, Y., Quigley, J.P., French, D.L., and Fisher, S.J.
   Interleukin-1 regulates human cytotrophoblast metalloproteinase activity and invasion *in vitro*.
   J. Biol. Chem. 269(25):17125-17131 (1994)
23. Hoffman, Glatstein, I., Schatz, F., Heller, D., and Deligdisch, L.
   Immunohistochemical localisation of urokinase-type plasminogen activator and the plasminogen activator inhibitors 1 and 2 in early human implantation sites.
   Am. J. Obstet. Gynecol. 170:671-676 (1994)
24. Salamonsen, L.A., Nagase, H., and Woolley, D.E.
   Matrix metalloproteinases and their tissue inhibitors at the ovine trophoblast-uterine interface.
   J. Reproduction and Fertility Supplement 49:29,37 (1995)
25. Librach, C.L., Werb, Z., Fitzgerald, M.L., Chiu, K., Corwin, N.M., Esteves, R.A., Grobelny, D., Galardy, R., Damsky, C.H., and Fisher, S.J.
   92-kD type IV collagenase mediates invasion of human cytotrophoblasts.
   J. Cell. Biol. 113(2):437-449 (1991)
26. Behrendtsen, O., Alexander C., and Werb, Z. Metalloproteinases mediate extracellular matrix degradation by cells from mouse blastocyst outgrowths.
   Development 114:447-56 (1992)
27. Yagel, S., Geva, T.E., Solomon, H., Shimonovitz, S., Reich, R., Finci-Yeheskel, Z., Mayer, M., and Milwidsky, A. High levels of human chorionic gonadotropin retard first trimester trophoblast invasion in vitro by decreasing urokinase plasminogen activator and collagenase activities.
   J. Clin. Endocrin. Metabolism 77(6):1506- (1993)
28. Feng, Z., Marti, A., Jehn, B., Altermatt, H.J., Chicaiza, G., and Jaggi, R.
   Glucocorticoid and progesterone inhibit involution and programmed cell death in the mouse mammary gland.
   J. Cell. Biol. 131(4):1095-1103 (1995)
29. Lage, A., Diaz, J.W., and Gonzalez, I.
   Effect of proteinase inhibitor in experimental tumours. Neoplasma 25(2):257-9 (1978)
30. Ohkoshi, M.
   Effect of aprotinin on growth of 3-methylcholanthrene-induced squamous cell-carcinoma in mice.
   Gann. 71(2):246-50 (1980)
31. Tanaka, N., Ogawa, H., Kinjo, M., Kohga, S., and Tanaka K.
   Ultrastructural study on the effects of tranexamic acid and urokinase on metastasis of Lewis lung carcinoma.
   Bri. J. Cancer 46(3):428-35 (1982)
32. Åstedt, B., Glifberg, I., Mattsson, W., and TropJ, C. Arrest of growth of ovarian tumour by tranexamic acid.
   J. Am. Med. Assoc.(JAMA) 238(2):154- (1977)
33. Åstedt, B.
   Adjuvant treatment of ovarian carcinoma with tranexamic acid. J. Clin. Pathol 33(14):74-76 (1980)
34. Bugge, T.H., Flick, M.J., Dougherty, C.C., and Degan, J.L.
   Plasminogen deficiency causes severe thrombosis but is compatible with development and reproduction.
   Genes Dev. 9:794-807 (1995).
35. Grobelny, D., Poncz, L., and Galardy, R.E.
   Inhibition of human skin fibroblast collagenase, thermolysin, and Pseudomonas aeruginosa by peptide hydroxamic acids. Biochemistry 31: 7152-7154 (1992)
36. Bogheart, E.R., Chan, S.K., Zimmer, C., Grobelny, D., Galardy, R-E., Vanaman, T.C., and Zimmer, S.G.
   Inhibition of collagenolytic activity relates to quantitative reduction of invasion *in vitro* in a c-Ha-ras transfected glial cell line. J. Neuro-Oncology 21:141-150 (1994)
37. Galardy, R.E., Grobelny, D., Foellmer, H.G., and Fernandez, L.A.
   Inhibition of angiogenesis by the matrix metalloproteinase inhibitor *N*-[2R-2-(Hydroxamidocarbonymethyl)-4-methylpentanoyl)]-L-tryptophan methylamide.
   Cancer Res. 54:4715-4718 (1994)
38. Eccles, S.A., Box, G.M., Court, W.J., Bone, E.A., Thomas, W., and Brown, P.D.
   Control of lymphatic and haematogenous metastasis of a rat mammary carcinoma by the matrix metalloproteinase inhibitor batimastat (BB-94).
   Cancer Res. 56:2815-2822 (1996)
39. Wojtowicz-Praga, S., Low, J., Marshall, J., Ness, E., Dickson, R., Barter, J., Sale, M., McCann, P., Moore, J., Cole, A., and Hawkins, M.J.
   Phase I trial of a novel matrix metalloproteinase inhibitor batimastat (BB-94) in patients with advanced cancer.
   Invest. New Drugs 14:193-202 (1996)
40. Gore, M., A=Hern, R., Stankiewicz, M., and Slevin, M. Tumour marker levels during marimastat therapy [letter] Lancet 348(9022):263-4 (1996)
41. Werb, Z., Mainardi, C., Vater, C., and Harris, E.D. Endogenous activation of latent collagenase by rheumatoid synovial cells. Evidence for a role of plasminogen activator. The New England J. Med. 296:1017-1023 (1977).
42. Murphy, G., Ward, R., Gavrilovic, J., and Atkinson, S. Physiological mechanisms for metalloproteinase activation. Matrix. Suppl. 1:224-230 (1992).

## Claims

1. The use of at least one first substance which, in a mammal, effects inhibition or abolition of the *in vivo* protein cleaving actions of plasmin as well as of active derivatives thereof,
for the preparation of a pharmaceutical composition for administration to a mammal in which *the in vivo* protein cleaving actions of at least one proteolytic enzyme which is different from plasmin as well as from active derivatives thereof and which exerts its action on at least one extracellular protein on which plasmin and active derivatives of plasmin also act enzymatically are inhibited or abolished, said at least one proteolytic enzyme being_{.} non-murine (including human) analogue(s) of murine metaltoprotease(s) which, isolated or in combination, is/are essential for embryo implantation and/or would healing in Plg^{-/-} mice but not in wildtype mice, or which, isolated or in combination, is/are necessary for invasive tissue destruction associated with malignant growth in mice where the protein cleaving actions of plasmin are substantially abolished,
for preventing or arresting invasive tissue modelling in the mammal, the invasive tissue modelling not comprising contraction of tissue or corneal ulceration.

2. The use according to claim 1, wherein the mammal to which the pharmaceutical composition is to be administered is a mammal receiving an effective amount of at least one second substance which, in the mammal, effects inhibition or abolition of the *in vivo* protein cleaving actions of at least one proteolytic enzyme which is different from plasmin as well as from active derivatives thereof and which exerts its action on at least one extracellular protein on which plasmin and active derivatives of plasmin also act enzymatically, said at least one second substance being at least one metalloprotease inhibitor which, upon administration in an effective amount, results in a significantly higher inhibition of embryo implantation and/or wound heating in Plg^{-/-} mice than in wildtype mice, or results in abolition of invasive tissue destruction associated with malignant growth in mice where the protein cleaving actions of plasmin are substantially abolished, the pharmaceutical preparation containing the at least one first substance being for administration either simultaneously with the at least one said second substance or with such an interval from the administration of the at least one said second substance that both the said at least one second substance and the said. at least one first substance are simultaneously present in concentrations which effect substantial *in vivo* inhibition, preferably blocking, of their respective target proteases.

3. The use of at least one second substance
which, in a mammal, effects inhibition or abolition of the *in vivo* protein cleaving actions of at least one proteolytic enzyme which is different from plasmin as well as from active derivatives thereof and which exerts its action on at least one extracellular protein on which plasmin and active derivatives of plasmin also act enzymatically, said at least one second substance being at least one metalloprotease inhibitor which, upon administration in an effective amount, results in a significantly higher inhibition of embryo implantation and/or wound healing in Plg^{-/-} mice than in wildtype mice, or results in abolition of invasive tissue destruction associated with malignant growth in mice where the protein cleaving actions of plasmin are substantially abolished,
for the preparation of a pharmaceutical composition for administration to a mammal in which the *in vivo* protein cleaving actions of plasmin as well as of active derivatives thereof are inhibited or abolished,
for preventing or arresting invasive tissue modelling in the mammal, the invasive tissue modelling not comprising contraction of tissue or corneal ulceration.

4. The use according to claim 3, wherein the mamma) to which the pharmaceutical composition is to be administered is a mammal receiving an effective amount of at least one first substance which, in the mamma!, effects inhibition or abolition of the *in vivo* protein cleaving actions of plasmin as well as of active derivatives thereof, the pharmaceutical preparation containing the at least one second substance being for administration either simultaneously with the said at feast one first substance or with such an interval from the administration of the said at least one first substance that both the said at least one second substance and the said at least one first substance are simultaneously present in concentrations which effect substantial *in vivo* inhibition, preferably blocking, of their respective target proteases.

5. The use according to any of the preceding claims, wherein the at least one first substance is at least one first substance effecting substantial abolishment of the *in vivo* protein cleaving actions of plasmin.

6. The use according to any of the preceding claims, wherein the at least one second substance is at least one second substance effecting substantial abolishment of the *in vivo* protein cleaving actions of the at least one metalloprotease.

7. The use of at least one third substance which, in a mammal, effects inhibition of both the *in vivo* protein cleaving actions of plasmin as well as of active derivatives thereof and of the *in vivo* protein cleaving actions of at least one proteolytic enzyme which is different from plasmin as well as from active derivatives thereof and which exerts its action on at least one extracellular protein on which plasmin and active derivatives of plasmin also act enzymatically, said at least one proteolytic enzyme being at least one metalloprotease, the at least one third substance being at feast one substance which, upon administration in an effective amount, results in a significantly higher inhibition of embryo implantation and/or wound heating in Plg^{-/-} mice than in wildtype mice or results in abolition of invasive tissue destruction associated with malignant growth in mice where the protein cleaving actions of plasmin are substantially abolished,
for the preparation of a pharmaceutical composition for administration to a mammal
for preventing or arresting invasive tissue modelling in the mammal, the invasive tissue modelling not comprising contraction of tissue or corneal ulceration.

8. The use according to any of claims 1-6, wherein at least one of the at least one first and/or at least one second substances is a third substance as defined in claim 7.

9. The use according to any of claims 4, 5, 6 or 8, wherein the at least one first substance is administered in an amount which gives rise to a concentration which is at or below the maximum pharmacologically acceptable concentration of said first substance alone, and, when the concentration is below the maximum pharmacologically acceptable concentration of the first substance alone, said concentration is one which gives rise to substantially the same inhibition as the maximum pharmacologically acceptable concentration of the first substance alone, and the pharmaceutical composition containing the at feast one second substance is for administration in an amount which gives rise to a concentration which, when the pharmaceutical composition containing the at least one second substance is administered simultaneously with or after the administration of the at least one first substance, is at or below the maximum pharmacologically acceptable concentration, and which, if below the maximum pharmacologically acceptable concentration, is a concentration which gives rise to substantially the same inhibition as the maximum pharmacologically acceptable concentration.

10. The use according to claim 2, 5, 6 or 8, wherein the at least one second substance is administered in an amount which gives rise to a concentration which is at or below the maximum pharmacologically acceptable concentration of the second substance alone, and, when the concentration is below the maximum pharmacologically acceptable concentration of the second substance alone, said concentration is one which gives rise to substantially the same inhibition as the maximum pharmacologically acceptable concentration of the second substance alone, and the pharmaceutical composition containing the at least one first substance is for administration in an amount which gives rise to a concentration which, when the at least one first substance is administered simultaneously with or after the administration of the at least one second substance, is at or below the maximum pharmacologically acceptable concentration, and which, if below the maximum pharmacologically acceptable concentration, is a concentration which gives rise to substantially the same inhibition as the maximum pharmacologically acceptable concentration.

11. The use according to claim 7, wherein the pharmaceutical composition containing the at least one third substance is for administration in an amount which gives rise to a concentration of the at least one third substance which is at or below the maximum pharmacologically acceptable concentration of the at least one third substance, and, when the concentration is below the maximum pharmacologically acceptable concentration of the at least one third substance, said concentration is one which gives rise to substantially the same inhibition as the maximum pharmacologically acceptable concentration of the at least one third substance.

12. The use according to claim 4, 5 or 8, wherein the at least one second substance is administered in an amount which conforms with that used in claim 9, and wherein the pharmaceutical composition containing the at least one first substance is for administration in an amount which, when administered simultaneously with or after the administration of the second substance, gives rise to a concentration which results in substantially the same inhibition as the maximum pharmacologically acceptable concentration.

13. The use according to claim 2, 5, 6 or 8, wherein the at least one first substance is administered in an amount which conforms with that used in claim 10, and wherein the pharmaceutical composition containing the at least one second substance is for administration in an amount which, when administered simultaneously with or after the administration of the first substance, gives rise to a concentration which results in substantially the same inhibition as the maximum pharmacologically acceptable concentration.

14. The use according to any of claims 1-13, wherein the at least one first substance is administered in an amount of between 1 and 1000 mg per day, or the pharmaceutical composition containing the at least one first substance is for administration in an amount providing between 1 and 1000 mg per day of the at least one first substance.

15. The use according to any of claims 1-14, wherein the relative amounts of the at least one first and at least one second substances being so related that the simultaneous or sequential administration of an effective amount thereof to the mammal will lead to effective prevention or arrest of invasive tissue modelling in said mammal,

16. The use according to any of claims 1-15, wherein the metalloprotease is selected from the group consisting of a collagenase, a stromelysin, a gelatinase, an elastase, and a membrane type metalloprotease.

17. The use according to claim 16, wherein the collagenase is selected from the group consisting of MMP-1, MMP-8, and MMP-13.

18. The use according to claim 16, wherein the stromelysin is selected from the group consisting of MMP-3, MMP-7, MMP-10, and MMP-11.

19. The use according to claim 16, wherein the gelatinase is selected from the group consisting of MMP-2 and MMP-9.

20. The use according to claim 16, wherein the elastase is MMP-12.

21. The use according to claim 16, wherein the membrane type metalloprotease is selected from the group consisting of MMP-14, MMP-15, and MMP-16.

22. The use according to any of claims 1-21, wherein the invasive tissue modelling is that of a malignant neoplasm.

23. The use according to claim 21, wherein the malignant neoplasm is selected from the group consisting of carcinoma such as adenocarcinoma, sarcoma such as liposarcoma, fibrosarcoma, chondrosarcoma, osteosarcoma, leiomyosarcoma, rhabomyosarcoma, glioma, neuroblastoma, medullablastoma, malignant melanoma, neurofibrosarcoma, hemangiosarcoma, and lymphangiosarcoma, and other malignant neoplasms such as malignant teratoma, dysgerminoma, seminoma, choriocarcinoma, leukemia, and lymphoma.

24. The use according to claim 23, wherein the carcinoma is carcinoma of the lung, the breast, the prostate or the colon.

25. The use according to any of claims 1-22 for use in contraception.

26. The use according to any of claims 1-25, wherein the at least one first substance is selected from the group consisting of aprotinin, tranexamic acid, Nα-trans-4-aminomethylcyclohexane carbonyllysine 4 benzoylanilide, Nα-trans-4-aminomethylcyclohexane carbonyl-O-bromobenzyloxycarbonyltyrosine 4 acetylanilide, 1-(ethoxy-carbonyloxy)ethyl trans-4-aminomethylcyclohexanecarboxylate hydrochloride (KABI 2161), alpha-2-antiplasmin, alpha-2-makroglobulin, tumour associated trypsin inhibitor, urinary trypsin inhibitor, leupeptin, pyroglutamyl-Leu-Arg-CHO, 6-aminocaproic acid, p-aminobenzamidine, bis(5-amidino-2-benzimidazolyl)methane, alpha-N-acetyl-L-lysine methyl ester, tosyl-lysine chloromethyl ketone, and Boc-D-Phe-ProBoro-Arg-OH.

27. The use according to any of claims 1-26, wherein the at least one second substance is selected from the group consisting of a tissue inhibitor of metalloproteases, alpha-2-macroglobulin, Galardin™, N-[2R-2-(hydroxamidocarbonylmethyl)-4-methylpentanolyl]-L-tryptophan methylamide, batimastat, marimastat, Gl 129471, Gl 168, Gl 173, Gl 179, Gl 184, Cl-A, Cl-B, RP59794, SC-44463, Ro31-4724, CT1746, SCH 47890, a peptide hydroxamate, LMNKPRCGVPDVGG, TNF-α releasing protease inhibitor, Zincov®, Pro-Ileu, phosphoramidon, thiorphan, tiopronin, a tetracycline, N-acetylcysteine, EDTA, and 1,10 phenanthrolene.

28. The use according to claim 26, wherein the tissue inhibitor of metalloproteases is selected from the group consisting of TIMP-1, TIMP-2, and TIMP-3.

29. The use according to claim 26, wherein the peptide hydroxamate is Pro-Leu-Gly-NHOH.

30. The use according to any of claims 7-29, wherein the at least one third substance is selected from the group consisting of a conjugate of Galardin™ with aprotinin, a conjugate of Galardin^{TM} with tranexamic acid, and a conjugate of Galardin™ with leupeptin.

31. The use according to any of claims 1-30, wherein the at least one extracellular protein is selected from the group consisting of collagen, elastin, fibrin, and proteoglycan.

32. The use according to any of claims 1-30, wherein the at (east one extracellular protein is fibronectin or laminin.

33. The use according to any of claims 1-30 wherein the at least one extracellular protein is a growth factor or a cytokine, such as transforming growth factor β (TGFβ), TNFα, basic fibroblast growth factor, and precursors of TGFβ or of other growth factors or cytokines.

34. The use according to any of the claims 1-33, wherein the pharmaceutical composition is for administration via the parenteral (such as intravenous and intraarterially), intraperitoneal, intramuscular, subcutaneous, intradermal, oral, buccal, sublingual, nasal, rectal or transdermal route.

35. The use according to any of claims 1-34, wherein the substances independently are targeted for a specific site of action.

36. The use according to any of claim 1-35, wherein the pharmaceutical composition is for systemic use.

37. The use of at least one first substance
which, in a mammal, effects inhibition or abolition of the *in vivo* protein cleaving actions of at feast one first protease, A,
for. the preparation of a pharmaceutical composition for administration to a mamma) in which the *in vivo* protein cleaving actions of at least one other protease, B, are Inhibited or abolished,
for preventing or arresting invasive tissue remodelling in the mammal, the invasive tissue remodelling not comprising contraction of tissue or corneal ulceration,
the first protease, A, and the other protease, B, having at least one extracellular protein as a common substrate, said at least one protease, B, being essential for embryo implantation and/or wound healing and/or necessary for invasive tissue destruction associated with malignant growth in the mammal when the protein cleaving actions of A are substantially abolished in the mammal but not when the protein cleaving actions of A are substantially intact in the mammal, wherein one of the proteases, A and B, being at least one metalloprotease and the other protease being plasmin or active derivatives thereof.

38. The use according to claim 37, wherein the *in vivo* protein cleaving actions of the at least one other protease, B, are inhibited or abolished by at least one second substance different from said at least one first substance.

39. A method of screening for a substance which is capable of interfering with invasive tissue modelling in a mammal, including a human being, the method comprising providing an animal except humans wherein has been substantially abolished the *in vivo* protein cleaving actions of plasmin, and thereafter assessing the effect of administration of the substance to the animal on at least one process known to involve invasive tissue modelling, and finally establishing as a result that the substance is capable of interfering with invasive tissue modelling if the at least one process is inhibited to a significantly higher degree than in both the anima) when it does not receive the substance and in a reference animal which has not had the *in vivo* protein cleaving actions of the at least one protease substantially abolished.

40. A method according to claim 39, wherein the substantial abolishment of the *in vivo* protein cleaving actions of the at least one protease has been accomplished by genetic modification of the animal or by pharmaceutical inhibition.

41. A method according to claim 39 or 40 wherein the at least one protease is selected from the group defined in any of claims 16-21.

## Patentansprüche

1. Die Verwendung zumindest einer ersten Wirksubstanz, die in Säugern eine Hemmung oder Aufhebung der in vivo-proteinspaltenden Aktivitäten des Plasmins oder aktiver Derivate davon bewirkt,
für die Herstellung einer pharmazeutischen Zubereitung bestimmt zur Anwendung in einem Säuger, in dem durch die Anwendung die in-vivo-Proteinspaltungsaktivitäten zumindest eines proteolytischen Enzyms - verschieden von Plasmin sowie aktiver Derivate davon - , welches seine Aktivität auf zumindest ein extrazelluläres Protein ausübt, auf welches Plasmin und aktive Derivate von Plasmin ebenfalls enzymatisch einwirken können, gehemmt oder aufgehoben werden, wobei zumindest ein proteolytisches Enzym oder mehrere proteolytische Enzyme (ein) Nicht-Nager-(einschließend menschliche(s)) Analog(e) von Nager-Metalloproteinasen ist/sind, das/die isoliert oder in Kombination essentiell ist/sind für die Embryo-Einnistung und/oder Wundheilung in Plg^{-/-} Mäusen nicht aber in Wildtyp-Mäusen, oder das/die isoliert oder in Kombination notwendig ist/sind für die invasive Gewebezerstörung infolge maligner Geschwulste in Mäusen in denen die Proteinspaltungsaktivitäten des Plasmins im Wesentlichen beseitigt sind,
zur Verhinderung oder Aufhaltung invasiver Gewebemodellierung in dem betreffenden Säuger, wobei die invasive Gewebemodellierung Gewebekontraktionen oder Homhautgeschwürbildungen nicht mit einschließt.

2. Die Anwendung nach Anspruch 1, wobei der Säuger in dem die pharmazeutische Zubereitung angewendet wird ein Säuger ist, der eine wirksame Menge zumindest einer zweiten Substanz erhält, die in dem Säuger eine Hemmung oder Aufhebung der in-vivo-Proteinspaltungsaktivitäten zumindest eines proteolytischen Enzyms hervorruft, welches verschieden ist von Plasmin sowie aktiver Derivate davon und welches seine Aktivität auf zumindest ein extrazelluläres Protein ausübt, auf welches Plasmin und aktive Derivate von Plasmin ebenfalls enzymatisch einwirken können,
wobei die besagte zweite Substanz aus zumindest einem Metalloproteinaseinhibitor besteht, der, nach Verabreichung in wirksamen Mengen, zu einer signifikant stärkeren Hemmung der Embryo-Einnistung und/oder des Wundheilungsprozesses in Plg^{-/-} Mäusen als in Wildtyp-Mäusen führt, oder der zur Aufhebung der mit malignem Geschwulstwachstum einhergehenden invasiven Gewebezerstörung in Mäusen führt, in denen die Proteinspaltungsaktivitäten des Plasmins im Wesentlichen beseitigt sind,
wobei die pharmazeutische Zubereitung welche zumindest eine besagte erste Substanz enthält, zur Verabreichung gedacht ist entweder gleichzeitig mit der zumindest einen zweiten Substanz oder in einem solchen Intervall mit der Verabreichung der zumindest einen zweiten Substanz, dass sowohl die besagte zumindest eine zweite Substanz als auch die besagte zumindest eine erste Substanz gleichzeitig in Konzentrationen vorhanden sind, die eine beträchtliche in vivo-Hemmung, bevorzugt aber ein Blockierung, ihrer jeweiligen Zielproteasen bewirken.

3. Die Verwendung zumindest einer zweiten Substanz
die in einem Säuger eine Hemmung oder Aufhebung der in vivo-proteinspaltenden Aktivitäten zumindest eines proteolytischen Enzyms bewirkt, welches verschieden ist von Plasmin sowie aktiver Derivate davon und welches seine Aktivität auf zumindest ein extrazelluläres Protein ausübt, auf welches Plasmin und aktive Derivate von Plasmin ebenfalls enzymatisch einwirken können, wobei die besagte zweite Substanz aus zumindest einem Metalloproteinaseinhibitor besteht, der, nach Verabreichung in wirksamen Mengen, zu einer signifikant stärkeren Hemmung der Embryo-Einnistung und/oder des Wundheilungsprozesses in Plg^{-/-} Mäusen als in Wildtyp-Mäusen führt, oder der zur Aufhebung der mit malignem Geschwulstwachstum einhergehenden invasiven Gewebezerstörung in Mäusen führt, in denen die Proteinspaltungsaktivitäten des Plasmins im Wesentlichen beseitigt sind,
zur Herstellung einer pharmazeutischen Zubereitung für die Anwendung in einem Säuger in dem die in-vivo-Proteinspaltungsaktivitäten des Plasmins wie auch aktiver Derivate davon gehemmt oder beseitigt sind,
die zur Verhinderung oder Aufhaltung invasiver Gewebemodellierung in dem Säuger angewendet wird, wobei die invasive Gewebemodellierung Gewebekontraktionen oder Hornhautgeschwürbildungen nicht mit einschließt.

4. Die Anwendung nach Anspruch 3, wobei der Säuger dem die pharmazeutische Zubereitung verabreicht werden soll ein Säuger ist, welcher eine wirksame Menge zumindest einer ersten Substanz erhält, die in dem Säuger eine Hemmung oder Aufhebung der in-vivo-Proteinspaltungsaktivitäten von Plasmin sowie aktiver Derivate davon bewirkt, wobei die pharmazeutische Zubereitung die die zumindest eine zweite Substanz enthält zur Verabreichung gedacht ist entweder gleichzeitig mit der besagten zumindest einen ersten Substanz oder in einem solchen Intervall mit der Verabreichung der besagten zumindest einen ersten Substanz, dass sowohl die besagte zumindest eine zweite Substanz als auch die besagte zumindest eine erste Substanz gleichzeitig in Konzentrationen vorhanden sind, die eine beträchtliche in vivo-Hemmung, bevorzugt aber ein Blockierung, ihrer jeweiligen Zielproteasen bewirken.

5. Die Anwendung nach einem der voranstehenden Ansprüche, wobei die zumindest eine erste Substanz zumindest eine erste Substanz ist, die eine deutliche Unterbindung der in vivo-Proteinspaltungsaktivitäten des Plasmins bewirkt.

6. Die Anwendung nach einem der voranstehenden Ansprüche, wobei die zumindest eine zweite Substanz zumindest eine zweite Substanz ist, die eine deutliche Unterbindung der in vivo-Proteinspaltungsaktivitäten der zumindest einen Metalloprotease bewirkt.

7. Die Verwendung zumindest einer dritten Substanz, die in einem Säuger sowohl die die Hemmung der in-vivo-Proteinspaltungsaktivitäten von Plasmin sowie aktiver Derivate davon als auch die Hemmung der in-vivo-Proteinspaltungsaktivitäten zumindest eines proteolytischen Enzyms bewirkt, welches verschieden ist von Plasmin sowie aktiver Derivate davon und welches seine Aktivität auf zumindest ein extrazelluläres Protein ausübt, auf welches Plasmin und aktive Derivate von Plasmin ebenfalls enzymatisch einwirken können, wobei das zumindest eine proteolytische Enzym zumindest eine Metalloprotease ist, und wobei die zumindest eine dritte Substanz zumindest eine dritte Substanz ist, die, nach Verabreichung in einer wirksamen Menge, zu einer signifikant stärkeren Hemmung der Embryo-Einnistung und/oder des Wundheilungsprozesses in Plg^{-/-} Mäusen als in Wildtyp-Mäusen führt, oder der zur Aufhebung der mit malignem Geschwulstwachstum einhergehenden invasiven Gewebezerstörung in Mäusen führt, in denen die Proteinspaltungsaktivitäten des Plasmins im Wesentlichen beseitigt sind,
zur Herstellung einer pharmazeutischen Zubereitung für die Anwendung in einem Säuger zur Verhinderung oder Aufhaltung invasiver Gewebemodellierung in dem Säuger, wobei die invasive Gewebemodellierung Gewebekontraktionen oder Hornhautgeschwürbildungen nicht mit einschließt.

8. Die Anwendung nach einem der Ansprüche 1-6, wobei zumindest eine der zumindest einen ersten und/oder der zumindest einen zweiten Substanz eine dritte Substanz ist die wie in Anspruch 7 definiert ist.

9. Die Anwendung nach einem der Ansprüche 4, 5, 6 oder 8, wobei die zumindest eine erste Substanz in einer Menge verabreicht wird, die eine Konzentration hervorruft welche bei der oder unterhalb der pharmakologisch maximal akzeptablen Konzentration der besagten ersten Substanz alleine liegt, und, wenn die Konzentration unter der pharmakologisch maximal akzeptablen Konzentration der besagten ersten Substanz alleine liegt, die besagte Konzentration eine ist, die im wesentlichen die gleiche Hemmung wie die pharmakologisch maximal akzeptable Konzentration der ersten Substanz alleine hervorruft, und wobei die pharmazeutische Zubereitung welche die zumindest eine zweite Substanz enthält für die Verabreichung in einer Menge bestimmt ist, die eine Konzentration hervorruft, die, wenn die pharmazeutische Zubereitung enthaltend die zumindest eine zweite Substanz gleichzeitig mit oder nach der Verabreichung der zumindest einen ersten Substanz verabreicht wird, bei der oder unter der pharmakologisch maximal akzeptablen Konzentration liegt, und die, falls unterhalb der der pharmakologisch maximal akzeptablen Konzentration, eine Konzentration ist die im wesentlichen die gleiche Hemmung wie die pharmakologisch maximal akzeptable Konzentration hervorruft.

10. Die Anwendung nach einem der Ansprüche 2, 5, 6 oder 8, wobei die zumindest eine zweite Substanz in einer Menge verabreicht wird, die eine Konzentration hervorruft welche bei der oder unterhalb der pharmakologisch maximal akzeptablen Konzentration der zweiten Substanz alleine liegt, und, wenn die Konzentration unter der pharmakologisch maximal akzeptablen Konzentration der zweiten Substanz alleine liegt, die besagte Konzentration eine ist, die im wesentlichen die gleiche Hemmung wie die pharmakologisch maximal akzeptable Konzentration der zweiten Substanz alleine hervorruft, und wobei die pharmazeutische Zubereitung welche die zumindest eine erste Substanz enthält für die Verabreichung in einer Menge bestimmt ist, die eine Konzentration hervorruft, die, wenn die pharmazeutische Zubereitung enthaltend die zumindest eine erste Substanz gleichzeitig mit oder nach der Verabreichung der zumindest einen zweiten Substanz verabreicht wird, bei der oder unter der pharmakologisch maximal akzeptablen Konzentration liegt, und die, falls unterhalb der der pharmakologisch maximal akzeptablen Konzentration, eine Konzentration ist die im wesentlichen die gleiche Hemmung wie die pharmakologisch maximal akzeptable Konzentration hervorruft.

11. Die Anwendung nach Anspruch 7, wobei die pharmazeutische Zubereitung enthaltend die zumindest eine dritte Substanz bestimmt ist für die Verabreichung in einer Menge, die eine Konzentration der zumindest einen dritten Substanz hervorruft, welche bei der oder unterhalb der pharmakologisch maximal akzeptablen Konzentration der zumindest einen dritten Substanz liegt, und, wenn die Konzentration unter der pharmakologisch maximal akzeptablen Konzentration der zumindest einen dritten Substanz liegt, die besagte Konzentration eine ist, die im wesentlichen die gleiche Hemmung wie die pharmakologisch maximal akzeptable Konzentration der zumindest einen dritten Substanz hervorruft.

12. Die Anwendung nach einem der Ansprüche 4, 5 oder 8, wobei die zumindest eine zweite Substanz in einer Menge verabreicht wird welche sich nach der in Anspruch 9 verwendeten Menge richtet, und wobei die pharmazeutische Zubereitung enthaltend die zumindest eine erste Substanz für die Verabreichung in einer Menge bestimmt ist, welche, wenn gleichzeitig mit oder nach Verabreichung der zweiten Substanz verabreicht, geeignet ist eine Konzentration hervorzurufen, die im wesentlichen die gleiche Hemmung wie die pharmakologisch maximal akzeptable Konzentration hervorruft.

13. Die Anwendung nach einem der Ansprüche 2, 5, 6 oder 8, wobei die zumindest eine erste Substanz in einer Menge verabreicht wird welche sich nach der in Anspruch 10 verwendeten Menge richtet, und wobei die pharmazeutische Zubereitung enthaltend die zumindest eine zweite Substanz für die Verabreichung in einer Menge bestimmt ist, welche, wenn gleichzeitig mit oder nach Verabreichung der ersten Substanz verabreicht, geeignet ist eine Konzentration hervorzurufen, die im wesentlichen die gleiche Hemmung wie die pharmakologisch maximal akzeptable Konzentration hervorruft.

14. Die Anwendung nach einem der Ansprüche 1-13, wobei die zumindest eine erste Substanz in einer Menge zwischen 1 und 1000 mg pro Tag verabreicht wird, oder die pharmazeutischen Zubereitung enthaltend die zumindest eine erste Substanz für die Verabreichung in einer Menge bestimmt ist die geeignet ist, zwischen 1 und 1000 mg pro Tag der zumindest einen ersten Substanz abzugeben.

15. Die Anwendung nach einem der Ansprüche 1-14, wobei die relativen Mengen der zumindest einen ersten und der zumindest einen zweiten Substanz so in Beziehung zueinander stehen, dass die gleichzeitige oder aufeinander folgende Verabreichung einer wirksamen Menge davon an einen Säuger zu einer wirkungsvollen Verhinderung oder Aufhaltung der invasiven Geweberemodellierung bei dem besagten Säuger führt.

16. Die Anwendung nach einem der Ansprüche 1-15, wobei die Metalloprotease ausgewählt wird aus einer Gruppe bestehend aus einer Kollagenase, einem Stromelysin, einer Gelatinase, einer Elastase und einer Metalloprotease vom Membrantyp.

17. Die Anwendung nach Anspruch 16, wobei die Kollagenase ausgewählt wird aus der Gruppe bestehend aus MMP-1, MMP-8 und MMP-13.

18. Die Anwendung nach Anspruch 16, wobei das Stromelysin ausgewählt wird aus der Gruppe bestehend aus MMP-3, MMP-7, MMP-10 und MMP-11.

19. Die Anwendung nach Anspruch 16, wobei die Gelatinase ausgewählt wird aus der Gruppe bestehend aus MMP-2 und MMP-9.

20. Die Anwendung nach Anspruch 16, wobei die Elastase identisch ist mit MMP-12.

21. Die Anwendung nach Anspruch 16, wobei die Metalloprotease vom Membrantyp ausgewählt wird aus der Gruppe bestehend aus MMP-14, MMP-15 und MMP-16.

22. Die Anwendung nach einem der Ansprüche 1-21, wobei die invasive Geweberemodellierung durch eine maligne Neoplasie erfolgt.

23. Die Anwendung nach Anspruch 21, wobei die maligne Neoplasie ausgewählt wird aus der Gruppe von Karzinomen wie zum Beispiel Adenokarzinome, aus der Gruppe von Sarkomen wie zum Beispiel Liposarkom, Fibrosarkom, Chondrosarkom, Osteosarkom, Leiomyosarkom, Rhabdomyosarkom, Gliom, Neuroblastom, MeduJlablastom, Malignes Melanom Neurofibrosarkom, Hämangiosarkom und Lymphangiosarkom, und anderen malignen Neoplasien wie zum Beispiel malignes Teratom, Dysgerminom, Seminom, Choriokarzinom, Leukämie und Lyphom.

24. Die Anwendung nach Anspruch 23, wobei das Karzinom ein Karzinom der Lunge, der Brust, der Prostata oder des Kolon ist.

25. Die Anwendung nach einem der Ansprüche 1-22 zum Zwecke der Empfängnisverhütung.

26. Die Anwendung nach einem der Ansprüche 1-25, wobei die zumindest eine erste Substanz ausgewählt wird aus der Gruppe bestehend aus Aprotinin, Tranexamsäure, Nα-Trans-4-aminomethylcyclohexan-carbonyllysin-4-benzoylanidid, Nα-Trans-4-arninomethylcyclohexan-carbonyl-O-brombenzyloxycarbonyltyrosin-4-acetylanilid, 1-(Ethoxycarbonyloxy)ethyl-trans-4-aminomethylcyclohexancarboxylat- hydrochlorid (KABI 2161), Alpha-2-Antiplasmin, Alpha-2-Makroglobulin, Tumor-assoziierter Trypsininhibitor, Urinärer Trypsininhibitor, Leupeptin, Pyroglutamyl-Leu-Arg-CHO, 6-Aminocapronsäure, p-Aminobenzamidin, Bis(5-amidin-2-benzimidazolyl)methan, Alpha-N-Acetyl-L-lysin.methylester, Tosyllysin-chlormethylketon und Boc-D-Phe-ProBoro-Arg-OH.

27. Die Anwendung nach einem der Ansprüche 1-26, wobei die zumindest eine zweite Substanz ausgewählt wird aus der Gruppe bestehend aus Gewebeinhibitor der Metalloproteasen, Alpha-2-Makroglobulin, Galardin®, N-[2R-2-(hydroxamidocarbonylmethyl)-4-methylpentanoyl]-L-tryptophanmethylamid, Batimastat, Marimastat, GI 129471, GI 168, GI 173, GI 179, GI 184, Cl-A, Cl-B, RP59794, SC-44463, Ro31-4724, CT1746, SCH 47890, ein Peptidhydroxamat, LMHKPRCGVPDVGG, TNF-α-freisetzender Proteaseinhibitor, Zincov®, Pro-Ileu, Phosphoramidon, Thiorphan, Tiopronin, ein Tetracyclin, N-Acetylcystein, EDTA und 1, 10-Phenanthrolen.

28. Die Anwendung nach Anspruch 26, wobei der Gewebeinhibitor der Metalloproteasen ausgewählt wird aus der Gruppe bestehend aus TIMP-1, TIMP-2 und TIMP-3.

29. Die Anwendung nach Anspruch 26, wobei das Peptidhydroxamat Pro-Leu-Gly-NHOH ist.

30. Die Anwendung nach einem der Ansprüche 7-29, wobei die zumindest eine dritte Substanz ausgewählt wird aus der Gruppe bestehend aus einem Konjugat von Galardin mit Aprotinin, einem Konjugat von Galardin mit Tranexamsäure und einem Konjugat von Galardin mit Leupeptin.

31. Die Anwendung nach einem der Ansprüche 1-30, wobei das zumindest eine extrazelluläre Protein ausgewählt wird aus der Gruppe bestehend aus Kollagen, Elastin, Fibrin und Proteoglykan.

32. Die Anwendung nach einem der Ansprüche 1-30, wobei das zumindest eine extrazelluläre Protein Fibronectin oder Laminin ist.

33. Die Anwendung nach einem der Ansprüche 1-30, wobei das zumindest eine extrazelluläre Protein ein Wachstumsfaktor oder ein Cytokin ist, wie zum Beispiel der Transforming Growth Factor β (TGFβ), TNFα, Basischer Fibroblasten Growth Factor und Vorläufer des TGFβ oder anderer Wachstumsfaktoren oder Cytokine.

34. Die Anwendung nach einem der Ansprüche 1-33, wobei die pharmazeutische Zubereitung bestimmt ist für die Verabreichung auf parenteralem (wie zum Beispiel intravenös und intraarteriell), intraperitonealem; intramuskulärem; subkutanem, intradermalem, oralem, bukkalem, sublingualem, nasalem, rektalem oder transdermalem Weg.

35. Die Anwendung nach einem der Ansprüche 1-34, wobei die Substanzen unabhängig auf einen spezifischen Wirkort gerichtet sind.

36. Die Anwendung nach einem der Ansprüche 1-35, wobei die pharmazeutische Zubereitung für den systemischen Einsatz bestimmt ist.

37. Die Verwendung zumindest einer ersten Substanz, die
in einem Säuger eine Hemmung oder Aufhebung der in-vivo-Proteinspaltungsaktivitäten zumindest einer ersten Protease, A, bewirkt,
für die Herstellung einer pharmazeutischen Zubereitung für die Verabreichung an einen Säuger in dem die in-vivo-Proteinspaltungsaktivitäten zumindest einer anderen Protease, B, gehemmt oder aufgehoben sind,
für die Verhinderung oder Aufhaltung der invasiven Geweberemodellierung in dem Säuger, wobei die invasive Gewebemodellierung Gewebekontraktionen oder Hornhautgeschwürbildungen nicht mit einschließt,
wobei die erste Protease, A, und die andere protease, B, zumindest ein extrazelluläres Protein als Substrat gemeinsam haben, wobei die besagte zumindest eine Protease, B, essentiell ist für Embryo-Einnistung und/oder Wundheilung und/oder essentiell ist für die invasive Gewebezerstörung infolge einer malignen Geschwulstbildung im Säuger wenn die Proteinspaltungsaktivitäten von A im wesentlichen aufgehoben sind in dem Säuger, aber nicht wenn die Proteinspaltungsaktivitäten von A im wesentlichen intakt sind in dem Säuger, wobei eine der Proteasen A und B zumindest eine Metalloprotease darstellt und die jeweils andere Protease identisch ist mit Plasmin oder aktiven Derivaten davon.

38. Die Anwendung nach Anspruch 37, wobei die in-vivo-Proteinspaltungsaktivitäten der zumindest einen anderen Protease, B, gehemmt oder aufgehoben sind durch zumindest eine zweite Substanz, die von der besagten zumindest einen ersten Substanz verschieden ist.

39. Eine Methode zum Screening nach einer Substanz welche in der Lage ist, in die invasive Geweberemodellierung einzugreifen in einem Säuger, einen Menschen eingeschlossen, wobei die Methode einen Säuger vorsieht, nicht jedoch einen Menschen, wobei in dem Säuger die in-vivo-Proteinspaltungsaktivitäten des Plasmins weitgehend aufgehoben sind, und die weiterhin die Auswirkungen der Verabreichung der Substanz an das Tier anhand zumindest eines Prozesses abschätzt, der bekanntermaßen eine invasive Geweberemodellierung beinhaltet, und die schließlich als Ergebnis feststellt, dass die Substanz in der Lage ist, in die invasive Geweberemodellierung einzugreifen, falls der zumindest eine Prozess in einem signifikant höheren Maße gehemmt wird als sowohl in dem Tier, wenn es die Substanz nicht erhält, als auch in einem Referenztier, in welchem die in-vivo-Proteinspaltungsaktivitäten der zumindest einen Protease nicht weitgehend aufgehoben gewesen sind.

40. Eine Methode gemäß Anspruch 39, bei welcher die weitgehende Aufhebung der in-vivo-Proteinspaltungsaktivitäten der zumindest einen Protease durch genetische Veränderung des Tieres oder durch pharmazeutische Hemmung erreicht worden ist.

41. Eine Methode gemäß Anspruch 39 oder 40, wobei die zumindest eine Protease ausgewählt wird aus der Gruppe wie in einem der Ansprüche 16-21 definiert.

## Revendications

1. Utilisation d'au moins une première substance qui, chez un mammifère, effectue l'inhibition ou la suppression des actions de clivage protéique *in vivo* de la plasmine ainsi que des dérivés actifs de celle-ci,
pour la préparation d'une composition pharmaceutique destinée à être administrée à un mammifère dans lequel les actions de clivage protéique *in vivo* d'au moins une enzyme protéolytique qui est différente de la plasmine ainsi que des dérivés actifs de celle-ci et qui exerce son action sur au moins une protéine extracellulaire sur laquelle la plasmine et les dérivés actifs de la plasmine agissent également enzymatiquement sont inhibées ou supprimées, ladite au moins une enzyme protéolytique étant un ou des analogue(s) non murin(s) (y compris humains) d'une ou de métalloprotéase(s) murine(s) qui, isolé(s) ou en combinaison, est/sont essentiel(s) à l'implantation des embryons et/ou à la cicatrisation des plaies chez les souris Plg^{-/-} mais pas chez les souris de type sauvage, ou qui, isolé(s) ou en combinaison, est/sont nécessaire(s) à la destruction de tissu invasif associé à une croissance maligne chez les souris où les actions de clivage protéique de la plasmine sont substantiellement supprimées,
pour empêcher ou interrompre le modelage du tissu invasif chez le mammifère, le modelage de tissu invasif ne comprenant pas la contraction des tissus ou l'ulcération cornéenne.

2. Utilisation selon la revendication 1, dans laquelle le mammifère auquel la composition pharmaceutique doit être administrée est un mammifère recevant une quantité efficace d'au moins une deuxième substance qui, chez le mammifère, effectue l'inhibition ou la suppression des actions de clivage protéique in vivo d'au moins une enzyme protéolytique qui est différente de la plasmine ainsi que des dérivés actifs de celle-ci et qui exerce son action sur au moins une protéine extracellulaire sur laquelle la plasmine et les dérivés actifs de la plasmine agissent également enzymatiquement, ladite au moins une deuxième substance étant au moins un inhibiteur des métalloprotéases qui, administrée en quantité efficace, aboutit à une inhibition significativement plus élevée de l'implantation des embryons et/ou de la cicatrisation des plaies chez les souris Plg^{-/-} que chez les souris de type sauvage, ou aboutit à la suppression de la destruction de tissu invasif associée à une croissance maligne chez les souris où les actions de clivage protéique de la plasmine sont substantiellement supprimées, la préparation pharmaceutique contenant la au moins une deuxième substance étant destinée à être administrée soit simultanément avec ladite au moins une deuxième substance ou avec un tel intervalle avec l'administration de ladite au moins une deuxième substance qu'à la fois ladite au moins une deuxième substance et ladite au moins une première substance sont simultanément présentes en concentrations qui effectuent une inhibition *in vivo* substantielle, de préférence par blocage, de leurs protéases cibles respectives.

3. Utilisation d'au moins une deuxième substance
qui, chez un mammifère effectue l'inhibition ou la suppression des actions de clivage protéique *in vivo* d'au moins une enzyme protéolytique qui est différente de la plasmine ainsi que des dérivés actifs de celle-ci et qui exerce son action sur au moins une protéine extracellulaire sur laquelle la plasmine et les dérivés actifs de la plasmine agissent également enzymatiquement, ladite au moins une deuxième substance étant au moins un inhibiteur des métalloprotéases qui, administrée en quantité efficace, aboutit à une inhibition significativement plus élevée de l'implantation des embryons et/ou de la cicatrisation des plaies chez les souris Plg^{-/-} que chez les souris de type sauvage, ou aboutit à la suppression de la destruction de tissu invasif associée à une croissance maligne chez les souris où les actions de clivage protéique de la plasmine sont substantiellement supprimées,
pour la préparation d'une composition pharmaceutique destinée à être administrée à un mammifère dans lequel les actions de clivage protéique *in vivo* de la plasmine ainsi que des dérivés actifs de celle-ci sont inhibées ou supprimées,
pour empêcher ou interrompre le modelage du tissu invasif chez le mammifère, le modelage de tissu invasif ne comprenant pas la contraction des tissus ou l'ulcération cornéenne.

4. Utilisation selon la revendication 3, dans laquelle le mammifère auquel la composition pharmaceutique doit être administrée est un mammifère recevant une quantité efficace d'au moins une première substance qui, chez le mammifère, effectue l'inhibition ou la suppression des actions de clivage protéique in vivo de la plasmine ainsi que des dérivés actifs de celle-ci, la préparation pharmaceutique contenant la au moins une deuxième substance étant destinée à être administrée soit simultanément avec ladite au moins une première substance ou avec un tel intervalle avec l'administration de ladite au moins une première substance qu'à la fois ladite au moins une deuxième substance et ladite au moins une première substance sont simultanément présentes en concentrations qui effectuent une inhibition *in vivo* substantielle, de préférence par blocage, de leurs protéases cibles respectives.

5. Utilisation selon l'une quelconque des revendications précédentes, dans laquelle la au moins une première substance est au moins une première substance effectuant une suppression substantielle des actions de clivage protéique *in vivo* de la plasmine.

6. Utilisation selon l'une quelconque des revendications précédentes, dans laquelle la au moins une deuxième substance est au moins une deuxième substance effectuant une suppression substantielle des actions de clivage protéique *in vivo* de la au moins une métalloprotéase.

7. Utilisation d'au moins une troisième substance qui, chez un mammifère, effectue l'inhibition à la fois des actions de clivage protéique in vivo de la plasmine ainsi que des dérivés actifs de celle-ci et des actions de clivage protéique *in vivo* d'au moins une enzyme protéolytique qui est différente de la plasmine ainsi que des dérivés actifs de celle-ci et qui exerce son action sur au moins une protéine extracellulaire sur laquelle la plasmine et les dérivés actifs de celle-ci agissent également enzymatiquement, ladite au moins une enzyme protéolytique étant au moins une métalloprotéase, la au moins une troisième substance étant au moins une substance qui, administrée en quantité efficace, aboutit à une inhibition significativement plus élevée de l'implantation des embryons et/ou de la cicatrisation des plaies chez les souris Plg^{-/-} que chez les souris de type sauvage, ou aboutit à la suppression de la destruction de tissu invasif associée à une croissance maligne chez les souris où les actions de clivage protéique de la plasmine sont substantiellement supprimées,
pour la préparation d'une composition pharmaceutique destinée à être administrée à un mammifère pour empêcher ou interrompre le modelage de tissu invasif chez le mammifère, le modelage de tissu invasif ne comprenant pas la contraction des tissus ou l'ulcération cornéenne.

8. Utilisation selon l'une quelconque des revendications 1 à 6, dans laquelle au moins une de la au moins première et/ou de la au moins deuxième substances est une troisième substance telle que définie dans la revendication 7.

9. Utilisation selon l'une quelconque des revendications 4, 5, 6 ou 8, dans laquelle, la au moins une première substance est administrée en quantité qui donne lieu à une concentration qui est au niveau ou en dessous de la concentration pharmacologiquement acceptable maximum de ladite première substance seule, et, lorsque la concentration est en dessous de la concentration pharmacologiquement acceptable maximum de la première substance seule, ladite concentration est une concentration qui donne lieu substantiellement à la même inhibition que la concentration pharmacologiquement acceptable maximum de la première substance seule, et la composition pharmaceutique contenant la au moins une deuxième substance est destinée à être administrée en quantité qui donne lieu à une concentration qui; lorsque la composition pharmaceutique contenant la au moins une deuxième substance est administrée simultanément ou après l'administration de la au moins une première substance, est au niveau ou en dessous de la concentration pharmacologiquement acceptable maximum, et qui, si elle est en dessous de la concentration pharmacologiquement acceptable maximum, est une concentration qui donne lieu substantiellement à la même inhibition que la concentration pharmacologiquement acceptable maximum.

10. Utilisation selon la revendication 2, 5, 6 ou 8, dans laquelle la au moins une deuxième substance est administrée en quantité qui donne lieu à une concentration qui est au niveau ou en dessous de la concentration pharmacologiquement acceptable maximum de la deuxième substance seule, et, lorsque la concentration est en dessous de la concentration pharmacologiquement acceptable maximum de la deuxième substance seule, ladite concentration est une concentration qui donne lieu substantiellement à la même inhibition que la concentration pharmacologiquement acceptable maximum de la deuxième substance seule, et la composition pharmaceutique contenant la au moins une première substance est destinée à être administrée en quantité qui donne lieu à une concentration qui, lorsque la au moins une première substance est administrée simultanément ou après l'administration de la au moins une deuxième substance, est au niveau ou en dessous de la concentration pharmacologiquement acceptable maximum, et qui, si elle est en dessous de la concentration pharmacologiquement acceptable maximum, est une concentration qui donne lieu substantiellement à la même inhibition que la concentration pharmacologiquement acceptable maximum.

11. Utilisation selon la revendication 7, dans laquelle la composition pharmaceutique contenant la au moins troisième substance est destinée à être administrée en quantité qui donne lieu à une concentration de la au moins troisième substance qui est au niveau ou en dessous de la concentration pharmacologiquement acceptable maximum de la au moins troisième substance, et, lorsque la concentration est en dessous de la concentration pharmacologiquement acceptable maximum de la au moins troisième substance, ladite concentration est une concentration qui donne lieu substantiellement à la même inhibition que la concentration pharmacologiquement acceptable maximum de la au moins troisième substance.

12. Utilisation selon la revendication 4, 5 ou 8, dans laquelle la au moins deuxième substance est administrée en quantité qui est en conformité avec celle utilisée dans la revendication 9, et dans laquelle la composition pharmaceutique contenant la au moins première substance est destinée à être administrée en quantité qui, lorsqu'elle est administrée simultanément ou après l'administration de la deuxième substance, donne lieu à une concentration qui aboutit à substantiellement la même inhibition que la concentration pharmacologiquement acceptable maximum.

13. Utilisation selon la revendication 2, 5, 6 ou 8, dans laquelle la au moins première substance est administrée en quantité qui est en conformité avec celle utilisée dans la revendication 10, et dans laquelle la composition pharmaceutique contenant la au moins deuxième substance est destinée à être administrée en quantité qui, lorsqu'elle est administrée simultanément ou après l'administration de la première substance, donne lieu à une concentration qui aboutit à substantiellement la même inhibition que la concentration pharmacologiquement acceptable maximum.

14. Utilisation selon l'une quelconque des revendications 1 à 13, dans laquelle la au moins une première substance est administrée en quantité comprise entre 1 et 1000 mg par jour, ou la composition pharmaceutique contenant la au moins une première substance est destinée à être administrée en quantité apportant entre 1 et 1000 mg par jour de la au moins une première substance.

15. Utilisation selon l'une quelconque des revendications 1 à 14, dans laquelle les quantités relatives de la au moins une première et de la au moins une deuxième substances sont liées de telle sorte que l'administration simultanée ou séquentielle d'une quantité efficace de celles-ci au mammifère mènera à une prévention ou une interruption efficace du modelage de tissu invasif dans ledit mammifère.

16. Utilisation selon l'une quelconque des revendications 1 à 15, dans laquelle la métalloprotéase est choisie dans le groupe constitué par une collagénase, une stromélysine, une gélatinase, une élastase et une métalloprotéase de type membranaire.

17. Utilisation selon la revendication 16, dans laquelle la collagénase est choisie dans le groupe constitué par MMP-1, MMP-8, et MMP-13.

18. Utilisation selon la revendication 16, dans laquelle la stromélysine est choisie dans le groupe constitué par MMP-3, MMP-7, MMP-10 et MMP-11.

19. Utilisation selon la revendication 16, dans laquelle la gélatinase est choisie dans le groupe constitué par MMP-2 et MMP-9.

20. Utilisation selon la revendication 16, dans laquelle l'élastase est MMP-12.

21. Utilisation selon la revendication 16, dans laquelle la métalloprotéase de type membranaire est choisie dans le groupe constitué par MMP-14, MMP-15, et MMP-16.

22. Utilisation selon l'une quelconque des revendications 1 à 21, dans laquelle le modelage de tissu invasif est celui d'un néoplasme malin.

23. Utilisation selon la revendication 21, dans laquelle le néoplasme malin est choisi dans le groupe constitué par des carcinomes tels que l'adénocarcinome, des sarcomes tels que le liposarcome, le fibrosarcome, le chondrosarcome, l'ostéosarcome, le léiomyosarcome, le rhabdomyosarcome, le gliome, le neuroblastome, le médulloblastome, le mélanome malin, le neurofibrosarcome, l'hernangiosarcome, et le lymphangiosarcome, et autres néoplasmes malins tels que le tératome malin, le dysgerminome, le seminome, le chorio-épithéliome, la leucémie, et le lymphome.

24. Utilisation selon la revendication 23, dans laquelle le carcinome est le cancer du poumon, des seins, de la prostate ou du colon.

25. Utilisation selon l'une quelconque des revendications 1 à 22 destinée pour une utilisation en contraception.

26. Utilisation selon l'une quelconque des revendications 1 à 25, dans laquelle la au moins une première substance est choisie dans le groupe constitué par l'aprotinine, l'acide tranexamique, Nα-trans-4-aminométhyl-cyclohexane carbonyl lysine 4 benzoylanilide, Nα-trans-4-aminométhyl-cyclohexane carbonyl-O-bromobenzyloxycarbonyl tyrosine 4 acétylanilide, l'hydrochlorure de 1-(éthoxycarbonyloxy)éthyl trans-4-aminométhyl cyclohexane carboxylate (KABI 2161), l'alpha 2 antiplasmine, l'alpha 2 macroglobuline, un inhibiteur de la trypsine associé à une tumeur, un inhibiteur urinaire de la trypsine, la leupeptine, la pyroglutamyl-Leu-Arg-CHO, l'acide 6-aminocaproïque, la p-aminobenzamidine, bis(5-amidino-2-benzymidazolyl)méthane, l'ester méthylique d'alpha-N-acétyl-L-lysine, tosyl-lysine chlorométhyl cétone, et Boc-D-Phe-ProBoro-Arg-OH.

27. Utilisation selon l'une quelconque des revendications 1 à 26, dans laquelle la au moins une deuxième substance est choisie dans le groupe constitué par un inhibiteur tissulaire des métalloprotéases, d'alpha 2 macroglobuline, Galardin^{TM}, N-[2R-2-(hydroxamidocarbonylméthyl)-4-méthylpentanolyl]-L-tryptophane méthylamide, batimastat, marimastat, Gl 129471, Gl 168, Gl173, Gl 179, GL 184, Cl-A, Cl-B, RP59794, SC-44463, Ro31-4724, CT1746, SCH 47890, un hydroxamate peptidique, LMHKPRCGVPDVGG, un inhibiteur de protéase libérant TNF-α, Zincov®, Pro-Ileu, le phosphoramidon, la thiophane, la tiopronine, une tétracycline, N-acétylcystéine, EDTA, et 1,10 phénanthrolène.

28. Utilisation selon la revendication 26, dans laquelle l'inhibiteur tissulaire des métalloprotéases est choisi dans le groupe constitué par TIMP-1, TIMP-2, et TIMP-3.

29. Utilisation selon la revendication 26, dans laquelle l'hydroxymate peptidique est Pro-Leu-Gly-NHOH.

30. Utilisation selon l'une quelconque des revendications 7 à 29, dans laquelle la au moins une troisième substance est choisie dans le groupe constitué par un conjugué de Galardin^{TM} avec de l'aprotinine, d'un conjugué de Galardin™ avec de l'acide tranexamique, et d'un Galardin™ avec de la leupeptine.

31. Utilisation selon l'une quelconque des revendications 1 à 30, dans laquelle la au moins une protéine extracellulaire est choisie dans le groupe constitué par le collagène, l'élastine, la fibrine et la protéoglycane.

32. Utilisation selon l'une quelconque des revendications 1 à 30, dans laquelle la au moins une protéine extracellulaire est la fibronectine ou la laminine.

33. Utilisation selon l'une quelconque des revendications 1 à 30, dans laquelle la au moins une protéine extracellulaire est une facteur de croissance ou une cytokine, tel que le facteur de croissance transformant β (TGFβ), le TNFα, le facteur de croissance basique des fibroblastes, et des précurseurs de TGFβ ou d'autres facteurs de croissance ou de cytokines.

34. Utilisation selon l'une quelconque des revendications 1 à 33, dans laquelle la composition pharmaceutique est destinée à une administration par voie parentérale (telle que intraveineuse ou intra-artérielle), intrapéritonéale, intramusculaire, sous-cutanée, intradermique, orale, buccale, sublinguale, nasale, rectale ou transdermique.

35. Utilisation selon l'une quelconque des revendications 1 à 34, dans laquelle les substances indépendamment sont ciblées pour un site d'action spécifique.

36. Utilisation selon l'une quelconque des revendications 1 à 35, dans laquelle la composition pharmaceutique est destinée à une utilisation systémique.

37. Utilisation d'au moins une première substance
qui, chez un mammifère, effectue l'inhibition ou la suppression des actions de clivage protéique *in vivo* d'au moins une première protéase, A,
pour la préparation d'une composition pharmaceutique destinée à être administrée à un mammifère chez lequel les actions de clivage protéique *in vivo* d'au moins une autre protéase, B, sont inhibées ou supprimées,
pour prévenir ou interrompre le remodelage de tissu invasif chez le mammifère, le remodelage de tissu invasif ne comprenant pas la contraction des tissus ou l'ulcération cornéenne,
la première protéase, A, et l'autre protéase, B, ayant au moins une protéine extracellulaire comme substrat commun, ladite au moins une protéase, B, étant essentielle pour l'implantation des embryons et/ou la cicatrisation de plaies et/ou étant nécessaire pour la destruction de tissu invasif associé à une croissance maligne chez un mammifère lorsque les actions de clivage protéique de A sont substantiellement supprimées chez le mammifère mais pas lorsque les actions de clivage protéique de A sont substantiellement intactes chez le mammifère, dans laquelle une des protéases, A et B, est au moins une métalloprotéase et l'autre protéase est la plasmine ou des dérivés actifs de celle-ci.

38. Utilisation selon la revendication 37, dans laquelle les actions de clivage protéique *in vivo* de la au moins une autre protéase, B, sont inhibées ou supprimées par au moins une deuxième substance différente de ladite au moins une première substance.

39. Procédé de criblage d'une substance qui est capable d'interférer avec le modelage de tissu invasif chez un mammifère, comprenant l'être humain, le procédé comprenant de fournir un animal à l'exception des humains dans lequel ont été substantiellement supprimées les actions de clivage protéique *in vivo* de la plasmine, et après cela d'évaluer l'effet de l'administration de la substance à l'animal sur au moins un processus connu pour impliquer le modelage de tissu invasif, et enfin d'établir comme résultat que la substance est capable d'interférer avec le modelage de tissu invasif si le au moins un processus est inhibé à un degré significativement plus élevé que chez l'animal lorsqu'il ne reçoit pas la substance et que chez un animal de référence qui n'a pas eu les actions de clivage protéique *in vivo* de la au moins une protéase substantiellement abolies.

40. Procédé selon la revendication 39, dans lequel la suppression substantielle des actions de clivage protéique *in vivo* de la au moins une protéase a été accomplie par modification génétique de l'animal ou par inhibition pharmaceutique.

41. Procédé selon la revendication 39 ou 40, dans lequel la au moins une protéase est choisie dans le groupe défini dans l'une quelconque des revendications 16 à 21.
